# EUROPEAN PATENT APPLICATION

(11) **EP 2 248 896 A2**
(43) Date of publication of application: **10.11.2010**
(21) Application number: 10174533.9
(22) Date of filing: 29.12.2004
(51) Int. Cl.: C12N 15/113, A61K 31/713, C12Q 1/68, C12N 5/10, G01N 33/68, A61P 19/08, A61P 19/10

(54) **Methods for inducing differentiation of undifferentiated mammalian cells into osteoblasts**

(30) Priority: 29.12.2003 WO PCT/EP03/14994
(62) Divisional of application: 04804467.1
(71) Applicant: Galapagos N.V., 2800 Mechelen (BE)
(72) Inventor: Van Rompaye, Luc Juliaan Corina, 3140 Keerbergen (BE); Tomme, Peter Herwig Maria, 9000 Gent (BE); Brown, Robin John, 9800 Bachte-Maria-Leerne (BE)
(74) Representative: van Kooij, Adriaan

(57) **Abstract**

The present invention provides methods for inducing differentiation of undifferentiated mammalian cells into osteoblasts, and methods for identifying a compound that induces differentiation of undifferentiated mammalian cells into osteoblasts. In addition, the present invention provides polynucleotides and vectors, and the use thereof as a medicament for the treatment of a disease involving a systemic or local decrease in mean bone density. Moreover, the present invention provides methods for in vitro production of bone tissue, osteoblast cells and methods for diagnosing a pathological condition involving a systemic or local decrease in mean bone density or a susceptibility to the condition.

SEQUENCE LISTING

<110>  Galapagos Genomics N.V.

<120>  Methods for inducing differentiation of undifferentiated
       mammalian cells into osteoblasts

<130>  4/2JR23/18A

<140>  PCT/EP04/014885
<141>  2004-12-29

<150>  PCT/EP03/14994
<151>  2003-12-29

<160>  334

<170>  PatentIn version 3.3

<210>  1
<211>  19
<212>  DNA
<213>  Artificial Sequence

<220>
<223>  Synthetic oligonucleotide

<400>  1
gtacctgcag gtgctcagc                                                    19


<210>  2
<211>  19
<212>  DNA
<213>  Artificial Sequence

<220>
<223>  Synthetic oligonucleotide

<400>  2
atgggcttca acagccacc                                                    19


<210>  3
<211>  19
<212>  DNA
<213>  Artificial Sequence

<220>
<223>  Synthetic oligonucleotide

<400>  3
caacttgtac ctgggcagc                                                    19


<210>  4
<211>  19
<212>  DNA
<213>  Artificial Sequence

<220>
<223>  Synthetic oligonucleotide

<400>  4
ggtgaaggag gtgctggac                                                    19


<210>  5
<211>  19

<212>  DNA
<213>  Artificial Sequence

<220>
<223>  Synthetic oligonucleotide

<400>  5
gaatctcttc cgcaagatc                                                    19


<210>  6
<211>  19
<212>  DNA
<213>  Artificial Sequence

<220>
<223>  Synthetic oligonucleotide

<400>  6
catgctgttt gagagcatc                                                    19


<210>  7
<211>  19
<212>  DNA
<213>  Artificial Sequence

<220>
<223>  Synthetic oligonucleotide

<400>  7
tttgtgctga ttccatggc                                                    19


<210>  8
<211>  19
<212>  DNA
<213>  Artificial Sequence

<220>
<223>  Synthetic oligonucleotide

<400>  8
gacggtgtta atgatagcc                                                    19


<210>  9
<211>  19
<212>  DNA
<213>  Artificial Sequence

<220>
<223>  Synthetic oligonucleotide

<400>  9
cttcggcact cctgagttc                                                    19


<210>  10
<211>  19
<212>  DNA
<213>  Artificial Sequence

<220>
<223>  Synthetic oligonucleotide

<400>  10
gcacagtttg agaagcagc                                                    19

<210>  11
<211>  19
<212>  DNA
<213>  Artificial Sequence

<220>
<223>  Synthetic oligonucleotide

<400>  11
acgcaaagtg gccaggagc                                                    19


<210>  12
<211>  19
<212>  DNA
<213>  Artificial Sequence

<220>
<223>  Synthetic oligonucleotide

<400>  12
cgatgtgcct tcaagattc                                                    19


<210>  13
<211>  19
<212>  DNA
<213>  Artificial Sequence

<220>
<223>  Synthetic oligonucleotide

<400>  13
gtgcacggat tcagagagc                                                    19


<210>  14
<211>  19
<212>  DNA
<213>  Artificial Sequence

<220>
<223>  Synthetic oligonucleotide

<400>  14
cttctacacg tccatgctc                                                    19


<210>  15
<211>  19
<212>  DNA
<213>  Artificial Sequence

<220>
<223>  Synthetic oligonucleotide

<400>  15
caacctgctg gtgctcgtc                                                    19


<210>  16
<211>  19
<212>  DNA
<213>  Artificial Sequence

<220>
<223>  Synthetic oligonucleotide

<400>  16
ctctcttaga tctggaacc                                                    19


<210>  17
<211>  19
<212>  DNA
<213>  Artificial Sequence

<220>
<223>  Synthetic oligonucleotide

<400>  17
agcaggaagg cggacatac                                                    19


<210>  18
<211>  19
<212>  DNA
<213>  Artificial Sequence

<220>
<223>  Synthetic oligonucleotide

<400>  18
ctccagctga gtgagcagc                                                    19


<210>  19
<211>  19
<212>  DNA
<213>  Artificial Sequence

<220>
<223>  Synthetic oligonucleotide

<400>  19
taaaccagca gaggagctc                                                    19


<210>  20
<211>  19
<212>  DNA
<213>  Artificial Sequence

<220>
<223>  Synthetic oligonucleotide

<400>  20
tcaggtagtt ggttctgac                                                    19


<210>  21
<211>  19
<212>  DNA
<213>  Artificial Sequence

<220>
<223>  Synthetic oligonucleotide

<400>  21
ctgcgccgaa caaatgtac                                                    19


<210>  22
<211>  19
<212>  DNA
<213>  Artificial Sequence

<220>
<223>  Synthetic oligonucleotide

<400>  22
tgtggcgact tgtgcacac                                                    19


<210>  23
<211>  19
<212>  DNA
<213>  Artificial Sequence

<220>
<223>  Synthetic oligonucleotide

<400>  23
tctctcagtg tagaatgcc                                                    19


<210>  24
<211>  19
<212>  DNA
<213>  Artificial Sequence

<220>
<223>  Synthetic oligonucleotide

<400>  24
ccaggaggtg aaaccacac                                                    19


<210>  25
<211>  19
<212>  DNA
<213>  Artificial Sequence

<220>
<223>  Synthetic oligonucleotide

<400>  25
tcaggcgact actttactc                                                    19


<210>  26
<211>  19
<212>  DNA
<213>  Artificial Sequence

<220>
<223>  Synthetic oligonucleotide

<400>  26
gtgtactggt acaaggacc                                                    19


<210>  27
<211>  19
<212>  DNA
<213>  Artificial Sequence

<220>
<223>  Synthetic oligonucleotide

<400>  27
gagcaggttt ccaaaggac                                                    19


<210>  28
<211>  19

<212>  DNA
<213>  Artificial Sequence

<220>
<223>  Synthetic oligonucleotide

<400>  28
tctctcatca atactggtc                                                    19


<210>  29
<211>  19
<212>  DNA
<213>  Artificial Sequence

<220>
<223>  Synthetic oligonucleotide

<400>  29
tgcagagcag acaagtggc                                                    19


<210>  30
<211>  19
<212>  DNA
<213>  Artificial Sequence

<220>
<223>  Synthetic oligonucleotide

<400>  30
tcatgttgac caagcaagc                                                    19


<210>  31
<211>  19
<212>  DNA
<213>  Artificial Sequence

<220>
<223>  Synthetic oligonucleotide

<400>  31
ctatgccatc accttctgc                                                    19


<210>  32
<211>  19
<212>  DNA
<213>  Artificial Sequence

<220>
<223>  Synthetic oligonucleotide

<400>  32
tgtgccgaag gatgtaagc                                                    19


<210>  33
<211>  19
<212>  DNA
<213>  Artificial Sequence

<220>
<223>  Synthetic oligonucleotide

<400>  33
ccgggacata actaaatcc                                                    19

<210>  34
<211>  19
<212>  DNA
<213>  Artificial Sequence

<220>
<223>  Synthetic oligonucleotide

<400>  34
agcaggctat gggatcaac                                                    19


<210>  35
<211>  19
<212>  DNA
<213>  Artificial Sequence

<220>
<223>  Synthetic oligonucleotide

<400>  35
ccacaaggtt gcagcattc                                                    19


<210>  36
<211>  19
<212>  DNA
<213>  Artificial Sequence

<220>
<223>  Synthetic oligonucleotide

<400>  36
gggctcagcc aggagattc                                                    19


<210>  37
<211>  19
<212>  DNA
<213>  Artificial Sequence

<220>
<223>  Synthetic oligonucleotide

<400>  37
caggtagaca tggcggcac                                                    19


<210>  38
<211>  19
<212>  DNA
<213>  Artificial Sequence

<220>
<223>  Synthetic oligonucleotide

<400>  38
ctctccagga cactgactc                                                    19


<210>  39
<211>  19
<212>  DNA
<213>  Artificial Sequence

<220>
<223>  Synthetic oligonucleotide

<400>  39
gcacgatttg gaggtcgcc                                                    19


<210>  40
<211>  19
<212>  DNA
<213>  Artificial Sequence

<220>
<223>  Synthetic oligonucleotide

<400>  40
cgctctggag tctctctcc                                                    19


<210>  41
<211>  19
<212>  DNA
<213>  Artificial Sequence

<220>
<223>  Synthetic oligonucleotide

<400>  41
ccccaactac acggagttc                                                    19


<210>  42
<211>  19
<212>  DNA
<213>  Artificial Sequence

<220>
<223>  Synthetic oligonucleotide

<400>  42
ggactctcag ttcagcatc                                                    19


<210>  43
<211>  19
<212>  DNA
<213>  Artificial Sequence

<220>
<223>  Synthetic oligonucleotide

<400>  43
ctttgcgata catgagccc                                                    19


<210>  44
<211>  19
<212>  DNA
<213>  Artificial Sequence

<220>
<223>  Synthetic oligonucleotide

<400>  44
cttcctgaag accaggttc                                                    19


<210>  45
<211>  19
<212>  DNA
<213>  Artificial Sequence

<220>
<223>  Synthetic oligonucleotide

<400>  45
gaatgctggc aacaagacc                                                    19


<210>  46
<211>  19
<212>  DNA
<213>  Artificial Sequence

<220>
<223>  Synthetic oligonucleotide

<400>  46
gaagatgtca acgcccatc                                                    19


<210>  47
<211>  19
<212>  DNA
<213>  Artificial Sequence

<220>
<223>  Synthetic oligonucleotide

<400>  47
ttctacgcac cagaactcc                                                    19


<210>  48
<211>  19
<212>  DNA
<213>  Artificial Sequence

<220>
<223>  Synthetic oligonucleotide

<400>  48
tgacggttgc atttgccac                                                    19


<210>  49
<211>  19
<212>  DNA
<213>  Artificial Sequence

<220>
<223>  Synthetic oligonucleotide

<400>  49
ccagacctcg agcaactcc                                                    19


<210>  50
<211>  19
<212>  DNA
<213>  Artificial Sequence

<220>
<223>  Synthetic oligonucleotide

<400>  50
ggagtgcaat ctggtttac                                                    19


<210>  51
<211>  19

<212>  DNA
<213>  Artificial Sequence

<220>
<223>  Synthetic oligonucleotide

<400>  51
cagtcagtgc aacagtgtc                                                    19


<210>  52
<211>  19
<212>  DNA
<213>  Artificial Sequence

<220>
<223>  Synthetic oligonucleotide

<400>  52
cgtgcttgtc atgttcggc                                                    19


<210>  53
<211>  19
<212>  DNA
<213>  Artificial Sequence

<220>
<223>  Synthetic oligonucleotide

<400>  53
tgacaggttc cgtctgggc                                                    19


<210>  54
<211>  19
<212>  DNA
<213>  Artificial Sequence

<220>
<223>  Synthetic oligonucleotide

<400>  54
gtacctgcgg cagttgttc                                                    19


<210>  55
<211>  19
<212>  DNA
<213>  Artificial Sequence

<220>
<223>  Synthetic oligonucleotide

<400>  55
gaccatcacc atcctggcc                                                    19


<210>  56
<211>  19
<212>  DNA
<213>  Artificial Sequence

<220>
<223>  Synthetic oligonucleotide

<400>  56
gcaggcagag gacagattc                                                    19

<210>  57
<211>  19
<212>  DNA
<213>  Artificial Sequence

<220>
<223>  Synthetic oligonucleotide

<400>  57
caaagccatg cccgtaagc                                                    19


<210>  58
<211>  19
<212>  DNA
<213>  Artificial Sequence

<220>
<223>  Synthetic oligonucleotide

<400>  58
aagtgccctg gccttcttc                                                    19


<210>  59
<211>  19
<212>  DNA
<213>  Artificial Sequence

<220>
<223>  Synthetic oligonucleotide

<400>  59
ctaccacaag caggtgtcc                                                    19


<210>  60
<211>  19
<212>  DNA
<213>  Artificial Sequence

<220>
<223>  Synthetic oligonucleotide

<400>  60
tgcgtgcttc tttgtgggc                                                    19


<210>  61
<211>  19
<212>  DNA
<213>  Artificial Sequence

<220>
<223>  Synthetic oligonucleotide

<400>  61
ctacgtcatc ctgtgcctc                                                    19


<210>  62
<211>  19
<212>  DNA
<213>  Artificial Sequence

<220>
<223>  Synthetic oligonucleotide

<400>  62
ttcggacacc cacaaatgc                                                    19


<210>  63
<211>  19
<212>  DNA
<213>  Artificial Sequence

<220>
<223>  Synthetic oligonucleotide

<400>  63
gttgtcctgt tctgacgtc                                                    19


<210>  64
<211>  19
<212>  DNA
<213>  Artificial Sequence

<220>
<223>  Synthetic oligonucleotide

<400>  64
gcaggctttc gagtatggc                                                    19


<210>  65
<211>  19
<212>  DNA
<213>  Artificial Sequence

<220>
<223>  Synthetic oligonucleotide

<400>  65
cctctctgtc aacacatgc                                                    19


<210>  66
<211>  19
<212>  DNA
<213>  Artificial Sequence

<220>
<223>  Synthetic oligonucleotide

<400>  66
atgacgctac gcaagaacc                                                    19


<210>  67
<211>  19
<212>  DNA
<213>  Artificial Sequence

<220>
<223>  Synthetic oligonucleotide

<400>  67
agcgggtgct tacattgcc                                                    19


<210>  68
<211>  19
<212>  DNA
<213>  Artificial Sequence

<220>
<223>  Synthetic oligonucleotide

<400>  68
gcaagaatgc acagagagc                                                    19


<210>  69
<211>  19
<212>  DNA
<213>  Artificial Sequence

<220>
<223>  Synthetic oligonucleotide

<400>  69
tttgtgctga cagctgctc                                                    19


<210>  70
<211>  19
<212>  DNA
<213>  Artificial Sequence

<220>
<223>  Synthetic oligonucleotide

<400>  70
cacctgcttt ctcaatgcc                                                    19


<210>  71
<211>  19
<212>  DNA
<213>  Artificial Sequence

<220>
<223>  Synthetic oligonucleotide

<400>  71
ggttctctga ctcgaacac                                                    19


<210>  72
<211>  19
<212>  DNA
<213>  Artificial Sequence

<220>
<223>  Synthetic oligonucleotide

<400>  72
agcacctcgc tgacattcc                                                    19


<210>  73
<211>  19
<212>  DNA
<213>  Artificial Sequence

<220>
<223>  Synthetic oligonucleotide

<400>  73
cctgggcaac acctgctac                                                    19


<210>  74
<211>  19

<212>  DNA
<213>  Artificial Sequence

<220>
<223>  Synthetic oligonucleotide

<400>  74
cttaaggtgg acctgtggc                                                    19


<210>  75
<211>  19
<212>  DNA
<213>  Artificial Sequence

<220>
<223>  Synthetic oligonucleotide

<400>  75
ttgcacagaa aggaggtgc                                                    19


<210>  76
<211>  19
<212>  DNA
<213>  Artificial Sequence

<220>
<223>  Synthetic oligonucleotide

<400>  76
gcccgcagtc ttacacaac                                                    19


<210>  77
<211>  19
<212>  DNA
<213>  Artificial Sequence

<220>
<223>  Synthetic oligonucleotide

<400>  77
gcttctggtg gagaaggac                                                    19


<210>  78
<211>  19
<212>  DNA
<213>  Artificial Sequence

<220>
<223>  Synthetic oligonucleotide

<400>  78
gtgtatgaag tggtccacc                                                    19


<210>  79
<211>  19
<212>  DNA
<213>  Artificial Sequence

<220>
<223>  Synthetic oligonucleotide

<400>  79
gaaatctcac tgcttcgac                                                    19

<210>  80
<211>  19
<212>  DNA
<213>  Artificial Sequence

<220>
<223>  Synthetic oligonucleotide

<400>  80
cactttataa gcctgcggc                                                    19


<210>  81
<211>  19
<212>  DNA
<213>  Artificial Sequence

<220>
<223>  Synthetic oligonucleotide

<400>  81
tgggcatcct ggctgtaac                                                    19


<210>  82
<211>  19
<212>  DNA
<213>  Artificial Sequence

<220>
<223>  Synthetic oligonucleotide

<400>  82
atagactgca gaatgagcc                                                    19


<210>  83
<211>  19
<212>  DNA
<213>  Artificial Sequence

<220>
<223>  Synthetic oligonucleotide

<400>  83
cagtgccaag aaggagccc                                                    19


<210>  84
<211>  19
<212>  DNA
<213>  Artificial Sequence

<220>
<223>  Synthetic oligonucleotide

<400>  84
tgtgctcgaa ggaggaatc                                                    19


<210>  85
<211>  19
<212>  DNA
<213>  Artificial Sequence

<220>
<223>  Synthetic oligonucleotide

<400>  85
atgtatggat tccagcacc                                                    19


<210>  86
<211>  19
<212>  DNA
<213>  Artificial Sequence

<220>
<223>  Synthetic oligonucleotide

<400>  86
gctctgctat gtgtcagtc                                                    19


<210>  87
<211>  19
<212>  DNA
<213>  Artificial Sequence

<220>
<223>  Synthetic oligonucleotide

<400>  87
tgtgactaag ctggaagac                                                    19


<210>  88
<211>  19
<212>  DNA
<213>  Artificial Sequence

<220>
<223>  Synthetic oligonucleotide

<400>  88
tgcgcacaat actggccac                                                    19


<210>  89
<211>  19
<212>  DNA
<213>  Artificial Sequence

<220>
<223>  Synthetic oligonucleotide

<400>  89
ttcccgctac cagacctac                                                    19


<210>  90
<211>  19
<212>  DNA
<213>  Artificial Sequence

<220>
<223>  Synthetic oligonucleotide

<400>  90
agcatggtca ttcacatcc                                                    19


<210>  91
<211>  19
<212>  DNA
<213>  Artificial Sequence

<220>
<223>  Synthetic oligonucleotide

<400>  91
atgcaggtcc atatgtgac                                                    19


<210>  92
<211>  19
<212>  DNA
<213>  Artificial Sequence

<220>
<223>  Synthetic oligonucleotide

<400>  92
cctttctctg aacacggac                                                    19


<210>  93
<211>  19
<212>  DNA
<213>  Artificial Sequence

<220>
<223>  Synthetic oligonucleotide

<400>  93
gtcaggctga atgaggagc                                                    19


<210>  94
<211>  19
<212>  DNA
<213>  Artificial Sequence

<220>
<223>  Synthetic oligonucleotide

<400>  94
caggttctcc tcaaacggc                                                    19


<210>  95
<211>  19
<212>  DNA
<213>  Artificial Sequence

<220>
<223>  Synthetic oligonucleotide

<400>  95
gcataggagt ggtcatatc                                                    19


<210>  96
<211>  19
<212>  DNA
<213>  Artificial Sequence

<220>
<223>  Synthetic oligonucleotide

<400>  96
acatcctgct gtcagagcc                                                    19


<210>  97
<211>  19

<212>  DNA
<213>  Artificial Sequence

<220>
<223>  Synthetic oligonucleotide

<400>  97
catggagttc agcaagatc                                                    19


<210>  98
<211>  19
<212>  DNA
<213>  Artificial Sequence

<220>
<223>  Synthetic oligonucleotide

<400>  98
gttctccagt gccattggc                                                    19


<210>  99
<211>  19
<212>  DNA
<213>  Artificial Sequence

<220>
<223>  Synthetic oligonucleotide

<400>  99
ttcggcctgg acgtctatc                                                    19


<210>  100
<211>  19
<212>  DNA
<213>  Artificial Sequence

<220>
<223>  Synthetic oligonucleotide

<400>  100
gtgcattggt gaaagagac                                                    19


<210>  101
<211>  19
<212>  DNA
<213>  Artificial Sequence

<220>
<223>  Synthetic oligonucleotide

<400>  101
tgtgcaggat aattgctgc                                                    19


<210>  102
<211>  19
<212>  DNA
<213>  Artificial Sequence

<220>
<223>  Synthetic oligonucleotide

<400>  102
aggtgtcact gtacagtcc                                                    19

<210>  103
<211>  19
<212>  DNA
<213>  Artificial Sequence

<220>
<223>  Synthetic oligonucleotide

<400>  103
agtgcgcatc ttcggcctc                                                    19


<210>  104
<211>  19
<212>  DNA
<213>  Artificial Sequence

<220>
<223>  Synthetic oligonucleotide

<400>  104
tttgtggact cctacgatc                                                    19


<210>  105
<211>  19
<212>  DNA
<213>  Artificial Sequence

<220>
<223>  Synthetic oligonucleotide

<400>  105
gccctgatgt ccatcttcc                                                    19


<210>  106
<211>  19
<212>  DNA
<213>  Artificial Sequence

<220>
<223>  Synthetic oligonucleotide

<400>  106
catagggaag gacacttgc                                                    19


<210>  107
<211>  19
<212>  DNA
<213>  Artificial Sequence

<220>
<223>  Synthetic oligonucleotide

<400>  107
cctggatgtg agagagagc                                                    19


<210>  108
<211>  19
<212>  DNA
<213>  Artificial Sequence

<220>
<223>  Synthetic oligonucleotide

<400>  108
aacttgtact atgaaggcc                                                    19


<210>  109
<211>  19
<212>  DNA
<213>  Artificial Sequence

<220>
<223>  Synthetic oligonucleotide

<400>  109
gtattctgta caccctggc                                                    19


<210>  110
<211>  19
<212>  DNA
<213>  Artificial Sequence

<220>
<223>  Synthetic oligonucleotide

<400>  110
ttctcgcaat ggccaatgc                                                    19


<210>  111
<211>  19
<212>  DNA
<213>  Artificial Sequence

<220>
<223>  Synthetic oligonucleotide

<400>  111
gaagaacagc agcctggac                                                    19


<210>  112
<211>  19
<212>  DNA
<213>  Artificial Sequence

<220>
<223>  Synthetic oligonucleotide

<400>  112
tcaggcggat cttgacagc                                                    19


<210>  113
<211>  19
<212>  DNA
<213>  Artificial Sequence

<220>
<223>  Synthetic oligonucleotide

<400>  113
ttcggcactg gtttccctc                                                    19


<210>  114
<211>  19
<212>  DNA
<213>  Artificial Sequence

<220>
<223>  Synthetic oligonucleotide

<400>  114
gacgaatatc agctctgcc                                                    19


<210>  115
<211>  19
<212>  DNA
<213>  Artificial Sequence

<220>
<223>  Synthetic oligonucleotide

<400>  115
cctctctacg ccatctacc                                                    19


<210>  116
<211>  19
<212>  DNA
<213>  Artificial Sequence

<220>
<223>  Synthetic oligonucleotide

<400>  116
tctctccaca caaaccttc                                                    19


<210>  117
<211>  19
<212>  DNA
<213>  Artificial Sequence

<220>
<223>  Synthetic oligonucleotide

<400>  117
ggtcgggagg agaacagtc                                                    19


<210>  118
<211>  19
<212>  DNA
<213>  Artificial Sequence

<220>
<223>  Synthetic oligonucleotide

<400>  118
gcgaattcca ccagcattc                                                    19


<210>  119
<211>  19
<212>  DNA
<213>  Artificial Sequence

<220>
<223>  Synthetic oligonucleotide

<400>  119
tgtccaggac ctattgagc                                                    19


<210>  120
<211>  19

<212>  DNA
<213>  Artificial Sequence

<220>
<223>  Synthetic oligonucleotide

<400>  120
tgaccatctg catgatgtc                                                    19


<210>  121
<211>  19
<212>  DNA
<213>  Artificial Sequence

<220>
<223>  Synthetic oligonucleotide

<400>  121
gcatggcata gttggattc                                                    19


<210>  122
<211>  19
<212>  DNA
<213>  Artificial Sequence

<220>
<223>  Synthetic oligonucleotide

<400>  122
atgcaggaca tgaacaacc                                                    19


<210>  123
<211>  19
<212>  DNA
<213>  Artificial Sequence

<220>
<223>  Synthetic oligonucleotide

<400>  123
atgtgtgaat gtgggttgc                                                    19


<210>  124
<211>  19
<212>  DNA
<213>  Artificial Sequence

<220>
<223>  Synthetic oligonucleotide

<400>  124
tgcaggctat tctgtgagc                                                    19


<210>  125
<211>  19
<212>  DNA
<213>  Artificial Sequence

<220>
<223>  Synthetic oligonucleotide

<400>  125
tcggcacaac aatctagac                                                    19

<210>  126
<211>  19
<212>  DNA
<213>  Artificial Sequence

<220>
<223>  Synthetic oligonucleotide

<400>  126
gacaggtgga cagagcatc                                                    19


<210>  127
<211>  19
<212>  DNA
<213>  Artificial Sequence

<220>
<223>  Synthetic oligonucleotide

<400>  127
tgtgcgagac ctcgatttc                                                    19


<210>  128
<211>  19
<212>  DNA
<213>  Artificial Sequence

<220>
<223>  Synthetic oligonucleotide

<400>  128
agtttgtgta ggtatcgcc                                                    19


<210>  129
<211>  19
<212>  DNA
<213>  Artificial Sequence

<220>
<223>  Synthetic oligonucleotide

<400>  129
agcatgaaag aaaccctgc                                                    19


<210>  130
<211>  19
<212>  DNA
<213>  Artificial Sequence

<220>
<223>  Synthetic oligonucleotide

<400>  130
gaagatcacc attgctgac                                                    19


<210>  131
<211>  19
<212>  DNA
<213>  Artificial Sequence

<220>
<223>  Synthetic oligonucleotide

<400>  131
gcatgaatat tgtggaggc                                                    19


<210>  132
<211>  19
<212>  DNA
<213>  Artificial Sequence

<220>
<223>  Synthetic oligonucleotide

<400>  132
tgcaggcaag cagacggtc                                                    19


<210>  133
<211>  19
<212>  DNA
<213>  Artificial Sequence

<220>
<223>  Synthetic oligonucleotide

<400>  133
tgacgcagat gatgaatcc                                                    19


<210>  134
<211>  19
<212>  DNA
<213>  Artificial Sequence

<220>
<223>  Synthetic oligonucleotide

<400>  134
tggcgccatg tcagagtgc                                                    19


<210>  135
<211>  19
<212>  DNA
<213>  Artificial Sequence

<220>
<223>  Synthetic oligonucleotide

<400>  135
cttattgttc acattggcc                                                    19


<210>  136
<211>  19
<212>  DNA
<213>  Artificial Sequence

<220>
<223>  Synthetic oligonucleotide

<400>  136
tggcacctat gagaggatc                                                    19


<210>  137
<211>  19
<212>  DNA
<213>  Artificial Sequence

<220>
<223>  Synthetic oligonucleotide

<400>  137
tcaggtgtcc cattccagc                                                    19


<210>  138
<211>  19
<212>  DNA
<213>  Artificial Sequence

<220>
<223>  Synthetic oligonucleotide

<400>  138
ctcagaggtg gtggaagac                                                    19


<210>  139
<211>  19
<212>  DNA
<213>  Artificial Sequence

<220>
<223>  Synthetic oligonucleotide

<400>  139
tgcaggagga aattgatgc                                                    19


<210>  140
<211>  19
<212>  DNA
<213>  Artificial Sequence

<220>
<223>  Synthetic oligonucleotide

<400>  140
gagtccagcc ttcatgccc                                                    19


<210>  141
<211>  19
<212>  DNA
<213>  Artificial Sequence

<220>
<223>  Synthetic oligonucleotide

<400>  141
gtccagctga agaagatcc                                                    19


<210>  142
<211>  19
<212>  DNA
<213>  Artificial Sequence

<220>
<223>  Synthetic oligonucleotide

<400>  142
ttcggcactg aggtcttgc                                                    19


<210>  143
<211>  19

<212>  DNA
<213>  Artificial Sequence

<220>
<223>  Synthetic oligonucleotide

<400>  143
ttgcacactg agctggagc                                                    19


<210>  144
<211>  19
<212>  DNA
<213>  Artificial Sequence

<220>
<223>  Synthetic oligonucleotide

<400>  144
cctgctcttg agcaataac                                                    19


<210>  145
<211>  19
<212>  DNA
<213>  Artificial Sequence

<220>
<223>  Synthetic oligonucleotide

<400>  145
tgtccagacc acatggagc                                                    19


<210>  146
<211>  19
<212>  DNA
<213>  Artificial Sequence

<220>
<223>  Synthetic oligonucleotide

<400>  146
ttgcaggagg acaagatgc                                                    19


<210>  147
<211>  19
<212>  DNA
<213>  Artificial Sequence

<220>
<223>  Synthetic oligonucleotide

<400>  147
gattgtggcc aagaagtac                                                    19


<210>  148
<211>  19
<212>  DNA
<213>  Artificial Sequence

<220>
<223>  Synthetic oligonucleotide

<400>  148
cctcattatc accatgctc                                                    19

<210>  149
<211>  19
<212>  DNA
<213>  Artificial Sequence

<220>
<223>  Synthetic oligonucleotide

<400>  149
ctggttattg gcgggtatc                                                    19


<210>  150
<211>  19
<212>  DNA
<213>  Artificial Sequence

<220>
<223>  Synthetic oligonucleotide

<400>  150
tttgtgcttc accaagtgc                                                    19


<210>  151
<211>  19
<212>  DNA
<213>  Artificial Sequence

<220>
<223>  Synthetic oligonucleotide

<400>  151
ctgcacagtc aacacggtc                                                    19


<210>  152
<211>  19
<212>  DNA
<213>  Artificial Sequence

<220>
<223>  Synthetic oligonucleotide

<400>  152
gcagagccaa atatcagcc                                                    19


<210>  153
<211>  19
<212>  DNA
<213>  Artificial Sequence

<220>
<223>  Synthetic oligonucleotide

<400>  153
gttcaagaag ctgcgccac                                                    19


<210>  154
<211>  19
<212>  DNA
<213>  Artificial Sequence

<220>
<223>  Synthetic oligonucleotide

<400>  154
cgtcttcatc agcgtgcac                                                    19


<210>  155
<211>  19
<212>  DNA
<213>  Artificial Sequence

<220>
<223>  Synthetic oligonucleotide

<400>  155
gcagttccaa gcttgcatc                                                    19


<210>  156
<211>  19
<212>  DNA
<213>  Artificial Sequence

<220>
<223>  Synthetic oligonucleotide

<400>  156
gtacctgcgc cacttcttc                                                    19


<210>  157
<211>  19
<212>  DNA
<213>  Artificial Sequence

<220>
<223>  Synthetic oligonucleotide

<400>  157
gaccatcact atcctggcc                                                    19


<210>  158
<211>  19
<212>  DNA
<213>  Artificial Sequence

<220>
<223>  Synthetic oligonucleotide

<400>  158
gtccttctac atcaatgcc                                                    19


<210>  159
<211>  19
<212>  DNA
<213>  Artificial Sequence

<220>
<223>  Synthetic oligonucleotide

<400>  159
gaagaagcaa ctgggagcc                                                    19


<210>  160
<211>  19
<212>  DNA
<213>  Artificial Sequence

<220>
<223>  Synthetic oligonucleotide

<400>  160
gttccagacc ttcatgtgc                                                    19


<210>  161
<211>  19
<212>  DNA
<213>  Artificial Sequence

<220>
<223>  Synthetic oligonucleotide

<400>  161
ctggaccgag ctgcttgac                                                    19


<210>  162
<211>  19
<212>  DNA
<213>  Artificial Sequence

<220>
<223>  Synthetic oligonucleotide

<400>  162
gcagagcatg ggcttaagc                                                    19


<210>  163
<211>  19
<212>  DNA
<213>  Artificial Sequence

<220>
<223>  Synthetic oligonucleotide

<400>  163
agcaggcgga acatgttcc                                                    19


<210>  164
<211>  19
<212>  DNA
<213>  Artificial Sequence

<220>
<223>  Synthetic oligonucleotide

<400>  164
caacctgttc atccttaac                                                    19


<210>  165
<211>  19
<212>  DNA
<213>  Artificial Sequence

<220>
<223>  Synthetic oligonucleotide

<400>  165
atttggtgac acggctgac                                                    19


<210>  166
<211>  19

<212>  DNA
<213>  Artificial Sequence

<220>
<223>  Synthetic oligonucleotide

<400>  166
catatggctc ttcaagtac                                                    19


<210>  167
<211>  19
<212>  DNA
<213>  Artificial Sequence

<220>
<223>  Synthetic oligonucleotide

<400>  167
tggcacagta acagctgcc                                                    19


<210>  168
<211>  19
<212>  DNA
<213>  Artificial Sequence

<220>
<223>  Synthetic oligonucleotide

<400>  168
gttctctcag cacgttcgc                                                    19


<210>  169
<211>  19
<212>  DNA
<213>  Artificial Sequence

<220>
<223>  Synthetic oligonucleotide

<400>  169
tgcagacttc agtgcagcc                                                    19


<210>  170
<211>  19
<212>  DNA
<213>  Artificial Sequence

<220>
<223>  Synthetic oligonucleotide

<400>  170
ctttctggaa atcctgccc                                                    19


<210>  171
<211>  19
<212>  DNA
<213>  Artificial Sequence

<220>
<223>  Synthetic oligonucleotide

<400>  171
caacacagca gacacagtc                                                    19

<210>  172
<211>  19
<212>  DNA
<213>  Artificial Sequence

<220>
<223>  Synthetic oligonucleotide

<400>  172
tgggaccagt gactgcagc                                                    19


<210>  173
<211>  19
<212>  DNA
<213>  Artificial Sequence

<220>
<223>  Synthetic oligonucleotide

<400>  173
agccagagga gtttgtggc                                                    19


<210>  174
<211>  19
<212>  DNA
<213>  Artificial Sequence

<220>
<223>  Synthetic oligonucleotide

<400>  174
ctcgcaggaa attctggac                                                    19


<210>  175
<211>  19
<212>  DNA
<213>  Artificial Sequence

<220>
<223>  Synthetic oligonucleotide

<400>  175
atgcaggtca ggttgtttc                                                    19


<210>  176
<211>  19
<212>  DNA
<213>  Artificial Sequence

<220>
<223>  Synthetic oligonucleotide

<400>  176
atcaggcctt acatccagc                                                    19


<210>  177
<211>  19
<212>  DNA
<213>  Artificial Sequence

<220>
<223>  Synthetic oligonucleotide

<400>  177
ctgtgggaga aacagagac                                                    19


<210>  178
<211>  19
<212>  DNA
<213>  Artificial Sequence

<220>
<223>  Synthetic oligonucleotide

<400>  178
ctctgcgact actacctgc                                                    19


<210>  179
<211>  19
<212>  DNA
<213>  Artificial Sequence

<220>
<223>  Synthetic oligonucleotide

<400>  179
ctgatgaagg ccttcgacc                                                    19


<210>  180
<211>  19
<212>  DNA
<213>  Artificial Sequence

<220>
<223>  Synthetic oligonucleotide

<400>  180
accgtggaag gcctatcgc                                                    19


<210>  181
<211>  19
<212>  DNA
<213>  Artificial Sequence

<220>
<223>  Synthetic oligonucleotide

<400>  181
ttccagctga tatatccac                                                    19


<210>  182
<211>  19
<212>  DNA
<213>  Artificial Sequence

<220>
<223>  Synthetic oligonucleotide

<400>  182
gccagacact gacaagttc                                                    19


<210>  183
<211>  19
<212>  DNA
<213>  Artificial Sequence

<220>
<223>  Synthetic oligonucleotide

<400>  183
tcggcagggc cagcatttc                                                    19


<210>  184
<211>  19
<212>  DNA
<213>  Artificial Sequence

<220>
<223>  Synthetic oligonucleotide

<400>  184
gtcttcgacc ctcaggaac                                                    19


<210>  185
<211>  19
<212>  DNA
<213>  Artificial Sequence

<220>
<223>  Synthetic oligonucleotide

<400>  185
tcagaaggtt gtgcaggac                                                    19


<210>  186
<211>  19
<212>  DNA
<213>  Artificial Sequence

<220>
<223>  Synthetic oligonucleotide

<400>  186
caacttgcat gactacggc                                                    19


<210>  187
<211>  19
<212>  DNA
<213>  Artificial Sequence

<220>
<223>  Synthetic oligonucleotide

<400>  187
tgtgcaagag gttcgttgc                                                    19


<210>  188
<211>  19
<212>  DNA
<213>  Artificial Sequence

<220>
<223>  Synthetic oligonucleotide

<400>  188
accagtggta aatgtcagc                                                    19


<210>  189
<211>  19

<212>  DNA
<213>  Artificial Sequence

<220>
<223>  Synthetic oligonucleotide

<400>  189
ctctgtatcc cattccctc                                                    19


<210>  190
<211>  19
<212>  DNA
<213>  Artificial Sequence

<220>
<223>  Synthetic oligonucleotide

<400>  190
cttcttgaag catctatgc                                                    19


<210>  191
<211>  19
<212>  DNA
<213>  Artificial Sequence

<220>
<223>  Synthetic oligonucleotide

<400>  191
catgctgttg tcctttacc                                                    19


<210>  192
<211>  19
<212>  DNA
<213>  Artificial Sequence

<220>
<223>  Synthetic oligonucleotide

<400>  192
cgaagatgtt gacctggtc                                                    19


<210>  193
<211>  19
<212>  DNA
<213>  Artificial Sequence

<220>
<223>  Synthetic oligonucleotide

<400>  193
tgtcctgttg gatgcacac                                                    19


<210>  194
<211>  19
<212>  DNA
<213>  Artificial Sequence

<220>
<223>  Synthetic oligonucleotide

<400>  194
atgcagaccc tggaccaac                                                    19

<210>  195
<211>  19
<212>  DNA
<213>  Artificial Sequence

<220>
<223>  Synthetic oligonucleotide

<400>  195
gtagcactct gcgacatgc                                                    19


<210>  196
<211>  19
<212>  DNA
<213>  Artificial Sequence

<220>
<223>  Synthetic oligonucleotide

<400>  196
ctggaatggg atccgacac                                                    19


<210>  197
<211>  19
<212>  DNA
<213>  Artificial Sequence

<220>
<223>  Synthetic oligonucleotide

<400>  197
gttattcttc caccatggc                                                    19


<210>  198
<211>  19
<212>  DNA
<213>  Artificial Sequence

<220>
<223>  Synthetic oligonucleotide

<400>  198
tgccagcagt ctcttgatc                                                    19


<210>  199
<211>  19
<212>  DNA
<213>  Artificial Sequence

<220>
<223>  Synthetic oligonucleotide

<400>  199
gatcttccgg cccaaacac                                                    19


<210>  200
<211>  19
<212>  DNA
<213>  Artificial Sequence

<220>
<223>  Synthetic oligonucleotide

<400>  200
agcatgacag gaaacctgc                                                    19


<210>  201
<211>  19
<212>  DNA
<213>  Artificial Sequence

<220>
<223>  Synthetic oligonucleotide

<400>  201
tcttctctgt gaacatacc                                                    19


<210>  202
<211>  19
<212>  DNA
<213>  Artificial Sequence

<220>
<223>  Synthetic oligonucleotide

<400>  202
ccttgttggc caatgattc                                                    19


<210>  203
<211>  19
<212>  DNA
<213>  Artificial Sequence

<220>
<223>  Synthetic oligonucleotide

<400>  203
ccaggtttgt caacgtgac                                                    19


<210>  204
<211>  19
<212>  DNA
<213>  Artificial Sequence

<220>
<223>  Synthetic oligonucleotide

<400>  204
ttccagctca gtgctaatc                                                    19


<210>  205
<211>  19
<212>  DNA
<213>  Artificial Sequence

<220>
<223>  Synthetic oligonucleotide

<400>  205
agcaggaaca tctcttcgc                                                    19


<210>  206
<211>  19
<212>  DNA
<213>  Artificial Sequence

<220>
<223>  Synthetic oligonucleotide

<400>  206
caagttctcc tgcaagttc                                                    19


<210>  207
<211>  19
<212>  DNA
<213>  Artificial Sequence

<220>
<223>  Synthetic oligonucleotide

<400>  207
gagcatgacc ttcgatggc                                                    19


<210>  208
<211>  19
<212>  DNA
<213>  Artificial Sequence

<220>
<223>  Synthetic oligonucleotide

<400>  208
ctggcagaag cagcacaac                                                    19


<210>  209
<211>  19
<212>  DNA
<213>  Artificial Sequence

<220>
<223>  Synthetic oligonucleotide

<400>  209
tacctgctgg gcatcaagc                                                    19


<210>  210
<211>  19
<212>  DNA
<213>  Artificial Sequence

<220>
<223>  Synthetic oligonucleotide

<400>  210
ctggaagtcc tgtgtgatc                                                    19


<210>  211
<211>  19
<212>  DNA
<213>  Artificial Sequence

<220>
<223>  Synthetic oligonucleotide

<400>  211
ggacaccttc ccaaatgtc                                                    19


<210>  212
<211>  19

<212>  DNA
<213>  Artificial Sequence

<220>
<223>  Synthetic oligonucleotide

<400>  212
ggcagaaatt ccagagagc                                                    19


<210>  213
<211>  19
<212>  DNA
<213>  Artificial Sequence

<220>
<223>  Synthetic oligonucleotide

<400>  213
caacatccca actgtggtc                                                    19


<210>  214
<211>  19
<212>  DNA
<213>  Artificial Sequence

<220>
<223>  Synthetic oligonucleotide

<400>  214
tatcctgacc ttcctgcgc                                                    19


<210>  215
<211>  19
<212>  DNA
<213>  Artificial Sequence

<220>
<223>  Synthetic oligonucleotide

<400>  215
tgtttaccag tccgaagcc                                                    19


<210>  216
<211>  19
<212>  DNA
<213>  Artificial Sequence

<220>
<223>  Synthetic oligonucleotide

<400>  216
aactgtaccg cagagttcc                                                    19


<210>  217
<211>  19
<212>  DNA
<213>  Artificial Sequence

<220>
<223>  Synthetic oligonucleotide

<400>  217
agcatgatga tagttcctc                                                    19

<210>  218
<211>  19
<212>  DNA
<213>  Artificial Sequence

<220>
<223>  Synthetic oligonucleotide

<400>  218
caagtgccgt atcatccac                                                    19


<210>  219
<211>  19
<212>  DNA
<213>  Artificial Sequence

<220>
<223>  Synthetic oligonucleotide

<400>  219
tattcgtgcg gaggaagac                                                    19


<210>  220
<211>  19
<212>  DNA
<213>  Artificial Sequence

<220>
<223>  Synthetic oligonucleotide

<400>  220
gtacctgctg gtggagttc                                                    19


<210>  221
<211>  20
<212>  DNA
<213>  Artificial Sequence

<220>
<223>  Synthetic oligonucleotide

<400>  221
catggcgacc tgaaggaaag                                                   20


<210>  222
<211>  23
<212>  DNA
<213>  Artificial Sequence

<220>
<223>  Synthetic oligonucleotide

<400>  222
agagaccaaa agaccctgga agt                                               23


<210>  223
<211>  18
<212>  DNA
<213>  Artificial Sequence

<220>
<223>  Synthetic oligonucleotide

<400>  223
acagcggcgg cttctaca                                                    18


<210>  224
<211>  21
<212>  DNA
<213>  Artificial Sequence

<220>
<223>  Synthetic oligonucleotide

<400>  224
catcggcgtg tttggagtag t                                                21


<210>  225
<211>  22
<212>  DNA
<213>  Artificial Sequence

<220>
<223>  Synthetic oligonucleotide

<400>  225
atccggatca cctgtttgaa ac                                               22


<210>  226
<211>  23
<212>  DNA
<213>  Artificial Sequence

<220>
<223>  Synthetic oligonucleotide

<400>  226
gtggtgattt tgtccttctc gat                                              23


<210>  227
<211>  23
<212>  DNA
<213>  Artificial Sequence

<220>
<223>  Synthetic oligonucleotide

<400>  227
ccatacacag agggaagcat acg                                              23


<210>  228
<211>  24
<212>  DNA
<213>  Artificial Sequence

<220>
<223>  Synthetic oligonucleotide

<400>  228
cagtcttgct gtgatctggg agta                                             24


<210>  229
<211>  20
<212>  DNA
<213>  Artificial Sequence

<220>
<223>  Synthetic oligonucleotide

<400>  229
gcataggcga tgaggagcat                                                    20


<210>  230
<211>  19
<212>  DNA
<213>  Artificial Sequence

<220>
<223>  Synthetic oligonucleotide

<400>  230
gacctcggcc tgcattagg                                                     19


<210>  231
<211>  21
<212>  DNA
<213>  Artificial Sequence

<220>
<223>  Synthetic oligonucleotide

<400>  231
catcgtcgct ctgcaacttt t                                                  21


<210>  232
<211>  24
<212>  DNA
<213>  Artificial Sequence

<220>
<223>  Synthetic oligonucleotide

<400>  232
ccgctctgta cttctttgaa atga                                               24


<210>  233
<211>  23
<212>  DNA
<213>  Artificial Sequence

<220>
<223>  Synthetic oligonucleotide

<400>  233
cctggtggag accttgtaaa cct                                                23


<210>  234
<211>  25
<212>  DNA
<213>  Artificial Sequence

<220>
<223>  Synthetic oligonucleotide

<400>  234
agcaagaaca acttcagcag tgtaa                                              25


<210>  235
<211>  22

<212>  DNA
<213>  Artificial Sequence

<220>
<223>  Synthetic oligonucleotide

<400>  235
gccatgacca gctttcacta ca                                                22


<210>  236
<211>  21
<212>  DNA
<213>  Artificial Sequence

<220>
<223>  Synthetic oligonucleotide

<400>  236
gttaaggcag gccctcaggt a                                                 21


<210>  237
<211>  22
<212>  DNA
<213>  Artificial Sequence

<220>
<223>  Synthetic oligonucleotide

<400>  237
ctaaccgtgc tggcctatga ac                                                22


<210>  238
<211>  21
<212>  DNA
<213>  Artificial Sequence

<220>
<223>  Synthetic oligonucleotide

<400>  238
caggcccagg aaaaattgat c                                                 21


<210>  239
<211>  19
<212>  DNA
<213>  Artificial Sequence

<220>
<223>  Synthetic oligonucleotide

<400>  239
ttcgcgatca gccccttac                                                    19


<210>  240
<211>  22
<212>  DNA
<213>  Artificial Sequence

<220>
<223>  Synthetic oligonucleotide

<400>  240
actcactggc tgaggaggtc at                                                22

<210>  241
<211>  21
<212>  DNA
<213>  Artificial Sequence

<220>
<223>  Synthetic oligonucleotide

<400>  241
ttccagacca ttggcttgag t                                                21


<210>  242
<211>  21
<212>  DNA
<213>  Artificial Sequence

<220>
<223>  Synthetic oligonucleotide

<400>  242
actcccactg actttcctgc t                                                21


<210>  243
<211>  25
<212>  DNA
<213>  Artificial Sequence

<220>
<223>  Synthetic oligonucleotide

<400>  243
catgctgagt gacacagaca agaag                                            25


<210>  244
<211>  25
<212>  DNA
<213>  Artificial Sequence

<220>
<223>  Synthetic oligonucleotide

<400>  244
tggtagttgt tgtgagcata gtcca                                            25


<210>  245
<211>  21
<212>  DNA
<213>  Artificial Sequence

<220>
<223>  Synthetic oligonucleotide

<400>  245
ccgtttacgt ggagactcgc c                                                21


<210>  246
<211>  25
<212>  DNA
<213>  Artificial Sequence

<220>
<223>  Synthetic oligonucleotide

<400>  246
cccccacctt atatatattc tttcc                                          25


<210>  247
<211>  19
<212>  DNA
<213>  Artificial Sequence

<220>
<223>  Synthetic oligonucleotide

<400>  247
cgttggcaga atcatttac                                                 19


<210>  248
<211>  19
<212>  DNA
<213>  Artificial Sequence

<220>
<223>  Synthetic oligonucleotide

<400>  248
ttcgcggatg atgtacttc                                                 19


<210>  249
<211>  19
<212>  DNA
<213>  Artificial Sequence

<220>
<223>  Synthetic oligonucleotide

<400>  249
ctctcagtac tttaacatc                                                 19


<210>  250
<211>  19
<212>  DNA
<213>  Artificial Sequence

<220>
<223>  Synthetic oligonucleotide

<400>  250
cataacaaag gcatcgccc                                                 19


<210>  251
<211>  19
<212>  DNA
<213>  Artificial Sequence

<220>
<223>  Synthetic oligonucleotide

<400>  251
ttaatgatag ccatccagc                                                 19


<210>  252
<211>  19
<212>  DNA
<213>  Artificial Sequence

<220>
<223>  Synthetic oligonucleotide

<400>  252
attccaggcc atttatggc                                                    19


<210>  253
<211>  19
<212>  DNA
<213>  Artificial Sequence

<220>
<223>  Synthetic oligonucleotide

<400>  253
tgttagacta cgtgatgac                                                    19


<210>  254
<211>  19
<212>  DNA
<213>  Artificial Sequence

<220>
<223>  Synthetic oligonucleotide

<400>  254
caacctcacc tacaggatc                                                    19


<210>  255
<211>  19
<212>  DNA
<213>  Artificial Sequence

<220>
<223>  Synthetic oligonucleotide

<400>  255
tctacccgat caaccacac                                                    19


<210>  256
<211>  19
<212>  DNA
<213>  Artificial Sequence

<220>
<223>  Synthetic oligonucleotide

<400>  256
cccttggagg aactatgcc                                                    19


<210>  257
<211>  19
<212>  DNA
<213>  Artificial Sequence

<220>
<223>  Synthetic oligonucleotide

<400>  257
caactcagac aactgcatc                                                    19


<210>  258
<211>  19

<212>  DNA
<213>  Artificial Sequence

<220>
<223>  Synthetic oligonucleotide

<400>  258
tcattgtaag gctgtggcc                                                    19


<210>  259
<211>  19
<212>  DNA
<213>  Artificial Sequence

<220>
<223>  Synthetic oligonucleotide

<400>  259
ggaccaatgg gagatagac                                                    19


<210>  260
<211>  19
<212>  DNA
<213>  Artificial Sequence

<220>
<223>  Synthetic oligonucleotide

<400>  260
agcttatcca gctttatgc                                                    19


<210>  261
<211>  19
<212>  DNA
<213>  Artificial Sequence

<220>
<223>  Synthetic oligonucleotide

<400>  261
gtgcattaac aagaagaac                                                    19


<210>  262
<211>  19
<212>  DNA
<213>  Artificial Sequence

<220>
<223>  Synthetic oligonucleotide

<400>  262
acctgcagcc ttcactttc                                                    19


<210>  263
<211>  19
<212>  DNA
<213>  Artificial Sequence

<220>
<223>  Synthetic oligonucleotide

<400>  263
tcccttctgg atcgactac                                                    19

<210>  264
<211>  19
<212>  DNA
<213>  Artificial Sequence

<220>
<223>  Synthetic oligonucleotide

<400>  264
caattatgag tccacggtc                                                    19


<210>  265
<211>  19
<212>  DNA
<213>  Artificial Sequence

<220>
<223>  Synthetic oligonucleotide

<400>  265
aacgatgggc atgtatttc                                                    19


<210>  266
<211>  19
<212>  DNA
<213>  Artificial Sequence

<220>
<223>  Synthetic oligonucleotide

<400>  266
tgtctcctat gttcaggtc                                                    19


<210>  267
<211>  19
<212>  DNA
<213>  Artificial Sequence

<220>
<223>  Synthetic oligonucleotide

<400>  267
gtatggacag aactggctc                                                    19


<210>  268
<211>  19
<212>  DNA
<213>  Artificial Sequence

<220>
<223>  Synthetic oligonucleotide

<400>  268
gatgaacatg tatggagac                                                    19


<210>  269
<211>  19
<212>  DNA
<213>  Artificial Sequence

<220>
<223>  Synthetic oligonucleotide

<400>  269
attccttcaa acgtatggc                                                    19


<210>  270
<211>  19
<212>  DNA
<213>  Artificial Sequence

<220>
<223>  Synthetic oligonucleotide

<400>  270
gctcctggag aacatgtac                                                    19


<210>  271
<211>  19
<212>  DNA
<213>  Artificial Sequence

<220>
<223>  Synthetic oligonucleotide

<400>  271
accaacctca gaggttctc                                                    19


<210>  272
<211>  19
<212>  DNA
<213>  Artificial Sequence

<220>
<223>  Synthetic oligonucleotide

<400>  272
aagcggacct acaaacttc                                                    19


<210>  273
<211>  19
<212>  DNA
<213>  Artificial Sequence

<220>
<223>  Synthetic oligonucleotide

<400>  273
cgtctacatg tacgtgttc                                                    19


<210>  274
<211>  19
<212>  DNA
<213>  Artificial Sequence

<220>
<223>  Synthetic oligonucleotide

<400>  274
gtgtgtacat caactgttc                                                    19


<210>  275
<211>  19
<212>  DNA
<213>  Artificial Sequence

<220>
<223>  Synthetic oligonucleotide

<400>  275
ggcacagtcc aatctgaac                                                    19


<210>  276
<211>  19
<212>  DNA
<213>  Artificial Sequence

<220>
<223>  Synthetic oligonucleotide

<400>  276
cgcaagttct actccatcc                                                    19


<210>  277
<211>  19
<212>  DNA
<213>  Artificial Sequence

<220>
<223>  Synthetic oligonucleotide

<400>  277
ggtgttcagt ctggacaac                                                    19


<210>  278
<211>  19
<212>  DNA
<213>  Artificial Sequence

<220>
<223>  Synthetic oligonucleotide

<400>  278
gtgttcagtc tggacaacc                                                    19


<210>  279
<211>  19
<212>  DNA
<213>  Artificial Sequence

<220>
<223>  Synthetic oligonucleotide

<400>  279
acttgaactc tctccacac                                                    19


<210>  280
<211>  19
<212>  DNA
<213>  Artificial Sequence

<220>
<223>  Synthetic oligonucleotide

<400>  280
gtcttcaata acttgaagc                                                    19


<210>  281
<211>  19

<212>  DNA
<213>  Artificial Sequence

<220>
<223>  Synthetic oligonucleotide

<400>  281
ccacaacaag cacgtgttc                                                    19


<210>  282
<211>  19
<212>  DNA
<213>  Artificial Sequence

<220>
<223>  Synthetic oligonucleotide

<400>  282
atcaatcgaa agattcttc                                                    19


<210>  283
<211>  19
<212>  DNA
<213>  Artificial Sequence

<220>
<223>  Synthetic oligonucleotide

<400>  283
cgacgttgac aattccagc                                                    19


<210>  284
<211>  19
<212>  DNA
<213>  Artificial Sequence

<220>
<223>  Synthetic oligonucleotide

<400>  284
gcagagttgc agatttgtc                                                    19


<210>  285
<211>  19
<212>  DNA
<213>  Artificial Sequence

<220>
<223>  Synthetic oligonucleotide

<400>  285
tcagcattcc tacgataac                                                    19


<210>  286
<211>  19
<212>  DNA
<213>  Artificial Sequence

<220>
<223>  Synthetic oligonucleotide

<400>  286
ccggcagaag gataacctc                                                    19

<210>  287
<211>  19
<212>  DNA
<213>  Artificial Sequence

<220>
<223>  Synthetic oligonucleotide

<400>  287
ccagaactgt gaagtttac                                                    19


<210>  288
<211>  19
<212>  DNA
<213>  Artificial Sequence

<220>
<223>  Synthetic oligonucleotide

<400>  288
gcatggcaag aaagttaac                                                    19


<210>  289
<211>  19
<212>  DNA
<213>  Artificial Sequence

<220>
<223>  Synthetic oligonucleotide

<400>  289
agcatgttat gccttatgc                                                    19


<210>  290
<211>  19
<212>  DNA
<213>  Artificial Sequence

<220>
<223>  Synthetic oligonucleotide

<400>  290
tcattgtttc tgccatagc                                                    19


<210>  291
<211>  19
<212>  DNA
<213>  Artificial Sequence

<220>
<223>  Synthetic oligonucleotide

<400>  291
gatctttaac cagcctgac                                                    19


<210>  292
<211>  19
<212>  DNA
<213>  Artificial Sequence

<220>
<223>  Synthetic oligonucleotide

<400>  292
gcacaccgtg gtagtatac                                                    19


<210>  293
<211>  19
<212>  DNA
<213>  Artificial Sequence

<220>
<223>  Synthetic oligonucleotide

<400>  293
gtccttccac atcaagaac                                                    19


<210>  294
<211>  19
<212>  DNA
<213>  Artificial Sequence

<220>
<223>  Synthetic oligonucleotide

<400>  294
tgcaacctct tacccaagc                                                    19


<210>  295
<211>  19
<212>  DNA
<213>  Artificial Sequence

<220>
<223>  Synthetic oligonucleotide

<400>  295
ctcaatccct tcgtctacc                                                    19


<210>  296
<211>  19
<212>  DNA
<213>  Artificial Sequence

<220>
<223>  Synthetic oligonucleotide

<400>  296
cgtggtctac gcgttctac                                                    19


<210>  297
<211>  19
<212>  DNA
<213>  Artificial Sequence

<220>
<223>  Synthetic oligonucleotide

<400>  297
cattatcacc atgctcggc                                                    19


<210>  298
<211>  19
<212>  DNA
<213>  Artificial Sequence

<220>
<223>  Synthetic oligonucleotide

<400>  298
tctctcttcc tatcaatcc                                                    19


<210>  299
<211>  19
<212>  DNA
<213>  Artificial Sequence

<220>
<223>  Synthetic oligonucleotide

<400>  299
ccaaacgtgt ctggaagac                                                    19


<210>  300
<211>  19
<212>  DNA
<213>  Artificial Sequence

<220>
<223>  Synthetic oligonucleotide

<400>  300
gtacctgtag ccatctaac                                                    19


<210>  301
<211>  19
<212>  DNA
<213>  Artificial Sequence

<220>
<223>  Synthetic oligonucleotide

<400>  301
gctagtgaat aatgattgc                                                    19


<210>  302
<211>  19
<212>  DNA
<213>  Artificial Sequence

<220>
<223>  Synthetic oligonucleotide

<400>  302
acacgactat aagtctaac                                                    19


<210>  303
<211>  19
<212>  DNA
<213>  Artificial Sequence

<220>
<223>  Synthetic oligonucleotide

<400>  303
acattgcaat ggacaacac                                                    19


<210>  304
<211>  19

<212>  DNA
<213>  Artificial Sequence

<220>
<223>  Synthetic oligonucleotide

<400>  304
ggaatatcct gagtgttgc                                                    19


<210>  305
<211>  19
<212>  DNA
<213>  Artificial Sequence

<220>
<223>  Synthetic oligonucleotide

<400>  305
tgccagaggg atgaactac                                                    19


<210>  306
<211>  19
<212>  DNA
<213>  Artificial Sequence

<220>
<223>  Synthetic oligonucleotide

<400>  306
atggaagact gctggaatc                                                    19


<210>  307
<211>  19
<212>  DNA
<213>  Artificial Sequence

<220>
<223>  Synthetic oligonucleotide

<400>  307
gagcgcttca aacgagatc                                                    19


<210>  308
<211>  19
<212>  DNA
<213>  Artificial Sequence

<220>
<223>  Synthetic oligonucleotide

<400>  308
acctgtccct agattcttc                                                    19


<210>  309
<211>  19
<212>  DNA
<213>  Artificial Sequence

<220>
<223>  Synthetic oligonucleotide

<400>  309
acgcatcttg gcaaagagc                                                    19

<210>  310
<211>  19
<212>  DNA
<213>  Artificial Sequence

<220>
<223>  Synthetic oligonucleotide

<400>  310
attccagggt ttatgtgtc                                                    19


<210>  311
<211>  19
<212>  DNA
<213>  Artificial Sequence

<220>
<223>  Synthetic oligonucleotide

<400>  311
caacagtgca tctcttatc                                                    19


<210>  312
<211>  19
<212>  DNA
<213>  Artificial Sequence

<220>
<223>  Synthetic oligonucleotide

<400>  312
cagcatctac ctcctgaac                                                    19


<210>  313
<211>  19
<212>  DNA
<213>  Artificial Sequence

<220>
<223>  Synthetic oligonucleotide

<400>  313
cagcgctctc aatcccttc                                                    19


<210>  314
<211>  19
<212>  DNA
<213>  Artificial Sequence

<220>
<223>  Synthetic oligonucleotide

<400>  314
catcgactat atagcaggc                                                    19


<210>  315
<211>  19
<212>  DNA
<213>  Artificial Sequence

<220>
<223>  Synthetic oligonucleotide

<400>  315
ccaagagtct attcaaagc                                                    19


<210>  316
<211>  19
<212>  DNA
<213>  Artificial Sequence

<220>
<223>  Synthetic oligonucleotide

<400>  316
ccactaatgt caacaatgc                                                    19


<210>  317
<211>  19
<212>  DNA
<213>  Artificial Sequence

<220>
<223>  Synthetic oligonucleotide

<400>  317
ccactcgtca gatgtttgc                                                    19


<210>  318
<211>  19
<212>  DNA
<213>  Artificial Sequence

<220>
<223>  Synthetic oligonucleotide

<400>  318
ccctgtcatt gatggagac                                                    19


<210>  319
<211>  19
<212>  DNA
<213>  Artificial Sequence

<220>
<223>  Synthetic oligonucleotide

<400>  319
ccgagccata tacttgacc                                                    19


<210>  320
<211>  19
<212>  DNA
<213>  Artificial Sequence

<220>
<223>  Synthetic oligonucleotide

<400>  320
cctagagctg attgagttc                                                    19


<210>  321
<211>  19
<212>  DNA
<213>  Artificial Sequence

<220>
<223>  Synthetic oligonucleotide

<400>  321
cctcgacacc aagtcttgc                                                    19


<210>  322
<211>  19
<212>  DNA
<213>  Artificial Sequence

<220>
<223>  Synthetic oligonucleotide

<400>  322
cgctctttaa catgaatgc                                                    19


<210>  323
<211>  19
<212>  DNA
<213>  Artificial Sequence

<220>
<223>  Synthetic oligonucleotide

<400>  323
cgtggacatg gagtacgac                                                    19


<210>  324
<211>  19
<212>  DNA
<213>  Artificial Sequence

<220>
<223>  Synthetic oligonucleotide

<400>  324
ctcgtaatga gactatagc                                                    19


<210>  325
<211>  19
<212>  DNA
<213>  Artificial Sequence

<220>
<223>  Synthetic oligonucleotide

<400>  325
ctgcaaatct tcaggtttc                                                    19


<210>  326
<211>  19
<212>  DNA
<213>  Artificial Sequence

<220>
<223>  Synthetic oligonucleotide

<400>  326
gaagcacgat ttggaggtc                                                    19


<210>  327
<211>  19

<212>  DNA
<213>  Artificial Sequence

<220>
<223>  Synthetic oligonucleotide

<400>  327
gatgatgaag gagacgttc                                                    19


<210>  328
<211>  19
<212>  DNA
<213>  Artificial Sequence

<220>
<223>  Synthetic oligonucleotide

<400>  328
gatttggtta taagggttc                                                    19


<210>  329
<211>  19
<212>  DNA
<213>  Artificial Sequence

<220>
<223>  Synthetic oligonucleotide

<400>  329
gctactgccc tatatgatc                                                    19


<210>  330
<211>  19
<212>  DNA
<213>  Artificial Sequence

<220>
<223>  Synthetic oligonucleotide

<400>  330
tccggttcta tttgatcgc                                                    19


<210>  331
<211>  19
<212>  DNA
<213>  Artificial Sequence

<220>
<223>  Synthetic oligonucleotide

<400>  331
tcgcccttcc tattccttc                                                    19


<210>  332
<211>  19
<212>  DNA
<213>  Artificial Sequence

<220>
<223>  Synthetic oligonucleotide

<400>  332
tgtacgtgtt catcctggc                                                    19

<210>  333
<211>  19
<212>  DNA
<213>  Artificial Sequence

<220>
<223>  Synthetic oligonucleotide

<400>  333
tgtgacatta tgcctttgc                                                    19


<210>  334
<211>  12
<212>  DNA
<213>  Artificial Sequence

<220>
<223>  Synthetic oligonucleotide

<400>  334
gtttgctata ac                                                           12

## Description

The present invention relates to methods for inducing the differentiation of undifferentiated mammalian cells into osteoblasts. The invention further relates to methods for identifying one or more compounds that induce said differentiation, as well as to the compounds per se for inducing said differentiation.

Bone contains two distinct cell lineages, bone-forming cells (e.g. osteoblasts) and bone-resorbing cells (e.g. osteoclasts). It is a dynamic tissue that is continuously being destroyed (resorbed) and rebuilt, by an intricate interplay between these osteoblasts and osteoclasts.

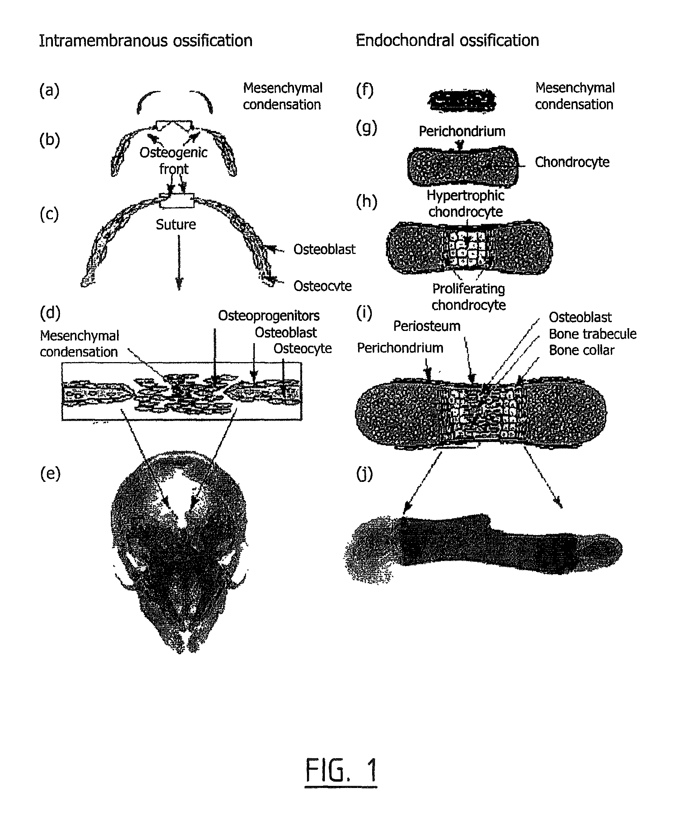

For osteoclasts, a cascade of transcription factors and growth factors involved in the progression from progenitor cell to functional osteoclast is well established. In contrast, little is known about the osteoblast lineage. Osteoblasts originate from differentiated mesenchymal progenitor cells (MPCs). During differentiation into osteoblasts bone alkaline phosphatase activity (BAP) becomes upregulated. Bone formation in vivo occurs through two distinct pathways during embryonic development: endochondral or intramembranous ossification (Figure 1). As shown in this figure, mesenchymal progenitor or stem cells represent the starting points for both forms of bone formation. During intramembranous ossification, flat bones such as those of the skull or clavicles, are formed directly from condensations of mesenchymal cells. During the formation of long bones, such as limb bones, mesenchymal condensations first lead to a cartilage intermediate that is invaded during further development by endothelial cells, osteoclasts and mesenchymal cells that will differentiate into osteoblasts and osteocytes (From Nakashima and de Crombrugghe, 2003).

A number of diseases are known which are caused by a disturbance of the fine-tuned balance between bone resorption and bone build-up. These skeletal diseases represent a large number of patients: hypercalcemia of malignancy, Paget's disease, inflammatory bone diseases like rheumatoid arthritis and periodontal disease, focal osteogenesis occurring during skeletal metastases, Crouzon's syndrome, rickets, opsismodysplasia, pycnodysostosis/Toulouse-Lautrec disease, osteogenesis imperfecta, but the single most important bone disease is osteoporosis.

Osteoporosis affects 1 in 5 women over 50 and 1 in 20 men over 50. A number of treatments are available for the patient. These mostly tackle the net increase in bone resorption, i.e.:
- hormone replacement therapy (HRT)
- selective estrogen receptor modulators (SERMs)
- bisphosphonates
- calcitonin.

While these treatments slow down bone resorption, they do not abolish fracturing because the lost bone is not sufficiently replenished. Fracturing will only be stopped when bone formation is sufficiently increased. Therefore, there is great interest in identifying osteogenic pathways that lend themselves to therapeutic intervention with bone anabolism as effect. Currently, only one bone anabolic therapy has reached the osteoporosis market: parathyroid hormone (PTH)1-34. PTH displays bone anabolic effects when administered intermittently. The treatment is very cumbersome because this biopharmaceutical needs to be injected daily by the patient. In addition, tumor formation was observed when treating animals at high doses. Also, it is a very expensive treatment.

Another class of bone anabolics, bone morphogenetic proteins (BMPs), have been approved but only for niche markets. There are disadvantages to their use as therapeutic agents to enhance bone healing. Receptors for the bone morphogenetic proteins have been identified in many tissues, and the BMPs themselves are expressed in a large variety of tissues in specific temporal and spatial patterns. This suggests that BMPs may have effects on many tissues other than bone, potentially limiting their usefulness as therapeutic agents when administered systemically. Thus, there is a need for novel anabolics that circumvent one or more of the shortcomings of the above mentioned anabolics.

It is an object of the present invention to provide a method for inducing differentiation of undifferentiated mammalian cells into osteoblasts.

This object is achieved by providing a method for inducing differentiation of undifferentiated mammalian cells into osteoblasts, comprising contacting said undifferentiated cells with an inhibitor of any of the polypeptides listed in table 4, and/or fragments and/or derivatives of said polypeptide.

It is to be understood that according to the present invention the term "inhibitor of a polypeptide" relates to any type of molecule that inhibits, e.g. downmodulates the biological activity of said polypeptide, by inhibiting the expression and/or translation of said polypeptide, or by inhibiting the polypeptide per se.

Undifferentiated cells are pluripotent cells which are in an early stage of specialization, i.e., which do not yet have their final function and can be induced to form almost any given cell type. In particular, these are cells which have not yet differentiated to *e.g*. osteoblasts or osteoclasts. Such cells are for example blood cells and cells present in bone marrow, as well as cells derived from adipose tissue. In addition, cells which still can be differentiated into mesenchymal precursor cells are ontemplated in the present invention, such as, for example, totipotent stem cells such as embryonic stem cells.

Osteoblast differentiation can be measured by measuring the level of enzymes that are induced during the differentiation process, such as alkaline phosphatase (BAP), type-1 collagen, osteocalcin and osteopontin. The alkaline phosphatase activity can be measured by adding methylumbelliferyl heptaphosphate (MUP) solution (Sigma) to the cells. The fluorescence generated upon cleavage of the MUP substrate by the AP activity is measured on a fluorescence plate reader (Fluostar, BMG).

In a preferred embodiment of the present invention, the inhibitor is an expression or translation inhibitor inhibiting the expression or translation of a polyribonucleotide encoding the polypeptide.

In another preferred embodiment the inhibitor is an nucleic acid expressing the expression or translation inhibitor inhibiting the expression or translation of a polyribonucleotide encoding the polypeptide.

Preferably, the nucleic acid is included within a vector. More preferably the vector is an adenoviral, retroviral, adeno-associated viral, lentiviral or a sendaiviral vector.

Nucleic acids expressing the inhibitor, e.g. antisense RNA, ribozyme, antisense oligodeoxynucleotide (ODN), and/or siRNA, such as vectors and viral vectors, have the advantage that they produce said inhibitors continuously. Vectors include plasmids and viruses. In the vector, the nucleic acid sequence may be placed under the control of a appropriate promoter, such as CMV, HSV, TK, SV40, or, the elongation factor among others. A prolonged production of the inhibitor will give a longer inhibitory effect and less transfections are needed.

In a particular preferred embodiment, the inhibitor is selected from the group consisting of an antisense RNA, a ribozyme that cleaves the polyribonucleotide, an antisense oligodeoxynucleotide (ODN), and a small interfering RNA (siRNA) that is sufficiently homologous to a portion of the polyribonucleotide such that the siRNA is capable of inhibiting the polyribonucleotide that would otherwise cause the production of the polypeptide.

Chemically modified variants of the inhibitors form also part of the present invention, and relate to inhibitors which are modified in order to enhance their stability. The inhibitors thus may contain a variety of modifications that confer resistance to nucleolytic degradation, such as, for example, modified internucleoside linkages, modified nucleic acid bases and/or modified sugars and the like. The antisense oligonucleotides of the invention can also be modified by chemically linking the oligonucleotide to one or more moieties or conjugates to enhance the activity, cellular distribution, or cellular uptake of the antisense oligonucleotide. Such moieties or conjugates include but are not limited to lipids such as cholesterol, cholic acid, thioether, aliphatic chains, phospholipids, polyamines, polyethylene glycol (PEG), or palmityl moieties. Other forms of chemical modification, including but not limited to 2'-O-methyl-, Methyl-phosphonate-, phosphothioate, inverted end- (3'-3'-linkage) modifications, are also possible.

One type of expression-inhibitory agent is a nucleic acid that is antisense to a nucleic acid comprising any of the genes listed in Table 4. For example, an antisense nucleic acid (e.g. DNA) may be introduced into cells in vitro, or administered to a subject in vivo, as gene therapy to inhibit cellular expression of nucleic acids comprising any of the genes listed in Table 4. Anti-sense nucleic acid is intended to mean an nucleic acid that has a nucleotide sequence that interacts through base pairing with a specific complementary nucleic acid sequence involved in the expression of the target such, that the expression of the gene is reduced. Preferably, the specific complementary nucleic acid sequence involved in the expression of the gene is a genomic DNA molecule or mRNA molecule that encodes the gene. This genomic DNA molecule can comprise regulatory regions of the gene, or the coding sequence for the mature gene.

Antisense nucleic acids preferably comprise a sequence containing from about 17 to about 100 nucleotides and more preferably the antisense nucleic acids comprise from about 18 to about 30 nucleotides. Antisense nucleic acids of the invention are preferably nucleic acid fragments capable of specifically hybridizing with all or part of a nucleic acid encoded by any of the genes listed in Table 4 or the corresponding messenger RNA, or they may be DNA sequences whose expression in the cell produces RNA complementary to all or part of the mRNA comprisingnucleic acid sequences encoded by any of the genes listed in Table 4. Antisense nucleic acids may be prepared by expression of all or part of a sequence selected from the group nucleic acid sequences encoded by any of the genes listed in Table 4, in the opposite orientation. Preferably the antisense nucleic acid is prepared by expression of a sequence selected from the groups consisting of SEQ ID NO: 1-226 and SEQ ID NO 247-333, in the opposite orientation. Preferably, the antisense sequence is at least about 17 nucleotides in length.

The term complementary to a nucleotide sequence in the context of antisense nucleic acids and methods therefore means sufficiently complementary to such a sequence as to allow hybridization to that sequence in a cell, *i*.*e*., under physiological conditions.

Another type of expression-inhibitor according to the invention is a nucleic acid that is able to catalyze cleavage of RNA molecules. The expression "ribozymes" as used herein relates to catalytic RNA molecules capable of cleaving other RNA molecules at phosphodiester bonds in a manner specific to the sequence. The hydrolysis of the target sequence to be cleaved is initiated by the formation of a catalytically active complex consisting of ribozyme and substrate RNA. All ribozymes capable of cleaving phosphodiester bonds in trans, that is to say intermolecularly, are suitable for the purposes of the invention.

Yet another type of expression-inhibition is RNA interference (RNAi). RNAi is the post-transcriptional process of gene silencing mediated by double stranded RNA (dsRNA) that is homologous in sequence to the silenced RNA and is observed in animals and plants.

According to a particular preferred embodiment of the invention the inhibitor is a siRNA, comprising a first nucleotide sequence of 17-23 nucleotides homologous to a nucleotide sequence selected from any of the target genes listed in table 4, and a second nucleotide sequence of 17-23 nucleotides complementary to said first nucleotide sequence. Preferably, the inhibitor is a siRNA, comprising a first nucleotide sequence of 17-23 nucleotides selected from the nucleotide sequences identified by SEQ. ID NO. 1-220 and 247-333 in table 4 and 5, and a second nucleotide sequence of 17-23 nucleotides complementary to said first nucleotide sequence.

In another preferred embodiment the siRNA further comprises a third nucleotide sequence connecting the first and second nucleotide sequence, and capable of forming a stem-loop structure within the siRNA.

Such self-complementing single stranded siRNA polynucleotide according to the present invention thus at least comprises a first guide sequence, and a second guide sequence, which complements the first guide sequence, and a third sequence capable of forming a stem-loop structure within said second sequence, which is covalently linked to the distal end of the first sequence and the proximal end of the second sequence. All nucleotides in the first and second sequences may base pair, or alternatively there may be mismatches between the first and second sequences. The nucleotide sequences are preferably between about 17 and 23 nts in length. Preferably the first or the second sequence is a oligonucleotide sequence between about 17 and 23 nt in length selected from the polynucleotide sequences of any of the genes listed in Table 4. Such siRNA polynucleotides with a loop are selfcomplementary and can form stable hairpins. Hairpins are more stable than ordinary dsRNA. In addition, they are more easily produced from vectors.

More preferably the first or second nucleotide sequence comprises a nucleotide sequence consisting of SEQ ID NO: 1-220 and SEQ ID NO 247-333. The nucleotide sequences of SEQ ID NO 1-220 and SEQ ID NO 247-333 are selected according to siRNA designing rules that give an improved reduction of the target sequences compared to nucleotide sequences that do not comply with these siRNA designing rules (see WO 2004/094636).

Preferably, the third nucleotide sequence is 4-30 nucleotides long, more preferably 5-15 nucleotides long and most preferably 8 nucleotides long. In a most preferred embodiment the third nucleotide sequence is UUGCUAUA (SEQ ID NO: 334).

The present invention further relates to a method for identifying a compound that induces differentiation of undifferentiated mammalian cells into osteoblasts, comprising:
(a) contacting one or more compounds with a polypeptide listed in table 4, encoded by any of the genes listed in table 4, and/or fragments and/or derivatives of said polypeptide;
(b) determining the binding affinity of the compound to the polypeptide;
(c) contacting a population of undifferentiated mammalian cells with the compound that exhibits a binding affinity of at least 10 micromolar; and
(d) identifying the compound that induces the differentiation of the undifferentiated mammalian cells.

The polypeptides or the polynucleotides of the present invention employed in the methods described above may be free in solution, affixed to a solid support, borne on a cell surface, or located intracellularly.

To perform the methods it is feasible to immobilize either the polypeptide of the present invention or the compound to facilitate separation of complexes from uncomplexed forms of the polypeptide, as well as to accommodate automation of the assay. Interaction (e.g., binding of) of the polypeptide of the present invention with a compound can be accomplished in any vessel suitable for containing the reactants. Examples of such vessels include microtiter plates, test tubes, and microcentrifuge tubes. In one embodiment, a fusion protein can be provided which adds a domain that allows the polypeptide to be bound to a matrix. For example, the polypeptide of the present invention can be "His" tagged, and subsequently adsorbed onto Ni-NTA microtitre plates, or ProtA fusions with the polypeptides of the present invention can be adsorbed to IgG, which are then combined with the cell lysates (*e.g*., (35)^{S}-labelled) and the candidate compound, and the mixture incubated under conditions favorable for complex formation (e.g., at physiological conditions for salt and pH). Following incubation, the plates are washed to remove any unbound label, and the matrix is immobilized. The amount of radioactivity can be determined directly, or in the supernatant after dissociation of the complexes. Alternatively, the complexes can be dissociated from the matrix, separated by SDS-PAGE, and the level of the protein binding to the protein of the present invention quantitated from the gel using standard electrophoretic techniques.

Other techniques for immobilizing protein on matrices can also be used in the method of identifying compounds. For example, either the polypeptide of the present invention or the compound can be immobilized utilizing conjugation of biotin and streptavidin. Biotinylated protein molecules of the present invention can be prepared from biotin-NHS (N-hydroxy-succinimide) using techniques well known in the art (e.g., biotinylation kit, Pierce Chemicals, Rockford, III.), and immobilized in the wells of streptavidin-coated 96 well plates (Pierce Chemical). Alternatively, antibodies reactive with the polypeptides of the present invention but which do not interfere with binding of the polypeptide to the compound can be derivatized to the wells of the plate, and the polypeptide of the present invention can be trapped in the wells by antibody conjugation. As described above, preparations of a labeled candidate compound are incubated in the wells of the plate presenting the polypeptide of the present invention, and the amount of complex trapped in the well can be quantitated.

The binding affinity of the compound with the polypeptide or polynucleotide can be measured by methods known in the art, such as using surface plasma resonance biosensors (Biacore), by saturation binding analysis with a labeled compound (e.g. Scatchard and Lindmo analysis), via displacement reactions, by differential UV spectrophotometer, fluorescence polarisation assay, Fluorometric Imaging Plate Reader (FLIPR^{®}) system, Fluorescence resonance energy transfer, and Bioluminescence resonance energy transfer. The binding affinity of compounds can also be expressed in a dissociation constant (Kd) or as IC50 or EC50. The IC50 represents the concentration of a compound that is required for 50% inhibition of binding of another ligand to the polypetide. The EC50 represents the concentration required for obtaining 50% of the maximum effect in vitro. The dissociation constant, Kd, is a measure of how well a ligand binds to the polypeptide, it is equivalent to the ligand concentration required to saturate exactly half of the binding-sites on the polypeptide. Compounds with a high affinity binding have low Kd, IC50 and EC50 values, i.e. in the range of 100 nM to 1 pM; a moderate to low affinity binding relates to a high Kd, IC50 and EC50 values, i.e. in the micromolar range.

For high-throughput purposes, libraries of compounds can be used such as peptide libraries (e.g. LOPAP™, Sigma Aldrich), lipid libraries (BioMol), synthetic compound libraries (e.g. LOPAC™, Sigma Aldrich) or natural compound libraries (Specs, TimTec).

The invention also relates to a method for identifying a compound or mixture of compounds that induces differentiation of undifferentiated mammalian cells into osteoblasts, comprising
(a) culturing a population of undifferentiated mammalian cells that express a polypeptide listed in Table 4, encoded by a gene listed in table 4, and/or fragments and/or derivatives of said polypeptide;
(b) exposing said population of cells to a compound or mixture of compounds; and
(c) selecting the compound or mixture of compounds that induces the differentiation of the undifferentiated cells into osteoblasts.

The compound or mixture of compounds that are identified according to this method can thus be applied to induce differentiation of undifferentiated mammalian cells into osteoblasts.

Preferably the compounds identified in these methods of the invention are low molecular weight compounds. Low molecular weight compounds, i.e. with a molecular weight of 500 Dalton or less, are likely to have good absorption and permeation in biological systems and are consequently more likely to be successful drug candidates than compounds with a molecular weight above 500 Dalton (Lipinsky et al, 2001).

According to another preferred embodiment the compounds are peptides. Peptides can be excellent drug candidates and there are multiple examples of commercially valuable peptides such as fertility hormones and platelet aggregation inhibitors.

According to another preferred embodiment the compounds are natural compounds. Natural compounds are compounds that have been extracted from natural sources, e.g. plants. Using natural compounds in screens has the advantage that more diverse molecules are screened. Natural compounds have an enormous variety of different molecules. Synthetic compounds do not exhibit such variety of different molecules. Other compounds may be selected from carbohydrates, and glycosylated polypeptides.

Moreover, the present invention relates to polynucleotide comprising a sequence of 17-23 nucleotides homologous to a nucleotide sequence selected from any of the target genes listed in table 4, and variants and/or the reverse complements thereof. Preferably, said polynucleotides have a nucleotide sequence selected from the nucleotide sequences identified by SEQ. ID NO. 1-220 and 247-333 in table 4 and 5, or said polynucleotides have a reverse complement of a nucleotide sequence selected from the nucleotide sequences identified by SEQ. ID NO. 1-220 and 247-333 in table 4 and 5. The polynucleotides of the invention have been shown to increase osteoblast differentiation.

The invention also relates to these polynucleotides for use as a medicament.

Furthermore, the invention relates to the use of said polynucleotides in the manufacture of a medicament for the treatment of a disease involving a systemic or local decrease in mean bone density.

As it is shown for the first time in this patent application that the polypeptides comprising an amino acid sequence selected from the group listed in Table 4 are involved in the differentiation of undifferentiated mammalian cells into osteoblasts, in still another preferred embodiment of the present invention, compounds that are known or will be known to inhibit the activity of any of the polypeptides comprising an amino acid sequence selected from the group listed in Table 4 can now also be used to induce differentiation of undifferentiated mammalian cells into osteoblasts. Therefore, the present invention also relates to the use of compounds that are known in the art to inhibit the activity of any of polypeptides comprising an amino acid sequence selected from the group listed in Table 4, as a medicament. Furthermore, the present invention also relates to the use of compounds that are known in the art to inhibit the activity of any of polypeptides comprising an amino acid sequence selected from the group listed in Table 4, as a medicament for the treatment of a disease involving a systemic or local decrease in mean bone density.

The invention furthermore relates to a vector comprising any of the polynucleotides described above. In addition, the invention relates to said vectors for use as a medicament.

In a preferred embodiment, the vector is an adenoviral, retroviral, adeno-associated viral, lentiviral or a sendaiviral vector. Preferably, the vector encodes a siRNA comprising a nucleotide sequence selected from the groups consisting of SEQ ID NO: 1-220 and SEQ ID NO 247-333.

The invention further relates to the use of said vectors for the manufacture of a medicament for the treatment of a disease involving a systemic or local decrease in mean bone density. In a preferred embodiment of the present invention the disease is selected from the group consisting of osteoporosis, hypercalcemia of malignancy, multiple myelomatosis, hyperparathyroidism, and hyperthyroidism.

Recombinant viruses are commonly used for gene transfer. To date, the three most commonly used viruses for gene transfer are adenovirus, retrovirus and adeno-associated virus. More recently lentiviruses, a subgroup of the retroviruses, and sendaivirus are being used. Adenoviruses are able to transduce both dividing and non-dividing cells and can be produced at high viral titres. Retroviruses can infect dividing cells only and integrate their genome into the host chromosome. Integration achieves long-term gene expression. Lentiviruses, a sub-family of retroviruses, share all the standard properties of retroviruses but in addition they have the capacity to transduce non-dividing cells. Adeno-associated virus (AAV) is a small, non-pathogenic, single-stranded DNA virus. It requires co-infection with a helper virus (adenovirus or herpes virus) in order to undergo productive infection. In the absence of helper virus, wild-type AAV integrates site-specifically, into the host genome. Similarly to retrovirus, integration facilitates longer gene expression. AAV can infect both non-dividing and dividing cells. Sendai virus is a member of the paramyxoviridae, and is a single-stranded RNA virus that is able to transfect both dividing and non-dividing cells. Its method of entering cells involves sialic acid and cholesterol that are common to many cell types. Sendai viral gene expression and replication are localized in the cytoplasm, in contrast to most viruses that need to enter the nucleus.

Furthermore, the present invention relates to a method for in vitro production of bone tissue, comprising
(a) applying undifferentiated mammalian cells on a substrate to form a cellular substrate;
(b) introducing any of the polynucleotides described above, or a vector comprising said polynucleotide, for a time sufficient to differentiate the undifferentiated cells into osteoblasts, thereby producing a continuous bone matrix. Preferably the continuous bone matrix comprises a thickness of at least 0.5 µm on the surface of the substrate.

The invention thus provides a method for producing a substrate with a matrix grown thereon, which can be used for the provision of load-bearing implants, including joint prostheses, such as artificial hip joints, knee joints and finger joints, and maxillofacial implants, such as dental implants. It can also be used for special surgery devices, such as spacers, or bone fillers, and for use in augmentation, obliteration or reconstitution of bone defects and damaged or lost bone. Bone formation can be optimized by variation in mineralization, both by inductive and by conductive processes.

A combination of the provision of a load-bearing implant (preferably coated with a matrix as described above) with a bone filler comprising a matrix as described, constitutes an advantageous method according to the present invention.

The method of the invention is also very suitable in relation to revision surgery, i.e., when previous surgical devices have to be replaced.

Suitable undifferentiated cells are bone marrow cells, including haematopoietic cells and in particular stromal cells. The marrow cells, and especially the stromal cells are found to be very effective in the bone producing process when taken from their original environment. The undifferentiated cells can be directly applied on the substrate or they can advantageously be multiplied in the absence of the substrate before being applied on the substrate. In the latter mode, the cells are still largely undifferentiated after multiplication and, for the purpose of the invention, they are still referred to as undifferentiated. Subsequently, the cells are allowed to differentiate. Differentiation can be induced or enhanced by the presence of suitable inductors, such as glucocorticoids, and dexamethasone. Especially suitable inductors of differentiation are the expression inhibitory agents of the present invention.

The use of undifferentiated cells provides several advantages. Firstly, their lower differentiation implies a higher proliferation rate and allows the eventual functionality to be better directed and controlled. Moreover, culturing these cells not only produces the required bone matrix containing organic and inorganic components, but also results in the presence, in the culture medium and in the matrix, of several factors which are essential for growth of the tissue and for adaptation to existing living tissue. Also, the culture medium can be a source of active factors such as growth factors, to be used in connection with the implanting process. Furthermore, such undifferentiated cells are often available in large quantities and more conveniently than *e.g*., mature bone cells, and exhibit a lower morbidity during recovery. Moreover, the undifferentiated cells can be obtained from the patient for whom the implant is intended. The bone resulting from these cells is autologous to the patient and thus no immune response will be induced. Matrices as thick as 100 µm can be produced as a result of the use of undifferentiated cells.

The substrate on which the undifferentiated cells can be applied and cultured can be a metal, such as titanium, cobalt/chromium alloy or stainless steel, a bioactive surface such as a calcium phosphate, polymer surfaces such as polyethylene, and the like. Although less preferred, siliceous material such as glass ceramics, can also be used as a substrate. Most preferred are metals, such as titanium, and calcium phosphates, even though calcium phosphate is not an indispensable component of the substrate. The substrate may be porous or non-porous. The cells can be applied at a rate of *e.g*., 10³ -10⁶ per cm², in particular 10⁴ -2X10⁵ cells per cm².

The culture medium to be used in the method according to the invention can be a commonly known culture medium such as MEM (minimum essential medium). Advantageously, the medium can be a conditioned medium. In this context, a conditioned medium is understood to be a medium wherein similar cells have previously been incubated, causing the medium to contain factors such as polypeptides, secreted by the cells which are important for cell growth and cell differentiation.

The cells are cultured for a time sufficient to produce a matrix layer, *e.g*., a matrix layer having a thickness of at least 0.5 µm, in particular from 1 up to 100 pm, more in particular of 10-50 µm. The cells may be contacted with the culture medium for *e.g*. 2-15 weeks, in particular 4-10 weeks.

The production of the matrix, when applied on a substrate, results in a continuous or quasi-continuous coating covering the substrate for at least 50%, in particular at least 80% of its surface area.

In another embodiment the present invention provides osteoblast cells obtainable by methods of the present invention.

Another embodiment of the present invention involves the use of the osteoblast cells obtainable by methods of the present invention for in vitro production of bone tissue.

The present invention relates further to a method for diagnosing a pathological condition involving a systemic or local decrease in mean bone density or a susceptibility to the condition in a subject, comprising
(a) determining the level of expression of a polynucleotide encoded by any of the target genes listed in table 4, in a biological sample derived from said subject; and
(b) comparing the level of expression with the level of expression of the polynucleotides in a sample derived from a healthy subject;
wherein an increase of the amount of polynucleotide in the sample of the subject compared to the sample of the healthy subject is indicative of the presence of the pathological condition.

In addition, the present invention relates to a method for diagnosing a pathological condition involving a systemic or local decrease in mean bone density or a susceptibility to the condition in a subject, comprising
(a) determining the amount of a polypeptide encoded by the genes listed in table 4 in a biological sample derived from said subject; and
(b) comparing the amount with the amount of said polypeptide in a biological sample derived from a healthy subject;
wherein an increase of the amount of polypeptide in the subject compared to the healthy subject is indicative of the presence of the pathological condition. Preferably the pathological condition is selected from the group consisting of osteoporosis, hypercalcemia of malignancy, multiple myelomatosis, hyperparathyroidism, and hyperthyroidism.

According to the present invention, the term "derivatives of a polypeptide" relates to those proteins, proteinaceous molecules, fractions of proteins, peptides, and oligopeptides that comprise a stretch of contiguous amino acid residues of the polypeptide and that retain the biological activity of the protein, e.g. polypeptides that have amino acid mutations compared to the amino acid sequence of a naturally-occurring form of the polypeptide. A derivative may further comprise additional naturally occurring, altered, glycosylated, acylated or non-naturally occurring amino acid residues compared to the amino acid sequence of a naturally occurring form of the polypeptide. It may also contain one or more non-amino acid substituents compared to the amino acid sequence of a naturally occurring form of the polypeptide, for example a reporter molecule or other ligand, covalently or non-covalently bound to the amino acid sequence.

The term "fragment of a polypeptide" relates to peptides, oligopeptides, polypeptides, proteins and enzymes that comprise a stretch of contiguous amino acid residues, and exhibit substantially a similar, but not necessarily identical, functional activity as the complete sequence.

The term "polynucleotide" refers to nucleic acids, such as double stranded, or single stranded DNA and (messenger) RNA, and all types of oligonucleotides. It also includes nucleic acids with modified backbones such as peptide nucleic acid (PNA), polysiloxane, and 2'-O-(2-methoxy)ethylphosphorothioate.

The term "derivatives of a polynucleotide" relates to DNA-molecules, RNA- molecules, and oligonucleotides that comprise a stretch or nucleic acid residues of the polynucleotide, e.g. polynucleotides that may have nucleic acid mutations as compared to the nucleic acid sequence of a naturally occurring form of the polynucleotide. A derivative may further comprise nucleic acids with modified backbones such as PNA, polysiloxane, and 2'-O-(2-methoxy)ethyl-phosphorothioate, non-naturally occurring nucleic acid residues, or one or more nuclei acid substituents, such as methyl-, thio-, sulphate, benzoyl-, phenyl-, amino-, propyl-, chloro-, and methanocarbanucleosides, or a reporter molecule to facilitate its detection.

The term "fragment of a polynucleotide" relates to oligonucleotides that comprise a stretch of contiguous nucleic acid residues that exhibit substantially a similar, but not necessarily identical, activity as the complete sequence.

The term "homologous sequence", whether a nucleic acid sequence or an amino acid sequence, is limited to a sequence that is identical or substantially identical to the parent sequence and has the same biological property as the parent sequence. The term is meant to include naturally occurring mutants and variants of the parent sequence. The parent sequence is either provided in table 4-6, or is indicated by the GenBank accession numbers in these tables. In general, a homologous sequence is at least 95% identical to the parent sequence. However, up to two mismatches are allowed for the 17-21 bp siRNA sequences, provided that the homologous sequence leads to a similar down-regulation of the corresponding gene as the parent sequence.

### Short Description Of The Figures

Figure 1. Intramembranous and endochondral ossification.
Figure 2. Principle of the osteoblast differentiation assay.
Figure 3. Lay-out of the 96 well knock-down control plate.
Figure 4. Lay-out of the 384 well control plate.
Figure 5. Performance of the knock-down control plate in the AP assay
Figure 6. Dot plot representation of raw data for one SilenceSelect screening plate
Figure 7. Profiling of target expression in MPCs (A) and knock-down of gene expression by Ad-siRNA (B)
Figure 8. Profiling of target expression in primary human OBs
Figure 9. Analyzing the upregulation of BAP-mRNA versus PLAP- or IAP-mRNA
Figure 10. Results mineralization assay
Figure 11. Pipetting scheme used for screening the Ad-shRNAs at 3 MOIs.

### EXAMPLES

### Example 1. Development of a hiqh-throuqhput screening method for the detection of endogenous alkaline phosphatase

### Principle of the assay

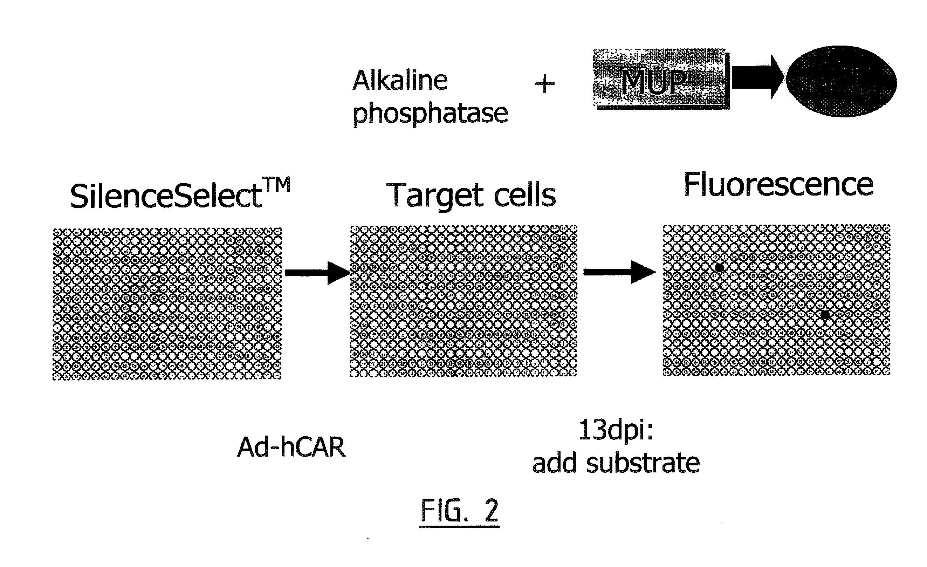

Mesenchymal progenitor cells (MPCs) are determined to differentiate into osteoblasts in the presence of appropriate factors (e.g. BMP2). An assay to screen for such factors was developed by monitoring the activity of alkaline phosphatase (AP) enzyme, an early marker in the osteoblast differentiation program. MPCs were seeded in 384 well plates and simultaneously co-infected one day later with adenoviruses encoding the human coxsackie and adenovirus receptor (hCAR; Ad-hCAR) and individual siRNA adenoviruses (Ad-siRNA) from the SilenceSelect™ collection. AdC15-hCAR/ AdC20-hCAR co-infection increases the AdC01-siRNA infection efficiency. Cellular AP activity was determined 13 days after the start of the infection (13 dpi). Figure 2 illustrates the principle of the assay.

### Development of the assay

MPCs were isolated from bone marrow of healthy volunteers, obtained after informed consent (Cambrex/Biowhittaker, Verviers, Belgium).

In a series of experiments, carried out in 384 well plates, several parameters were optimized: cell seeding density, multiplicities of infection (MOI) of control viruses (Ad-BMP2 or Ad-eGFP), MOI of Ad-hCAR, duration of infection, toxicity, infection efficiency (using Ad-eGFP) and the day of readout.

Using Ad-BMP2 (BMP2 overexpression) as a positive control for assay development, the following protocol resulted in the highest dynamic range for the assay with the lowest standard deviation on the background signal: MPCs were seeded on day0 at 500 cells per well of a 384 well plate and co-infected the next day using a mix of Ad-hCAR (5 µl of an Ad-hCAR solution: mix total MOI=155.7) and 1 µl of Ad-control-virus (Ad-BMP2 or Ad-eGFP; corresponds to a theoretical MOI of 5000). On day5, the medium containing virus was removed and replaced by fresh medium containing no virus. Upregulation of alkaline phosphatase was read at 13 dpi: 15 µl 4-Methylumbelliferylphosphate (MUP, Sigma) was added to each well, the plates were incubated for 15 min at 37°C and monitored for AP activity using a fluorescence plate reader (Fluostar, BMG).

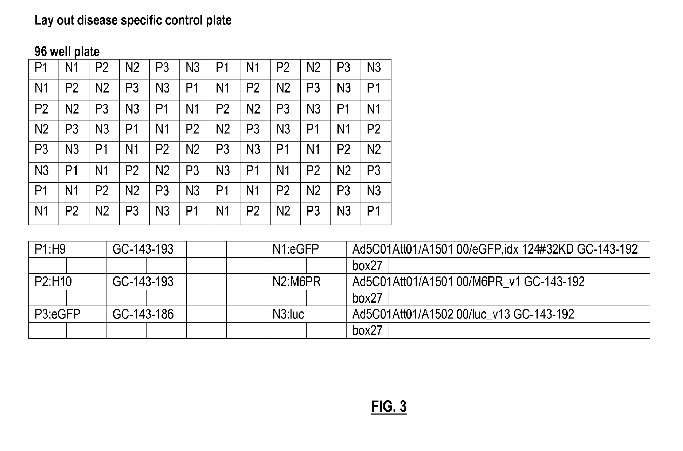

After optimisation of the assay, a small pilot screen was run (103 different Ad-siRNA viruses) with the use of robotics (96/384 channel dispensor Tecan Freedom 200 equipped with TeMO96, TeMO384 and RoMa, Tecan AG, Switzerland). The hits from this screen were collected and retested in the same assay. The two Ad-siRNAs that scored strongest (H9=H24-01 0; H10=H24-011) were used to generate a control plate (knock-down (KD) control plate) containing Ad-siRNAs. The control plate, a 96 well plate containing 3 negative (N1,N2,N3) and 3 positive (P1,P2,P3) control viruses is depicted in Figure 3. This "knock-down" control plate contains Ad-H9 (H24-010) and Ad-H10 (H24-011) as positive controls; Ad-eGFP (knock-in virus) as infection control; and Ad-eGFP-siRNA, Ad-M6PR-siRNA and Ad-Luc-siRNA (all 3 are knock-down viruses) as negative controls.

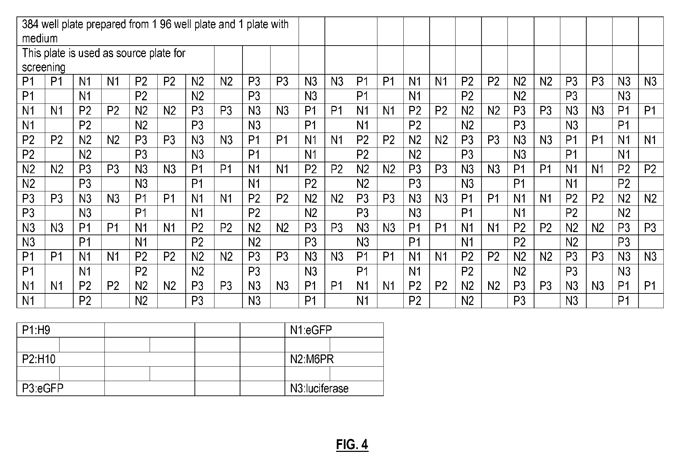

The control viruses were pipetted from 96 well KD control plates into 384 well plates using robotics. The final lay-out of the 384 well plate is depicted in Figure 4.

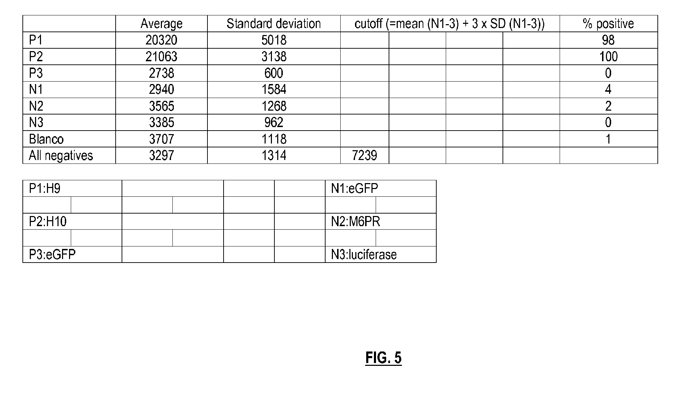

Figure 5 shows results from the automated screening procedure using the KD control plate. The mean and standard deviations of the KD negative controls (N1-N3) were used to calculate a cut-off for hit analysis, which was set at the mean for N1, N2, N3 ('All negatives') plus 3 times the standard deviation for 'All negatives'. The positive controls (P1 and P2), scored in more than 95% of the infected wells. The negative control viruses scored in less than 5% of the wells.

### Example 2. Screening of 7980 Ad-siRNA adenoviruses in the osteogenesis assay

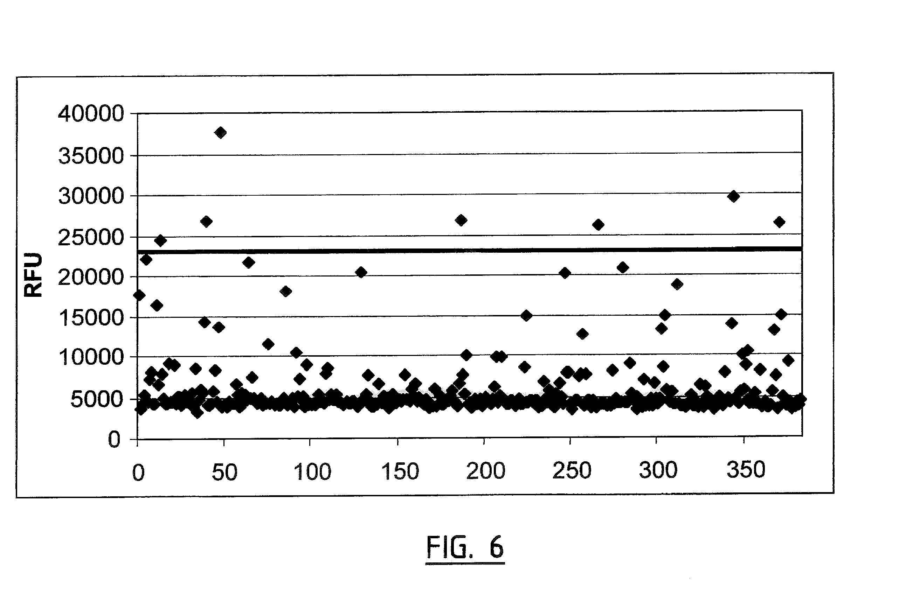

The optimized protocol for screening the SilenceSelect library is the following: on day 0, MPC cells are seeded in black 384 well plates with clear bottom (Costar or Nunc) in 60 µl medium at a density of 500 cells per well. One day later, 1 µl Ad-siRNA virus from the SilenceSelect™ collection, stored in 384 well plates (estimated titer of 2.5 x 10⁹ viral particals per ml) and 5 µl of Ad-hCAR solution (total MOI=155), dispensed in 96 well V-bottom plates, is transferred with the aid of a 96/384 channel dispensor (Tecan Freedom 200 equipped with TeMO96, TeMO384 and RoMa, Tecan AG, Switzerland) from the wells of a 96 well plate containing the Ad-hCAR solution to each of the wells of the 384 well plates containing MPCs. The KD control plate was run under the same conditions as the aliquot plates from the SilenceSelect collection. All Ad-siRNA viruses were screened in duplicate, with each singular on a different MPC plate. Plates were then incubated at 37°C. Four days post infection the medium containing the adenoviruses was replaced by fresh medium free of virus. Thirteen days post infection, the AP activity readout was performed. A typical result of a 384 well screening plate is depicted in Figure 6, in which the relative fluorescence units (RFU) are plotted for each of the data points of the 384 well plate on the Y-axis; while the numbers on the X-axis correspond to positions in the 384 well plate.

This duplicate screen was done twice, and all four data points were used for hit calling (see Example 3).

### Example 3. Target identification using the AP assay

After performing these 2 screens, the data obtained from measuring the AP activity were analyzed as follows: the background was calculated by taking the mean of the data points from all the plates except the control plate. A cut-off value for hit calling was calculated by adding 3 times the standard deviation of all data points, excluding the control plate. Each data point was analyzed for scoring above or under the cut-off. Only Ad-siRNAs inducing endogenous AP activity levels above the cut-off were of further interest. Hits were prioritized according to their scoring in single or duplo, in one or both of the screens. Data were collected for 7980 Ad-siRNA virus constructs representing 4091 independent genes. An overview of the constructs is given in Table 4.

One of the identified hits has been shown to be a bone anabolic factor before and therefore validates the assay:
H24-034: SRC

Marzia et al. (2000) showed that bone formation was increased in Src null mice compared to wild-type mice. Most relevant to this work, osteoblasts isolated from Src null mice or osteoblasts isolated from wild-type mice but transfected with Src-antisense oligonucleotides showed increased AP activity in vitro.

8 genes identified in the screen were targeted by 2 Ad-siRNAs. These genes are AVPR1B, FLJ22955, IL1F8, PPIA, USP38, C9, LOC254378 and BRS3 (see Table 4).

### Example 4. Quality control of the target Ad-siRNAs

The Ad-siRNA hits, hereafter referred to as 'targets' were subjected to further analysis to establish their therapeutic application as bone anabolic factor. A first step entailed a quality control on the Ad-siRNA selected for further analysis (this example). Other validation steps are the validation of targets at the mRNA level (Example 5), screening of the targets in osteogenesis assays such as the mineralization assay (Example 6), and development of additional Ad-siRNAs targeting the identified genes (Example 9). Targets that remain of interest after these validation assays are considered for drug discovery: assays are developed allowing the discovery and optimization of compounds that mimick the bone anabolic actions of the target Ad-siRNAs (Example 7). In addition, the anti-resorptive activities of the identified Ad-siRNAs are validated in osteoclast assays (Example 8).

### Quality control of target Ad-siRNAs:

Target Ad-siRNAs were propagated using PerC6 cells (Crucell, Leiden, The Netherlands) at a 96 well plate level, followed by rescreening these viruses at several MOIs in the primary assay (see Example 1) and by sequencing the siRNAs encoded by the target Ad-siRNA viruses.

PerC6/E2A cells were seeded in 96 well plates at a density of 40 000 cells per well in 180 µl PerC6/E2A medium. Cells were then incubated overnight at 39°C in a 10% C0₂ humidified incubator. One day later, cells were infected with 1 µl of crude cell lysate from SilenceSelect stocks containing target Ad-siRNAs. Cells were incubated further at 34°C, 10% CO₂ until appearance of cytopathic effect (as revealed by the swelling and rounding up of the cells, typically 7 days post infection). The supernatant was collected and the virus crude lysate was treated with proteinase K: 12 µl crude lysate was added to 4 µl Lysis buffer (1x Expand High Fidelity buffer with MgCl₂ (Roche Molecular Biochemicals, Cat. No 1332465) supplemented with 1 mg/ml proteinase K (Roche Molecular Biochemicals, Cat No 745 723) and 0.45% Tween-20 (Roche Molecular Biochemicals, Cat No 1335465) in sterile PCR tubes. These were incubated at 55°C for 2 h followed by a 15 min inactivation step at 95°C. For the PCR reaction, 1 µl lysate was added to a PCR master mix composed of 5µl 10x Expand High Fidelity buffer with MgCl₂, 0.5 µl of dNTP mix (10 mM for each dNTP), 1 µl of 'Forward primer' (10 mM stock, sequence: 5' CCG TTT ACG TGG AGA CTC GCC, SEQ ID NO: 245), 1 µl of 'Reverse Primer' (10 mM stock, sequence: 5' CCC CCA CCT TAT ATA TAT TCT TTC C, SEQ ID NO: 246), 0.2 µl of Expand High Fidelity DNA polymerase (3.5 U/µl, Roche Molecular Biochemicals) and 41.3 µl of H₂O. PCR was performed in a PE Biosystems GeneAmp PCR system 9700 as follows: the PCR mixture (50 µl in total) was incubated at 95°C for 5 min; each of 35 subsequent cycles ran at 95°C for 15 sec, 55°C for 30 sec, 68°C for 4 min.. A final incubation at 68°C was performed for 7 min. 5 µl of the PCR mixture was mixed with 2 µl of 6 x gel loading buffer, loaded on a 0.8% agarose gel containing 0.5 µg/pl ethidium bromide to resolve the amplification products. The size of the amplified fragments was estimated from a standard DNA ladder loaded on the same gel. The expected size was ∼500 bp.

For sequencing analysis, the siRNA constructs expressed by the target adenoviruses were amplified by PCR using primers complementary to vector sequences flanking the Sapl site of the pIPspAdapt6-U6 plasmid. The sequence of the PCR fragments was determined and compared with the expected sequence.

### Multiple MOI rescreen

The propagated target Ad-siRNAs were rescreened at several MOIs in the AP assay (see Example 1). The Ad-siRNAs had to score in duplo at at least one MOI to pass this quality control step.

All hits listed in Table 4 fulfilled this quality control step and thus:
a) showed the correct length of the PCR fragment
b) showed the correct sequence of the PCR fragment
c) induced AP activity in duplo at at least 1 MOI.

### Example 5. mRNA validation experiments for identified targets

An initial validation of the target Ad-siRNAs was carried out on RNA isolated from infected MPCs. First, the expression of the targets was analyzed in several isolates of primary human MPCs and osteoblasts (hOBs). Second, the knock-down of the target gene expression by the Ad-siRNA was verified at the mRNA level. Third, the upregulation of endogenous bone AP mRNA versus that of placental or intestinal AP mRNA was analyzed.

### MPC and osteoblast expression analysis for the identified targets profiling

Expression levels of target genes were determined in 4 different isolates of MPCs and 2 different isolates of hOBs. The MPCs and hOBs (obtained from Cambrex/Biowhittaker, Verviers, Belgium) were seeded at 3000 resp. 5000 cells/cm² in T180 flasks and cultured until they reached 80% confluency. The cells were washed with ice-cold PBS and harvested by adding 1050 µl SV RNA Lysis Buffer to a T180 flask. Total RNA was prepared using the SV Total RNA isolation System (Promega, Cat # Z31 00). The concentration of the total RNA was measured with the Ribogreen RNA Quantification kit (Molecular Probes, Leiden, The Netherlands, Cat No. R-11490). cDNA synthesis was performed using 40 ng total RNA per reaction using the TaqMan Universal PCR Master Mix, No AmpErase UNG, kit (Applied Biosystems, Warrington, UK,Part number 4324018). For each RT reaction a minus-RT reaction (negative control: no enzyme included in the reaction) was performed.

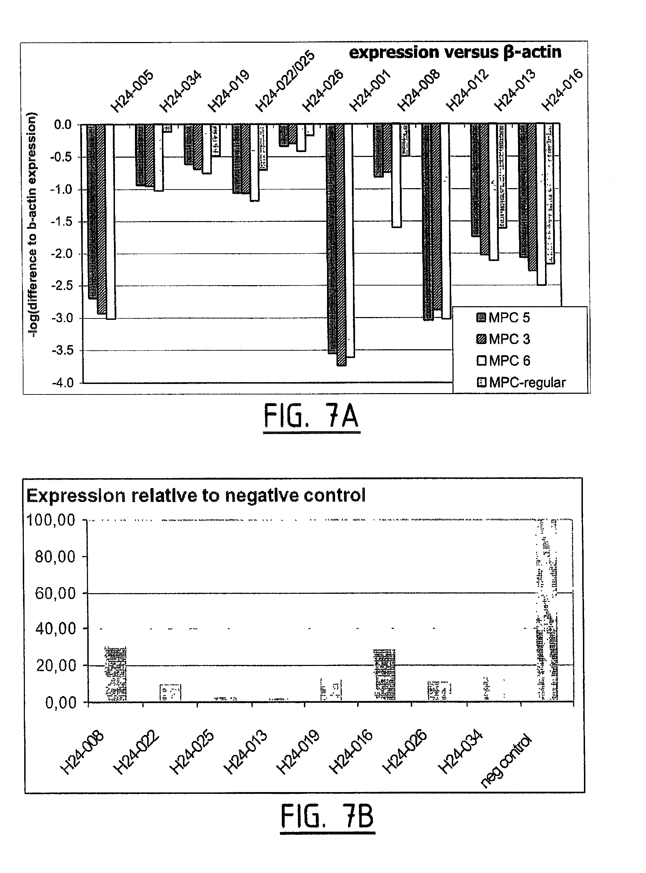

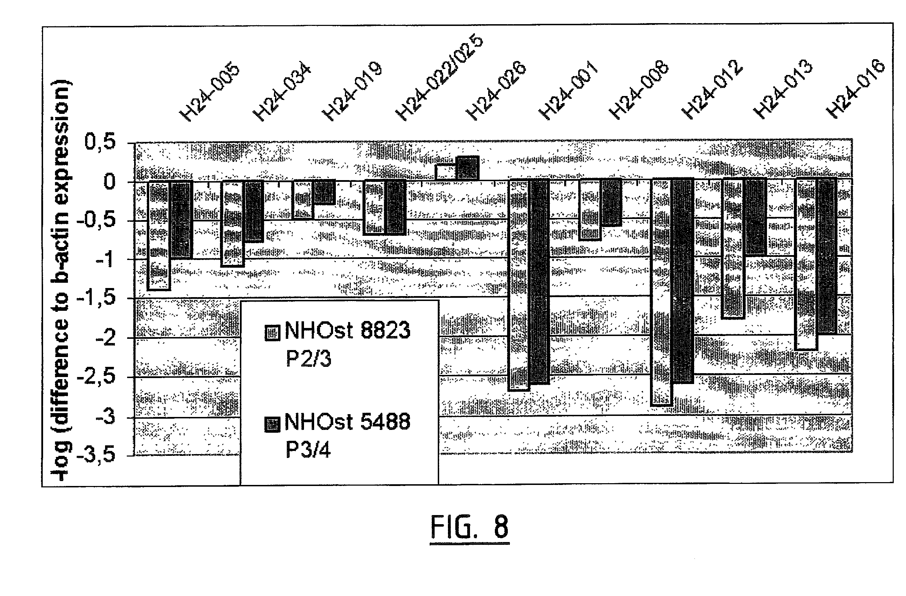

The real-time reverse transcriptase (rtRT)-PCR reaction was performed with gene specific primers on both cDNA and minus-RT samples, using the SYBR Green PCR Master Mix (Applied Biosystems, Warrington, UK, Part number 4309155). For the normalisation of the expression levels, a RT-PCR reaction was performed on human β-actin using the Human β-actin kit (Applied Biosystems, Warrington, UK, Part number 4310881 E). The following program was run on a real-time PCR apparatus (ABI PRISM 7000 Sequence Detection System): 10 min at 25°C, 30 min at 48°C, 5 min at 95°C. In Figures 7A and 8, relative expression levels for 10 genes are depicted for respectively MPC and hOB isolates. For the data in Figure 7A, total RNA was extracted from 4 different MPC isolates and used to analyze expression levels of target genes identified through the target Ad-siRNAs. RtRT-PCR compatible primer sets were developed for 10 genes and compared to expression levels of β-actin. Data are expressed as -log(difference to β-actin) (Y-axis). For the data presented in Figure 8, total RNA was extracted from 2 different hOB isolates.

### Analysis of the knock-down of the target gene expression by the Ad-siRNA was verified at the mRNA level.

To determine whether the target Ad-siRNAs result in knock-down of expression from the corresponding gene, total RNA was harvested from Ad-siRNA infected MPCs and gene expression was analyzed using gene-specific primers.

MPCs were seeded at 25 000 cells per well of a 24 well plate. After 24 hours the cells were infected with knock-down hit viruses or negative control viruses Ad-gPPARg and Ad-GL2.2 that knock down the expression of respectively PPARγ (all four known splice variants) and luciferase, both of which are not related to osteogenesis. For Ad-siRNAs, cells were co-infected with Ad-hCAR and Ad-siRNA crude lysates (MOI Ad-hCAR: 750; 40, 10, 3.3 µL virus with average titer of 2.5 x 10E9 virus particles/ml). At 5 dpi the medium was refreshed and at 14 dpi the cell lysates were prepared. Cells were processed for mRNA analysis as described in the previous section. mRNA levels for a specific gene were normalized for β-actin levels and compared to levels measured for the negative control Ad-siRNAs. An example of these kind of analyses is provided in Figure 7B. Data were normalized for β-actin expression levels and compared to endogenous gene expression for MPCs infected with negative control viruses (Y-axis: percent gene expression of endogenous gene; 100% = endogenous mRNA levels present in negative control sample).

### Analysis of the upregulation of endogenous bone AP mRNA versus that of placental or intestinal AP mRNA.

BAP is the physiologically relevant AP involved in bone formation. In order to determine whether the measured AP activities were due to upregulation of BAP expression or of another AP gene product, mRNA levels for all AP genes were analysed for infected MPCs.
mRNA levels were determined as described in the previous sections. The difference is in the primer set used (see Table 2): one set detects BAP ALPL (human alkaline phosphatase liver/bone/kidney) mRNA expression. Another set detects the expression of the 3 other AP genes (ALPI (human alkaline phosphatase intestinal), ALPP (human alkaline phosphatase placental (PLAP)), and ALPPL2 (human alkaline phosphatase placental-like)). ALPI, ALPP and ALPPL2 are highly similar at the nucleotide level and can therefore be amplified using one primer pair.

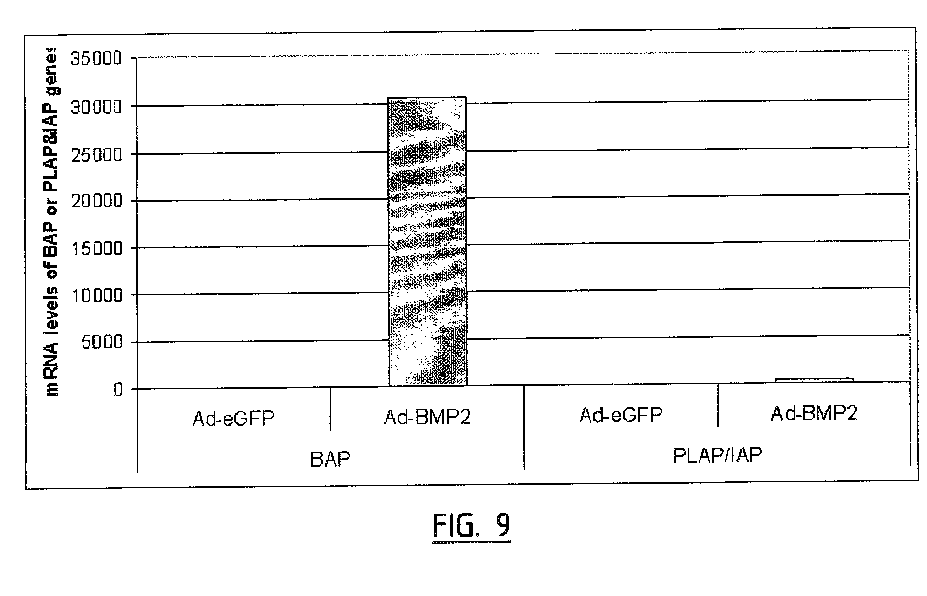

The primer pairs were first validated on RNA isolated from MPCs infected with Ad-eGFP and Ad-BMP2. Figure 9 illustrates the strong upregulation of BAP mRNA by Ad-BMP2 and the absence of upregulation of expression of any of the other AP genes. MPCs were infected in 24 well plate format using Ad-eGFP (negative control) or the osteogenic Ad-BMP2. Cells were harvested and RNA was prepared and subjected to rtRT-PCR using primer sets amplifying BAP mRNA or mRNA from the other 3 AP genes (PLAP/IAP). Ad-BMP2 strongly upregulates BAP mRNA levels but not the mRNA levels of the other 3 AP genes.

Both primer sets were then used to measure mRNA levels for all AP genes in RNA isolated from Ad-siRNA infected MPCs.

### Example 6. Mineralization

The process of osteogenesis consists of several successive events. During the initial phases of osteogenesis, bone alkaline phosphatase (BAP) becomes upregulated. It is however equally important to look at specific events occurring in later stages of osteogenesis such as mineralization.

### Assay setup

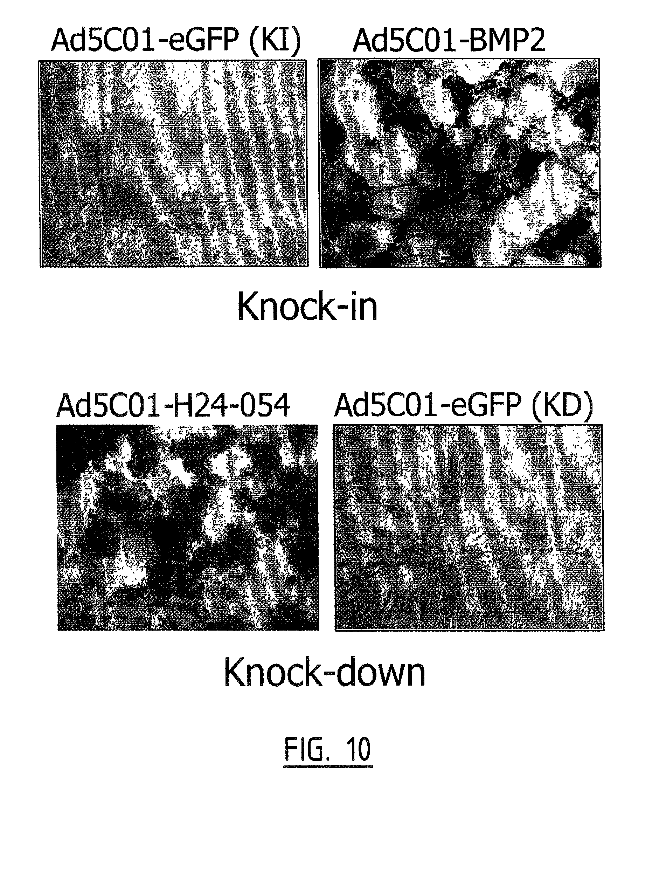

The process of osteogenesis consists of several successive events. During the initial phases of osteogenesis, bone alkaline phosphatase (BAP) becomes upregulated. Later, during differentiation, cells deposit (hydroxy)apatite (Ca²⁺-phosphate precipitate) on an extracellular matrix consisting mostly of collagen type I to form mineralized bone. In the bone cell mineralizing assay (BM assay), primary human MSCs are differentiated *in vitro* into mineralizing osteoblasts using BMP2 (recombinant or delivered by adenoviral transduction) as an osteogenic agent. Mineralization is then visualized by staining the MSCs with Alizarin Red, a dye with a high affinity for calcium (see Figure 10).

### Screening and hit calling

The following optimized protocol is used for screening Ad-siRNA and Ad-cDNA targets identified in the primary assay:

100,000 MPCs are seeded in each well of a 6 well plate in 2 ml MSC medium, containing 10% FCS. The next day, after incubation at 37°C, 10% CO₂ in a humidified incubator, cells are co-infected with AdC15-hCAR (final MOI of 750) and Ad-siRNA, Ad-cDNA or control viruses at a final MOI of 1250, 2500 and 5000. Cells are incubated at 37°C, 10% CO₂ in a humidified incubator for a further six days. Virus is removed and replaced by 2 ml fresh MSC medium, 10% FCS. Over the next 22 days, medium is refreshed 3 times in 2 weeks. Every other time, medium is refreshed half or completely. At 28 days after the start of the experiment, the conditioned medium is removed, cells are fixed using 10% paraformaldehyde and the monolayers stained with 1 mL of ∼1% Alizarin Red (Sigma, # A5533) in MilliQ water (pH adjusted to 4.2). Ad-eGFP, to assess infection efficiency, Ad-BMP2 as strong osteogenic inducer and Ad-H4-2 as a weak osteogenic factor are included in each experiment as negative and positive controls, respectively. Every experiment where Ad-H4-2 does not induce mineralization is entirely repeated.

The Ad-shRNAs that induced mineralization are presented in Table 6.

### Example 7. Drug discovery against the identified targets

Compounds are screened for binding to the polypeptides of the present invention. The affinity of the compounds to the polypeptides is determined in a displacement experiment. Such displacement experiments are well known in the art, and can be considered as a common technique among others to identify compounds that bind to polypeptides.

In brief, the polypeptides of the present invention are incubated with a labeled (radiolabeled, fluorescent- or antibody-labeled, or any other detectable label) ligand that is known to bind to the polypeptide and is further incubated with an unlabeled compound.

The displacement of the labeled ligand from the polypeptide is determined by measuring the amount of labeled ligand that is still associated with the polypeptide. The amount of the labeled ligand associated with the peptide is an indication of the affinity for the unlabeled compound.

The amount of labeled ligand associated with the polypeptide is plotted against the concentration of the unlabeled compound to calculate IC50 values. This value reflects the binding affinity of the unlabeled compound to its target, i.e. the polypeptides of the present invention.

Compounds are considered strong binders, when having an IC50 in the nanomolar and even picomolar range. Compounds that have an IC50 of at least 10 micromol or even better in the nmol to pmol range are applied in either the bone alkaline phosphatase assay (BAP) and/or in assays to determine their effect on the induction of osteoblast markers and osteoblast function. Compounds with a lower IC50 are generally considered as of less interest. The polypeptides of the present invention can be prepared in a number of ways depending on whether the assay will be run on cells, cell fractions or biochemically, on purified proteins. Such preparations are well known in the art, as are the different assays.

### Example 8. Osteoclast assays: validate anti-resorptive activity of identified targets

Throughout life, the skeleton is in a constant state of remodeling. Focal areas of bone are resorbed by osteoclasts and then replaced by bone matrix newly formed by osteoblasts. The development of osteoporosis is characterized by severe bone loss due to the deregulation of the balance between osteoclast and osteoblast activity, leading to an increased osteoclast-mediated bone resorption.

Osteoclasts emanate from cells of the monocyte/macrophage lineage. In vivo, the differentiation of osteoclast precursor cells towards osteoclasts is controlled by two central factors expressed by stromal cells (MPCs): receptor activator of NFκB ligand (RANKL) and osteoprotegerin (OPG). RANKL is a membrane bound ligand expressed on the surface of MPCs which drives osteoclast differentiation. OPG is a soluble decoy receptor for RANKL which inhibits osteoclast differentiation by scavenging active RANKL. The balance between RANKL and OPG expression by MPCs determines the level of osteoclast differentiation.

As MPCs control the differentiation of osteoclasts, it is important to know the effect of the identified target Ad-siRNAs on osteoclast differentiation or activity. Target Ad-siRNAs thatdecrease osteoclast differentiation/activity, are very valuable, as these are expected to increase bone apposition by two mechanisms: increase of differentiation / activity of osteoblasts and decrease in osteoclast activity. As illustrated by various precedents (Thirunavukkarasu et al., (2000) J Biol Chem 275 : 25163-72 ; Yamada et al., (2003) Blood 101 : 2227-34) such a pleiotropic effect of osteogenic factors can be expected.

### Osteoclast differentiation assay

The effect of osteogenic factors on osteoclastogenesis is evaluated through two types of assays.

In a first assay setup, a co-culture of MPCs with primary human mononuclear cells is performed. The effect of the infection of the MPC monolayer with a knock-down virus on its capacity to support osteoclastogenesis is evaluated. The desired effect is the following: knock-down of the Ad-siRNA target gene expression in the MPCs should inhibit osteoclast differentiation driven by a physiological trigger as *e.g*. a mixture of 10nM 1,25(OH)₂vitD₃ and 50nM M-CSF. The monocytes used can be derived from bone marrow or peripheral blood. In the present example, a differentiation experiment based on peripheral blood derived mononuclear cells (PBMCs) is described. MPCs (obtained from Cambrex/Biowhittaker, Verviers, Belgium) are seeded in 96 well plates (1000 cells per well) in α-MEM medium (GIBCO-Life Technologies) supplemented with 10% FBS and a day later, these are infected with a target Ad-siRNA. At least three days later, 100 000 PBMCs per well are added as well as M-CSF (R&D systems, 50ng/ml final concentration). Half the volume of medium is refreshed twice a week by medium + 50ng/ml M-CSF and 10nM 1,25(OH)₂vitD₃. Readout is performed 14 days after addition of the PBMCs to the coculture. Spontaneous osteoclast differentiation driven by the physiologically relevant mixture of triggers can be assessed by multiple readouts. Microscopic assessment of the number of 'TRAP positive', multinucleated cells per well is a generally accepted measure for the level of osteoclast differentiation. 'TRAP positive' means that the cells possess a tartrate resistant acidic phosphatase (TRAP) activity. To assess this, the coculture is subjected to an *in situ* TRAP staining performed according to the Acid Phosphatase detection kit (SIGMA, 386-A). Positive cells aquire a purple color upon treatment. As an alternative readout, a marker specific for mature osteoclasts is measured e.g. TRACP5b (tartrate resistant acidic phosphatase type 5b), calcitonin receptor (CTR) or Cathepsin K (CTSK). Measurement of the amounts of osteoclast-derived tartrate resistant acidic phosphatase protein (TRACP5b) in the coculture supernatant is performed by a commercially available ELISA (BoneTRAP assay, Sba sciences, Turku, Finland). CTR or CTSK are detected by immunocytochemistry, upon application of following general protocol. Medium is removed and the coculture is fixed (4% paraformaldehyde, 0.1 % TritonX-100, 4°C, 30 min), washed and blocking buffer (PBS + 1%BSA + 0.1 % Tween20) is added for an incubation of at least 4hrs. The blocking buffer is removed and the primary antibody directed against CathepsinK (e.g. Oncogene, IM55L) or Calcitonin receptor (e.g. Serotec, AHP635), dissolved at the desired concentration in a suited buffer (e.g. 0.05M Tris.HCl pH 7.4, 1 % BSA), is added to the wells. Incubation is performed overnight, 4°C. The mixture is removed, the cells washed (PBS + 0.1% Tween20) and the suited, HRP conjugated secondary antibody, diluted in the same buffer as the primary antibody, is added. After an incubation of at least 4hrs, a washing step is performed (PBS + 0.1 % Tween20) and luminol (a substrate for HRP yielding a luminescent signal: BM Chemiluminescence ELISA Substrate [POD] (luminol), Roche Diagnostics, Cat No 1582950) is added. After 5 min incubation, readout is performed with a luminometer (Luminoskan Ascent, Labsystem). The 2 assays described (assessment of the amount of multinuclear cells and immunochemistry for the detection of osteoclast-specific markers) allow to assess the differentiation of the mononuclear cells towards osteoclasts, but do not yield information about the bone resorptive activity of the osteoclasts formed.

Activity of the osteoclasts is measured in the pit formation assay. For this purpose, the co-culture and infection of cells is performed as described for assays described above with the difference that a bone-like substrate is present at the bottom of the well in which the co-culture is performed. This bone-like substrate can be a dentin slice (e.g. Kamiya Biomedical Company, Seattle (Cat No KT018)) or equivalent (Calcium carbonate coating, OAAS™, Gentaur; Biocoat™ Osteologic™, BD Biosciences) that is commercially available. The co-culture is performed for at least 14 days on the bone like substrate. Cells are then removed by treatment with sodium hypochlorite and the area resorbed by the osteoclasts (the resorption pit) can be assessed microsopically. This can be facilitated by the treatment of the surface of the dentin slice with toluidine blue.

In a second assay setup, the effect of the infection of the osteoclast precursor cells (PBMCs or BMMCs) with a hit virus on its ability to differentiate towards an osteoclast is measured in a monoculture assay. For this purpose, the monocytes (PBMCs or BMMCs) are seeded in a 384 well plate in αMEM medium supplemented with 10% serum and 25ng/ml recombinant M-CSF (R&D systems). One day after seeding, the cells are infected with target Ad-siRNAs. Four days after infection, recombinant RANKL is added to the wells (25ng/ml, R&D systems). Medium is refreshed twice a week. Fourteen days after addition of RANKL, the differentiation of the monocytes towards osteoclasts is measured using one of the readouts described for the former assay setup. This assay allows the identification of factors that are indispensable for the response of osteoclast precursor cells to M-CSF or RANKL.

### PBMC isolation

PBMCs are obtained from peripheral blood (obtained from patients after informed consent) subjected to the following protocol. Blood is aseptically poured into 50 ml Falcon tubes and spun at 3000 g for 10 min at 25°C. The buffy coat is then collected and diluted 1:1 with PBS. The diluted buffy coat is poured on top of 20 ml Lymphoprep (Sigma) contained in a 50ml Falcon tube. Upon centrifugation (35 min at 400 g at 25°C), a white layer of mononuclear cells on top of the Lymphoprep is collected and washed twice with PBS (centrifugation at 200 g, 10 min, 25°C) and rediluted in 7 ml PBS. This solution is pipetted onto a layer of 7ml of hyperosmolar Percoll gradient contained in a 15ml Falcon tube and centrifuged 35 min at 400 g at 25°C. The hyperosmolar Percoll gradient is prepared as follows : 1 volume of 1.5 M NaCl and 9 volumes of Percoll (Pharmacia, d=1,130 g/ml)are mixed. This mixture is added 1:1 to a PBS/Citrate buffer (NaH2PO41.49 mM, Na2HPO4 9.15 mM, NaCl 139.97 mM, Na-citrate (dihydrate) 13 mM, pH 7.2). After centrifugation, monocytes form a discrete ring on top of the gradient. Monocytes are collected and washed in culture medium. Cells are then ready to use in assays.

### Example 9. Analysis of 'off-target' knock down effect.

SiRNAs exert knock-down of gene expression through a recently discovered and partially understood mechanism. It is generally accepted that the specific annealing of the siRNA sequence to mRNA is responsible for a gene-specific 'on-target' knock-down. However, it cannot be excluded yet that limited mismatching between the siRNA and another mRNA can induce 'off-target' down-regulation of gene expression.

In order to exclude that the knock-down of (an) 'off-target' mRNA(s) was responsible for the observed osteogenic effect, additional siRNAs/shRNAs were designed for 38 targets that induced mineralization (Example 6) using stringent design criteria. The additional Ad-shRNAs were then tested in the BAP assay.

To address the question of possible 'off-target' effects, additional siRNA sequences were designed that align perfectly with the mRNA targeted by the original siRNA. Preferred siRNA sequences do not align to other mRNAs. However, in some cases only siRNAs could be designed that showed some overlap to other mRNAs. For siRNAs that aligned to a minimal number of 'off-target' mRNAs (maximum of 2 basepairs non-identity checked for every position of the 19mer) the following rules were applied: the putative 'off-target' mRNAs must be different from the putative 'off-target' mRNAs identified for the original siRNA; and putative 'off-target' mRNAs must be different from the putative 'off-target' mRNAs identified for all original target siRNAs. The only exception to these rules made during the course of these experiments were siRNAs designed for PPIA.

For each of the 38 selected target genes, 7 additional siRNAs were designed and processed to derive recombinant adenoviruses. All siRNAs were sequenced upon cloning, to verify their identity and exclude errors due to the oligonucleotide synthesis.

261 Ad-shRNAS were successfully generated and tested in the BAP assay at 3 MOIs in 2 independent experiments, in parallel with the original 38 Ad-shRNAs.

Recombinant adenoviruses encoding the designed shRNAs (Ad-shRNAs) were produced, titered, aliquoted in 96 well plates and stored at -80°C. These plates were processed in the primary BAP assay as follows:

MPC cells were seeded with a Multidrop 384 (Labsystems) in black 384 well plates with clear bottom (Costar or Nunc) in 60 µl MSC medium containing 10% fetal calf serum (Progentix, The Netherlands), at a density of 500 cells per well. One day later, a 96 well plate containing aliquoted Ad-shRNAs and another containing negative and positive control viruses (knock-down control plate) were thawed and virus aliquots transferred to the MPC plate using a 96-channel dispenser (Tecan Freedom 200 equipped with a TeMO96 and a RoMa plate handler, Tecan AG, Switzerland). For the control plate, 1 µL virus stock (average titer of of 2 x 10⁹ viral particles per ml) was transferred to the 384 well screening plates. On the control plate (see Figure 3), negative (N1, N2, N3) and positive (P1, P2) control viruses are diagonally distributed over the plate. N1, N2, N3: Ad-siRNAs targeting the eGFP, mannose-6-phosphate-receptor and luciferase mRNAs, respectively. P1 and P2: Ad-siRNAs targeting the PRKCN (H24-010) and MPP6 (H24-011) mRNAs. P3: Ad-eGFP: overexpression of eGFP allows monitoring of infection efficiency.

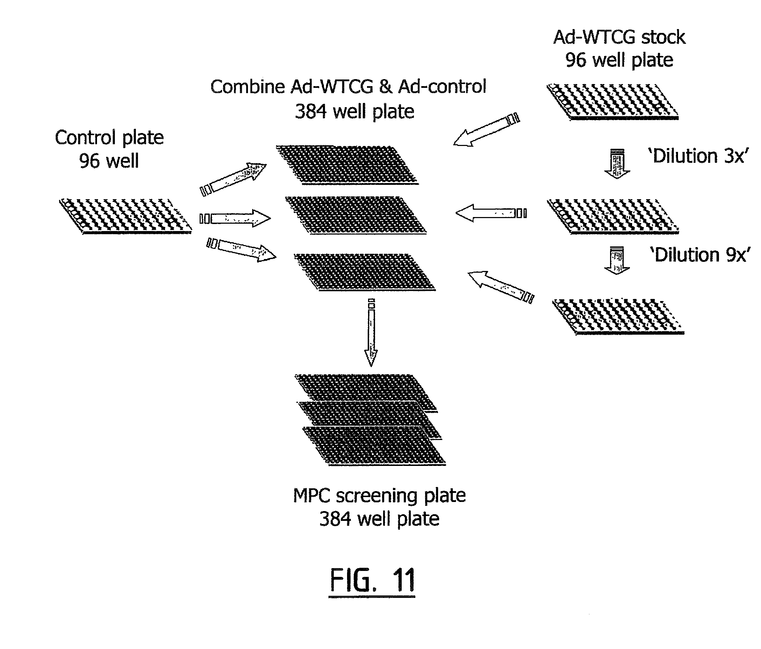

The Ad-shRNAs were screened at 3 multiplicities of infection (MOIs): 12,000, 4,000 and 1,333. Ad-shRNAs were aliquotted into the inner wells of a 96 well plate at a titer of 1.5 x 10⁹/4 µl and 1/3 and 1/9 dilutions were derived of this plate (Figure 11). The resulting 3 plates were used to infect 3 x 384 well plates seeded with MSCs, in the A1 and B2 positions of each quadrant using robotics. The viruses from the control plate were pipetted in the A2 and B1 positions.

Next, 5 µl of adenovirus expressing the human coxsackie and adenovirus receptor (hCAR) (AdC15-hCAR/AdC20-hCAR) was transferred into these wells (final MOI of 155) from a 96 well V-bottom plate with the aid of the 96-channel dispenser.

Plates were then incubated at 37°C, 10% CO₂ in a humidified incubator for four days. Four days post infection, the medium containing the adenoviruses was replaced by 60 µl fresh MSC medium containing 10% FCS free of virus. After an additional nine days of incubation, medium was removed, 15 µL of a 4-methylumbelliferylphosphate solution (Sigma, # M3168) was added to each well and the fluorescence of 4-methyl-umbelliferone released by the alkaline phosphatase activity was measured after 15 min incubation at 37°C using a fluorimeter (excitation: 360 nm; emission: 440 nm; FluoStar, BMG).

All Ad-shRNAs viruses were screened in duplicate at 3 MOIs in two independent screens. Thresholds were calculated for hit calling using either all negative controls present in one screening round ('Global' analysis) or using the negative controls present on one screening plate ('Local' analysis). Threshold was set at a BAP signal higher than the mean plus 3 times the standard deviation of negative controls. The two individual datapoints for each virus in the batch were analyzed independently. Hits were selected if one Ad-shRNAs scored at least at one MOI in duplicate in at least one of the 2 screens above the threshold (see Table 3).

A 'Global' analysis of the data identified 61 siRNAs targeting 32 loci and a 'Local' analysis' identified 84 siRNAs targeting 35 loci. The identity of the 38 selected genes is presented in Table 1 together with the final number of siRNAs that scored in the BAP assay. In this Table, the numbers indicate all siRNAs that scored in the BAP assay, including the original 38 siRNAs. Based on the 'Global' respectively 'Local' analysis, an average of 2.61 and 3.21 constructs was finally identified for each of the 38 validated targets.

All original 38 Ad-shRNAs scored in the BAP assay based on both the 'Global' and 'Local' analysis.

In conclusion, additional Ad-shRNAs targeting 38 selected targets were designed and constructed. Negative controls present on the control plates were used per plate ('Local' analysis) or per batch of plates ('Global' analysis) to determine the cutoff for hit calling.

The 'Global' analysis resulted in 61 viruses that scored positive in the BAP assay, confirming 32 of the 38 validated targets. The 'Local' analysis resulted in 84 viruses that scored positive in the BAP assay, confirming 35 of the 38 validated targets. All original 38 Ad-shRNA viruses scored in the BAP assay when using either the 'Global' or the 'Local' analysis. The targets LOC160662, PPIA and SLC39A4 were identified only in the 'Local' analysis.

**Table 1: Primer sets used:**

| Gene | primer name | sequence | SEQ ID |
|---|---|---|---|
| CALCRL | CALCRL_For | catggcgacctgaaggaaag | 221 |
| CALCRL | CALCRL_Rev | agagaccaaaagaccctggaagt | 222 |
| SRC | SRC_For | acagcggcggcttctaca | 223 |
| SRC | SRC_Rev | catcggcgtgtttggagtagt | 224 |
| PSMB3 | PSMB3_For | ATCCGGATCACCTGTTTGAAAC | 225 |
| PSMB3 | PSMB3_Rev | GTGGTGATTTTGTCCTTCTCGAT | 226 |
| HP43.8KD | HP43.8KD_For | CCATACACAGAGGGAAGCATACG | 227 |
| HP43.8KD | HP43.8KD_Rev | CAGTCTTGCTGTGATCTGGGAGTA | 228 |
| APEX | APEX_For | GCATAGGCGATGAGGAGCAT | 229 |
| APEX | APEX_Rev | GACCTCGGCCTGCATTAGG | 230 |
| GPR38 | GPR38_For | CATCGTCGCTCTGCAACTTTT | 231 |
| GPR38 | GPR38_Rev | CCGCTCTGTACTTCTTTGAAATGA | 232 |
| ROCK2 | ROCK2_For | CCTGGTGGAGACCTTGTAAACCT | 233 |
| ROCK2 | ROCK2_Rev | AGCAAGAACAACTTCAGCAGTGTAA | 234 |
| CXCR6 | CXCR6_For | GCCATGACCAGCTTTCACTACA | 235 |
| CXCR6 | CXCR6_Rev | GTTAAGGCAGGCCCTCAGGTA | 236 |
| OPN3 | OPN3_For | CTAACCGTGCTGGCCTATGAAC | 237 |
| OPN3 | OPN3_Rev | CAGGCCCAGGAAAAATTGATC | 238 |
| FUK | FUK_For | TTCGCGATCAGCCCCTTAC | 239 |
| FUK | FUK_Rev | ACTCACTGGCTGAGGAGGTCAT | 240 |

Gene-specific primers were developed for the genes denoted in the left column. Their orientation (For = sense, Rev = antisense) is indicated in the second column. The sequence of the primers is given in the third column.

**Table 2. Primers used for identification of PLAP and other phosphatases**

| **Name** | **Sequence** | **SEQ ID NO:** |
|---|---|---|
| JDO-05F (PLAP) | TTCCAGACCATTGGCTTGAGT | 241 |
| JDO-05bis R (PLAP/ALPI/ALPPL2) | ACTCCCACTGACTTTCCTGCT | 242 |
| JDO-21F (BAP) | CATGCTGAGTGACACAGACAAGAAG | 243 |
| JDO-21 R (BAP) | TGGTAGTTGTTGTGAGCATAGTCCA | 244 |

**Table 3: Identification of multiple siRNAs for selected validated targets in the BAP assay.**

| Gene ID | Global analysis - Redundacy | Local analysis - Redundancy |
|---|---|---|
| APG4B | 2 | 3 |
| C13orf6 | 2 | 2 |
| C20orf121 | 3 | 4 |
| CCR1 | 3 | 4 |
| CCR3 | 2 | 2 |
| CEL | 2 | 4 |
| CLIC6 | 2 | 2 |
| CSNK1G1 | 2 | 3 |
| DUSP5 | 2 | 2 |
| FLJ22955 | 3 | 4 |
| FRK | 3 | 3 |
| GPR124 | 2 | 3 |
| GPR23 | 2 | 3 |
| GPR64 | 4 | 5 |
| Grin2a - GRIN2A | 7 | 6 |
| GZMK | 2 | 2 |
| HRMT1L3 | 2 | 2 |
| IRAK2 | 4 | 4 |
| LOC160662 - LST-3 | 1 | 2 |
| LOC167417 - GPR150 | 4 | 5 |
| LOC254378 | 3 | 4 |
| MAP3K9 | 4 | 5 |
| MLNR | 4 | 3 |
| MMP23B -MMP23A | 3 | 2 |
| MNK2 | 2 | 3 |
| OR1A2 | 4 | 6 |
| PPIA | 1 | 3 |
| RASD1 | 4 | 5 |
| RDH11 | 3 | 4 |
| SENP3 | 4 | 4 |
| SLC16A3 | 2 | 3 |
| SLC26A8 | 1 | 1 |
| SLC39A4 | 1 | 2 |
| SLC4A10 | 1 | 1 |
| TAO1 | 3 | 4 |
| TAS1R3 | 2 | 3 |
| TRPM6 | 1 | 1 |
| ULK1 | 2 | 3 |

**Table 4. Overview of isolated knock-down constructs and corresponding target gene sequences**

| | | | | | | **SEQ ID NO:** |
|---|---|---|---|---|---|---|
| Hit ID | KD Target Sequence | Target Gene Symbol | GenBank Nucleotide Accession | GenBank Polypeptide Accession | Name | KD construct |
| H24-049 | GTACCTGCAGGTGCTCAGC | AVPR1B | NM_000707 | NP_000698 | arginine vasopressin receptor 1 B | 1 |
| H24-225 | ATGGGCTTCAACAGCCACC | AVPR1B | NM_000707 | NP_000698 | arginine vasopressin receptor 1 B | 2 |
| H24-001 | CAACTTGTACCTGGGCAGC | GPR38 | NM_001507 | NP_001498 | G protein-coupled receptor 38 | 3 |
| H24-002 | GGTGAAGGAGGTGCTGGAC | STK11 - LKB1 | NM_000455 - | NP_000446 | serine/threonine kinase 11 (Peutz-Jeghers syndrome) | 4 |
| | | | SK208 | | | |
| H24-003 | GAATCTCTTCCGCAAGATC | HSM801665 - | AL136697- | NP_076951 | hypothetical protein MGC8407 | 5 |
| | | MGC8407 | NM_024046 | | | |
| H24-004 | CATGCTGTTTGAGAGCATC | MKNK2 - MNK2 | NM_017572- | NP_060042- | MAP kinase-interacting serine/threonine kinase 2 | 6 |
| | | | SK236 - | NP_951009 | | |
| | | | NM_199054 | | | |
| H24-005 | TTTGTGCTGATTCCATGGC | CALCRL | NM_005795 | NP_005786 | calcitonin receptor-like | 7 |
| H24-006 | GACGGTGTTAATGATAGCC | CSNK1G1 - | NM_022048 - | NP_071331 | casein kinase 1, gamma 1 | 8 |
| | | CK1g1 | SK647 | | | |
| H24-007 | CTTCGGCACTCCTGAGTTC | HSA247087 - | AJ247087 - SK536 - | NP_872299 | myosin light chain kinase | 9 |
| | | caMLCK - MLCK NM_182493 | | | | |
| H24-008 | GCACAGTTTGAGAAGCAGC | ROCK2 | NM_004850 | NP_004841 | Rho-associated, coiled-coil containing protein kinase 2 | 10 |
| H24-009 | ACGCAAAGTGGCCAGGAGC | IPT | NM_017646 | NP_060116 | tRNA isopentenyltransferase 1 | 11 |
| H24-010 | CGATGTGCCTTCAAGATTC | PRKCN - PKD3 | NM_005813 - SK489 | NP_005804 | protein kinase C, nu | 12 |
| H24-011 | GTGCACGGATTCAGAGAGC | MPP6 | NM_016447 | NP_057531 | membrane protein, palmitoylated 6 | 13 |
| H24-012; | CTTCTACACGTCCATGCTC | TYMSTR | NM_006564 | NP_006555 | G protein-coupled receptor | 14 |
| H24-161 | | | | | | |
| H24-013 | CAACCTGCTGGTGCTCGTC | OPN3 | NM_014322 | NP_055137 | opsin 3 (encephalopsin, panopsin) | 15 |
| H24-014 | CTCTCTTAGATCTGGAACC | GZMK | NM_002104 | NP_002095 | granzyme K (serine protease, granzyme 3; tryptase II) | 16 |
| H24-015 | AGCAGGAAGGCGGACATAC | AF073344 - | AF073344 - | NP_006528 | ubiquitin-specific protease 3 - ubiquitin specific | 17 |
| | | USP3 | NM_006537 | | protease 3 | |
| H24-016 | CTCCAGCTGAGTGAGCAGC | FUK | NM_145059 | NP_659496 | L-fucose kinase | 18 |
| H24-017 | TAAACCAGCAGAGGAGCTC | MGC26954 | NM_145025 | NP_659462 | hypothetical protein MGC26954 | 19 |
| H24-018 | TCAGGTAGTTGGTTCTGAC | F13A1 | NM_000129 | NP_000120 | coagulation factor XIII, A1 polypeptide | 20 |
| H24-019 | CTGCGCCGAACAAATGTAC | PSMB3 | NM_002795 | NP_002786 | proteasome (prosome, macropain) subunit, beta type, | 21 |
| | | | | | 3 | |
| H24-020 | TGTGGCGACTTGTGCACAC | CLPX | NM_006660 | NP_006651 | ClpX caseinolytic protease X homolog (E. coli) | 22 |
| H24-021 | TCTCTCAGTGTAGAATGCC | FLJ14906 | NM_032859 | NP_116248 | hypothetical protein FLJ14906 | 23 |
| H24-022 | CCAGGAGGTGAAACCACAC | HP43.8KD | NM_032557 | NP_115946 | HP43.8KD protein | 24 |
| H24-023 | TCAGGCGACTACTTTACTC | USP9X | NM_004652 - NM_021906 | NP_004643 - NP_068706 | ubiquitin specific protease 9, X chromosome (fat facets-like Drosophila) | 25 |
| H24-024 | GTGTACTGGTACAAGGACC | MMP23A - | NM_004659 - | NP_004650 - | matrix metalloproteinase 23A - matrix | 26 |
| | | MMP23B | NM_006983 | NP_008914 | metalloproteinase 23B | |
| H24-025 | GAGCAGGTTTCCAAAGGAC | HP43.8KD | NM_032557 | NP_115946 | HP43.8KD protein | 27 |
| H24-026 | TCTCTCATCAATACTGGTC | APEX | NM_001641 - NM_080648 - NM_080649 | NP_001632 - NP_542379 - NP_542380 | APEX nuclease (multifunctional DNA repair enzyme) | 28 |
| H24-027 | TGCAGAGCAGACAAGTGGC | ADAMTS19 | NM_133638 | NP_598377 | a disintegrin-like and metalloprotease (reprolysin type) | 29 |
| | | | | | with thrombospondin type 1 motif, 19 | |
| H24-028 | TCATGTTGACCAAGCAAGC | ADAM22 | NM_004194 - | NP_004185 - | a disintegrin and metalloproteinase domain 22 | 30 |
| | | | NM_016351 - | NP_057435 - | | |
| | | | NM_021721 - | NP_068367 - | | |
| | | | NM_021722 - | NP_068368 - | | |
| | | | NM_021723 | NP_068369 | | |
| H24-029 | CTATGCCATCACCTTCTGC | LOC254378 | XM_174812 | XP_174812 | LOC254378 | 31 |
| H24-030 | TGTGCCGAAGGATGTAAGC | LOC137491 | XM_070459 | XP_070459 | similar to a disintegrin and metalloprotease domain 25 (testase 2) | 32 |
| H24-031 | CCGGGACATAACTAAATCC | LOC138529 | XM_070951 | XP_070951 | similar to bile salt-dependent lipase oncofetal isoform | 33 |
| H24-032 | AGCAGGCTATGGGATCAAC | C9 | NM_001737 | NP_001728 | complement component 9 | 34 |
| H24-033 | CCACAAGGTTGCAGCATTC | XYLB | NM_005108 | NP_005099 | xylulokinase homolog (H. influenzae) | 35 |
| H24-035 | GGGCTCAGCCAGGAGATTC | CABC1 - ADCK3 | NM_020247 - | NP_064632 | chaperone, ABC1 activity of bc1 complex like (S. | 36 |
| | | | SK609 | | pombe) | |
| H24-036 | CAGGTAGACATGGCGGCAC | FRK | NM_002031 | NP_002022 | fyn-related kinase | 37 |
| H24-037 | CTCTCCAGGACACTGACTC | GPR113 | NM_153835 | NP_722577 | G protein-coupled receptor 113 (GPR113) | 38 |
| H24-038 | GCACGATTTGGAGGTCGCC | ULK1 | NM_003565 | NP_003556 | unc-51-like kinase 1 (C. elegans) | 39 |
| H24-039 | CGCTCTGGAGTCTCTCTCC | NTRK1 | NM_002529 | NP_002520 | neurotrophic tyrosine kinase, receptor, type 1 | 40 |
| H24-040 | CCCCAACTACACGGAGTTC | GSK3A | NM_019884 | NP_063937 | glycogen synthase kinase 3 alpha | 41 |
| H24-041 | GGACTCTCAGTTCAGCATC | PIK3C2B | NM_002646 | NP_002637 | phosphoinositide-3-kinase, class 2, beta polypeptide | 42 |
| H24-042 | CTTTGCGATACATGAGCCC | PRKR | NM_002759 | NP_002750 | protein kinase, interferon-inducible double stranded RNA dependent | 43 |
| H24-043 | CTTCCTGAAGACCAGGTTC | STK36 - | AF200815 - | NP_056505 | FUSED serine/threonine kinase | 44 |
| | | AF200815 - | AX040393 - SK199 - | | | |
| | | AX040393 - | NM_015690 | | | |
| | | Fused | | | | |
| H24-044 | GAATGCTGGCAACAAGACC | HSA311797 - | AJ311797 - | NP_115811 | mixed lineage kinase 4alpha - mixed lineage kinase | 45 |
| | | HSA311798 - | AJ311798 - SK691 - | | 4beta | |
| | | MLK4 - | NM_032435 | | | |
| | | KIAA1804 | | | | |
| H24-045 | GAAGATGTCAACGCCCATC | CSNK1 E - CK1e | NM_001894 - | NP_001885 - | casein kinase 1, epsilon | 46 |
| | | | SK084 - | NP_689407 | | |
| | | | NM_152221 | | | |
| H24-046 | TTCTACGCACCAGAACTCC | CASR | NM_000388 | NP_000379 | calcium-sensing receptor (hypocalciuric hypercalcemia | 47 |
| | | | | | 1, severe neonatal hyperparathyroidism) | |
| H24-047 | TGACGGTTGCATTTGCCAC | GNRHR | NM_000406 | NP_000397 | gonadotropin-releasing hormone receptor | 48 |
| H24-048 | CCAGACCTCGAGCAACTCC | ADRA1B | NM_000679 | NP_000670 | adrenergic, alpha-1B-, receptor | 49 |
| H24-050 | GGAGTGCAATCTGGTTTAC | DRD1 | NM_000794 | NP_000785 | dopamine receptor D1 | 50 |
| H24-051 | CAGTCAGTGCAACAGTGTC | DRD3 | NM_000796 - | NP_000787 - | dopamine receptor D3 | 51 |
| | | | NM_033658- | NP 387507 - | | |
| | | | NM_033659 - | NP_387508 - | | |
| | | | NM_033663 | NP_387512 | | |
| H24-052 | CGTGCTTGTCATGTTCGGC | OPRD1 | NM_000911 | NP_000902 | opioid receptor, delta 1 | 52 |
| H24-053 | TGACAGGTTCCGTCTGGGC | TACR1 | NM_001058 | NP_001049 | tachykinin receptor 1 | 53 |
| H24-054 | GTACCTGCGGCAGTTGTTC | CCR1 | NM_001295 | NP_001286 | chemokine (C-C motif) receptor 1 | 54 |
| H24-055 | GACCATCACCATCCTGGCC | FZD2 | NM_001466 | NP_001457 | frizzled homolog 2 (Drosophila) | 55 |
| H24-056 | GCAGGCAGAGGACAGATTC | BRS3 | NM_001727 | NP_001718 | bombesin-like receptor 3 | 56 |
| H24-057 | CAAAGCCATGCCCGTAAGC | BRS3 | NM_001727 | NP_001718 | bombesin-like receptor 3 | 57 |
| H24-058 | AAGTGCCCTGGCCTTCTTC | FPR1 | NM_002029 | NP_002020 | formyl peptide receptor 1 | 58 |
| H24-059 | CTACCACAAGCAGGTGTCC | FZD5 | NM_003468 | NP_003459 | frizzled homolog 5 (Drosophila) | 59 |
| H24-060; | TGCGTGCTTCTTTGTGGGC | SMOH | NM_005631 | NP_005622 | smoothened homolog (Drosophila) | 60 |
| H24-168 | | | | | | |
| H24-061 | CTACGTCATCCTGTGCCTC | MC5R | NM_005913 | NP_005904 | melanocortin 5 receptor | 61 |
| H24-062; | TTCGGACACCCACAAATGC | RRH | NM_006583 | NP_006574 | retinal pigment epithelium-derived rhodopsin homolog | 62 |
| H24-166 | | | | | | |
| H24-064; | GTTGTCCTGTTCTGACGTC | OR1A2 | NM_012352 | NP_036484 | olfactory receptor, family 1, subfamily A, member 2 | 63 |
| H24-164 | | | | | | |
| H24-065 | GCAGGCTTTCGAGTATGGC | PACE4 | NM_002570 - | NP_002561 - | paired basic amino acid cleaving system 4 | 64 |
| | | | NM_138319 - | NP_612192 - | | |
| | | | NM_138320 - | NP_612193- | | |
| | | | NM_138321 - | NP_612194 - | | |
| | | | NM_138322 - | NP_612195- | | |
| | | | NM_138323 - | NP_612196- | | |
| | | | NM_138324 - | NP_612197- | | |
| | | | NM_138325 | NP_612198 | | |
| H24-066 | CCTCTCTGTCAACACATGC | LGR7 | NM_021634 | NP_067647 | leucine-rich repeat-containing G protein-coupled | 65 |
| | | | | | receptor 7 | |
| H24-067 | ATGACGCTACGCAAGAACC | BBP | NM_032027 | NP_114416 | beta-amyloid binding protein precursor | 66 |
| H24-068 | AGCGGGTGCTTACATTGCC | PSMB5 | NM_002797 | NP_002788 | proteasome (prosome, macropain) subunit, beta type, | 67 |
| | | | | | 5 | |
| H24-069 | GCAAGAATGCACAGAGAGC | RARB | NM_000965 - | NP_000956 - | retinoic acid receptor, beta | 68 |
| | | | NM_016152 | NP_057236 | | |
| H24-070 | TTTGTGCTGACAGCTGCTC | CTLA1 | NM_033423 | NP_219491 | similar to granzyme B (granzyme 2, cytotoxic T- | 69 |
| | | | | | lymphocyte-associated serine esterase 1) (H. sapiens) | |
| H24-071 | CACCTGCTTTCTCAATGCC | KIAA1453 | NM_025090 | NP_079366 | KIAA1453 protein | 70 |
| H24-072 | GGTTCTCTGACTCGAACAC | FETUB | NM_014375 | NP_055190 | fetuin B | 71 |
| H24-073 | AGCACCTCGCTGACATTCC | SENP3 | NM_015670 | NP_056485 | sentrin/SUMO-specific protease 3 | 72 |
| H24-074 | CCTGGGCAACACCTGCTAC | DKFZP586D222 3 | NM_018561 | NP_061031 | DKFZP586D2223 protein | 73 |
| H24-075 | CTTAAGGTGGACCTGTGGC | CPA6 | NM_020361 | NP_065094 | carboxypeptidase A6 | 74 |
| H24-076 | TTGCACAGAAAGGAGGTGC | SPPL2A | NM_032802 | NP_116191 | putative intramembrane cleaving protease | 75 |
| H24-077 | GCCCGCAGTCTTACACAAC | LOC121302 | XM_062575 | XP_062575 | similar to protease | 76 |
| H24-078 | GCTTCTGGTGGAGAAGGAC | TGM6 | NM_198994 | NP_945345 | transglutaminase 6 | 77 |
| H24-079 | GTGTATGAAGTGGTCCACC | LST-3 | XM_292093 | XP_292093 | liver-specific organic anion transporter 3 | 78 |
| H24-080 | GAAATCTCACTGCTTCGAC | LOC160131 | XM_090078 | XP_090078 | similar to caspase 1, isoform alpha precursor; | 79 |
| | | | | | interleukin 1-beta convertase; interleukin 1-B | |
| | | | | | converting enzyme; IL1B-convertase | |
| H24-081 | CACTTTATAAGCCTGCGGC | LOC284964 | XM_209423 | XP_209423 | similar to bA395L14.5 (novel phosphoglucomutase like | 80 |
| | | | | | protein) | |
| H24-082 | TGGGCATCCTGGCTGTAAC | LOC254378 | XM_174812 | XP_174812 | LOC254378 | 81 |
| H24-083 | ATAGACTGCAGAATGAGCC | C9 | NM_001737 | NP_001728 | complement component 9 | 82 |
| H24-084 | CAGTGCCAAGAAGGAGCCC | NAV2 | NM_018162 - | NP_060632- | neuron navigator 2 | 83 |
| | | | NM_145117 | NP_660093 | | |
| H24-085 | TGTGCTCGAAGGAGGAATC | D13S106E | NM_005800 | NP_005791 | highly charged protein D13S106E | 84 |
| H24-086 | ATGTATGGATTCCAGCACC | BCHE | NM_000055 | NP_000046 | butyrylcholinesterase | 85 |
| H24-087 | GCTCTGCTATGTGTCAGTC | HSHIN1 | NM_017493 - | NP_059963 - | Hin-1 | 86 |
| | | | NM_199324 | NP_955356 | | |
| H24-088 | TGTGACTAAGCTGGAAGAC | PKDREJ | NM_006071 | NP_006062 | polycystic kidney disease (polycystin) and REJ (sperm | 87 |
| | | | | | receptor for egg jelly homolog, sea urchin)-like | |
| H24-089 | TGCGCACAATACTGGCCAC | PTPN1 | NM_002827 | NP_002818 | protein tyrosine phosphatase, non-receptor type 1 | 88 |
| H24-090 | TTCCCGCTACCAGACCTAC | GRIK4 | NM_014619 | NP_055434 | glutamate receptor, ionotropic, kainate 4 | 89 |
| H24-091 | AGCATGGTCATTCACATCC | KCNK9 | NM_016601 | NP_057685 | potassium channel, subfamily K, member 9 | 90 |
| H24-092 | ATGCAGGTCCATATGTGAC | TRPM6 | NM_017662 | NP_060132 | transient receptor potential cation channel, subfamily | 91 |
| | | | | | M, member 6 | |
| H24-093 | CCTTTCTCTGAACACGGAC | ATR | NM_001184 | NP_001175 | ataxia telangiectasia and Rad3 related | 92 |
| H24-094 | GTCAGGCTGAATGAGGAGC | PAK6 | NM_020168 - | NP_064553 | p21(CDKN1A)-activated kinase 6 | 93 |
| | | | SK429 | | | |
| H24-095 | CAGGTTCTCCTCAAACGGC | TAS1R3 | XM_371210 | XP_371210 | taste receptor, type 1, member 3 | 94 |
| H24-096 | GCATAGGAGTGGTCATATC | LOC223021 | XM_167349 | XP_167349 | similar to tensin | 95 |
| H24-097 | ACATCCTGCTGTCAGAGCC | TA01 - PSK | NM_004783 - | NP_004774 - | thousand and one amino acid protein kinase - prostate | 96 |
| | | | NM_016151 | NP_057235 | derived STE20-like kinase PSK | |
| H24-098 | CATGGAGTTCAGCAAGATC | SLC10A1 | NM_003049 | NP_003040 | solute carrier family 10 (sodium/bile acid cotransporter | 97 |
| | | | | | family), member 1 | |
| H24-099 | GTTCTCCAGTGCCATTGGC | SLC16A3 | NM_004207 | NP_004198 | solute carrier family 16 (monocarboxylic acid | 98 |
| | | | | | transporters), member 3 | |
| H24-100 | TTCGGCCTGGACGTCTATC | ORCTL3 | NM_004256 | NP_004247 | organic cationic transporter-like 3 | 99 |
| H24-101 | GTGCATTGGTGAAAGAGAC | IL5 | NM_000879 | NP_000870 | interleukin 5 (colony-stimulating factor, eosinophil) | 100 |
| H24-102 | TGTGCAGGATAATTGCTGC | TGFB2 | NM_003238 | NP_003229 | transforming growth factor, beta 2 | 101 |
| H24-103 | AGGTGTCACTGTACAGTCC | TNFRSF10A | NM_003844 | NP_003835 | tumor necrosis factor receptor superfamily, member | 102 |
| | | | | | 10a | |
| H24-104 | AGTGCGCATCTTCGGCCTC | FGF14 | NM_004115 | NP_004106 | fibroblast growth factor 14 | 103 |
| H24-105 | TTTGTGGACTCCTACGATC | RHEB2 | NM_005614 | NP_005605 | Ras homolog enriched in brain 2 | 104 |
| H24-106 | GCCCTGATGTCCATCTTCC | NR1 | NM_014434 | NP_055249 | NADPH-dependent FMN and FAD containing oxidoreductase | 105 |
| H24-107 | CATAGGGAAGGACACTTGC | IL1F8 | NM_014438 | NP_055253 | interleukin 1 family, member 8 (eta) | 106 |
| H24-108 | CCTGGATGTGAGAGAGAGC | IL1F8 | NM_014438 | NP_055253 | interleukin 1 family, member 8 (eta) | 107 |
| H24-109 | AACTTGTACTATGAAGGCC | RASSF2 | NM_014737 - | NP_055552 - | Ras association (RaIGDS/AF-6) domain family 2 | 108 |
| | | | NM_170774 | NP_739580 | | |
| H24-110 | GTATTCTGTACACCCTGGC | ARSDR1 | NM_016026 | NP_057110 | androgen-regulated short-chain | 109 |
| | | | | | dehydrogenase/reductase 1 | |
| H24-111 | TTCTCGCAATGGCCAATGC | PPIL1 | NM_016059 | NP_057143 | peptidylprolyl isomerase (cyclophilin)-like 1 | 110 |
| H24-112 | GAAGAACAGCAGCCTGGAC | RASD1 | NM_016084 | NP_057168 | RAS, dexamethasone-induced 1 | 111 |
| H24-113 | TCAGGCGGATCTTGACAGC | DCXR | NM_016286 | NP_057370 | dicarbonyl/L-xylulose reductase | 112 |
| H24-114 | TTCGGCACTGGTTTCCCTC | CSNK2B | NM_001320 | NP_001311 | casein kinase 2, beta polypeptide | 113 |
| H24-115 | GACGAATATCAGCTCTGCC | PRKAG2 | NM_016203 | NP_057287 | protein kinase, AMP-activated, gamma 2 non-catalytic | 114 |
| | | | | | subunit | |
| H24-116 | CCTCTCTACGCCATCTACC | ABCG8 | NM_022437 | NP_071882 | ATP-binding cassette, sub-family G (WHITE), member | 115 |
| | | | | | 8 (sterolin 2) | |
| H24-117 | TCTCTCCACACAAACCTTC | C20orf121 | NM_024331 | NP_077307 | chromosome 20 open reading frame 121 | 116 |
| H24-118 | GGTCGGGAGGAGAACAGTC | OKB1 | NM_033125 | NP_149116 | organic cation transporter 0KB1 | 117 |
| H24-119 | GCGAATTCCACCAGCATTC | SLC26A8 | NM_052961 | NP_443193 | solute carrier family 26, member 8 | 118 |
| H24-120 | TGTCCAGGACCTATTGAGC | UGT1A1 | NM_000463 | NP_000454 | UDP glycosyltransferase 1 family, polypeptide A1 | 119 |
| H24-121 1 | TGACCATCTGCATGATGTC | HMGCR | NM_000859 | NP_000850 | 3-hydroxy-3-methylglutaryl-Coenzyme A reductase | 120 |
| H24-122 | GCATGGCATAGTTGGATTC | HPGD | NM_000860 | NP_000851 | hydroxyprostaglandin dehydrogenase 15-(NAD) | 121 |
| H24-123 | ATGCAGGACATGAACAACC | PLCG2 | NM_002661 | NP_002652 | phospholipase C, gamma 2 (phosphatidylinositol- | 122 |
| | | | | | specific) | |
| H24-124 | ATGTGTGAATGTGGGTTGC | TXNRD1 | NM_182729 - | NP_877393 - | thioredoxin reductase 1 | 123 |
| | | | NM_003330 - | NP_003321 - | | |
| | | | NM_182742 - | NP_877419 - | | |
| | | | NM_182743 | NP_877420 | | |
| H24-125 | TGCAGGCTATTCTGTGAGC | B4GALT5 | NM_004776 | NP_004767 | UDP-Gal:betaGlcNAc beta 1,4- galactosyltransferase, | 124 |
| | | | | | polypeptide 5 | |
| H24-126 | TCGGCACAACAATCTAGAC | IDH3B | NM_174855 - | NP_777280 - | isocitrate dehydrogenase 3 (NAD+) beta | 125 |
| | | | NM_006899 - | NP_008830 - | | |
| | | | NM_174856 | NP_777281 | | |
| H24-127 | GACAGGTGGACAGAGCATC | SIAT4B | NM_006927 | NP_008858 | sialyltransferase 4B (beta-galactosidase alpha-2,3-sialytransferase) | 126 |
| H24-128 | TGTGCGAGACCTCGATTTC | HRMT1L3 | NM_019854 | NP_062828 | HMT1 hnRNP methyltransferase-like 3 (S. cerevisiae) | 127 |
| H24-129 | AGTTTGTGTAGGTATCGCC | B3GALT1 | NM_020981 | NP_066191 | UDP-Gal:betaGlcNAc beta 1,3-galactosyltransferase, | 128 |
| | | | | | polypeptide 1 | |
| H24-130 | AGCATGAAAGAAACCCTGC | ENSG00000169 | ENSG00000169066 | NP_066284 - | 0 - peroxisomal short-chain alcohol dehydrogenase | 129 |
| | | 066 - DHRS4 - | -NM_021004 - | NP_932349 | | |
| | | DHRS4L2 | NM_198083 | | | |
| H24-131 | GAAGATCACCATTGCTGAC | PPIA - | XM_292085 - | XP_292085 - | similar to peptidylprolyl isomerase A (cyclophilin A) - | 130 |
| | | LOC341457 - | XM_497621 - | XP_497621 - | similar to Peptidyl-prolyl cis-trans isomerase A | |
| | | LOC126170 - | XM_371409 - | XP_371409 - | (PPlase) (Rotamase) (Cyclophilin A) (Cyclosporin A- | |
| | | LOC388817 - | NM_203431 - | NP_982255 - | binding protein) (SP18) | |
| | | LOC402102 - | XM-497870 - | XP_497870 - | | |
| | | LOC440892 - | XM_496579 - | XP_496579 - | | |
| | | LOC439953 - | XM_495800 - | XP_496579 - | | |
| | | LOC343384 - | NM_203430 - | XP_495800 - | | |
| | | OAS2 - | XM_291544 - | NP_982254 - | | |
| | | LOC390299 - | NM_178230 - | XP_291544 - | | |
| | | LOC388687 - | XM_372452 - | NP_839944 - | | |
| | | LOC388686 - | XM_372452 - | XP_372452 - | | |
| | | LOC128192 - | XM_371304 - | XP_372452- | | |
| | | LOC392352 | XM_371302 - | XP_371304 - | | |
| | | | XM_060887 - | XP_371302 - | | |
| | | | XM_373301 - | XP_060887 - | | |
| | | | NM_021130 | XP_373301 - | | |
| | | | | NP_066953 | | |
| H24-132 | GCATGAATATTGTGGAGGC | PPIA - | XM-497621 - | XP_497621 - | similar to peptidylprolyl isomerase A (cyclophilin A) - | 131 |
| | | LOC126170 - | XM_371409 - | XP_371409 - | similar to Peptidyl-prolyl cis-trans isomerase A | |
| | | LOC388817 - | NM_ 203431 - | NP_982255 - | (PPlase) (Rotamase) (Cyclophilin A) (Cyclosporin A- | |
| | | LOC402102 - | XM-497870 - | XP_497870 - | binding protein) (SP18) | |
| | | LOC391062 - | XM_372785 - | XP_372785 - | | |
| | | PPIA - | NM_203430 - | NP_982254 - | | |
| | | LOC390299 - | XM_372452 - | XP_372452 - | | |
| | | LOC391352 - | XM_372916 - | XP_372916 - | | |
| | | LOC128192 | XM_060887 - | XP_060887 - | | |
| | | | NM_021130 | NP_066953 | | |
| H24-133 | TGCAGGCAAGCAGACGGTC | OXCT2 | NM_022120 | NP_071403 | 3-oxoacid CoA transferase 2 | 132 |
| H24-134 | TGACGCAGATGATGAATCC | LOC83468 | NM_031302 | NP_112592 | gycosyltransferase | 133 |
| H24-135 | TGGCGCCATGTCAGAGTGC | HDAC10 | NM_032019 | NP_114408 | histone deacetylase 10 | 134 |
| H24-136 | CTTATTGTTCACATTGGCC | LOC170327 | XM_093255 | XP_093255 | similar to cytochrome P-450 | 135 |
| H24-137 | TGGCACCTATGAGAGGATC | LOC166624 | XM_093980 | XP_093980 | similar to UDP-glucuronosyltransferase | 136 |
| H24-138 | TCAGGTGTCCCATTCCAGC | IRAK2 | NM_001570 - | NP_001561 | interleukin-1 receptor-associated kinase 2 | 137 |
| | | | SK180 | | | |
| H24-139 | CTCAGAGGTGGTGGAAGAC | ITK | NM_005546 - | NP_005537 | IL2-inducible T-cell kinase | 138 |
| | | | SK184 | | | |
| H24-140 | TGCAGGAGGAAATTGATGC | AF182273 - | AF182273 - | NP_000767 - | cytochrome P450, subfamily IIIA (niphedipine oxidase), | 139 |
| | | CYP3A3 - | NM_000776 - | NP_059488 | polypeptide 3 - cytochrome P450, subfamily IIIA | |
| | | CYP3A4 | NM_017460 | | (niphedipine oxidase), polypeptide 4 | |
| H24-141 | GAGTCCAGCCTTCATGCCC | HUMCYPIIF - | J02906 - | NP_000765 | cytochrome P450, subfamily IIF, polypeptide 1 | 140 |
| | | CYP2F1 | NM_000774 | | | |
| H24-142 | GTCCAGCTGAAGAAGATCC | GRIN2A | NM_000833 | NP_000824 | glutamate receptor, ionotropic, N-methyl D-aspartate | 141 |
| | | | | | 2A | |
| H24-143 | TTCGGCACTGAGGTCTTGC | FLJ22955 | NM_024819 | NP_079095 | hypothetical protein FLJ22955 | 142 |
| H24-144 | TTGCACACTGAGCTGGAGC | FLJ22955 | NM_024819 | NP_079095 | hypothetical protein FLJ22955 | 143 |
| H24-145 | CCTGCTCTTGAGCAATAAC | TEM5 | NM_032777 | NP_116166 | tumor endothelial marker 5 precursor | 144 |
| H24-146 | TGTCCAGACCACATGGAGC | LOC126537 | XM-497611 | XP-497611 | similar to cytochrome P450, subfamily IVF, polypeptide | 145 |
| | | | | | 2; leukotriene B4 omega-hydroxylase; leukotriene-B4 | |
| | | | | | 20-monooxygenase | |
| H24-147 | TTGCAGGAGGACAAGATGC | GPR153 | NM_207370 | NP_997253 | G protein-coupled receptor 153 | 146 |
| H24-148 | GATTGTGGCCAAGAAGTAC | CLIC6 | NM_053277 | NP_444507 | chloride intracellular channel 6 | 147 |
| H24-149 | CCTCATTATCACCATGCTC | GPR150 | NM_199243 | NP_954713 | G protein-coupled receptor 150 | 148 |
| H24-150 | CTGGTTATTGGCGGGTATC | FLJ16008 | NM_001001665 | NP_001001665 | FLJ16008 protein | 149 |
| H24-151 | TTTGTGCTTCACCAAGTGC | GPR97 | NM_170776 | NP_740746 | G protein-coupled receptor 97 | 150 |
| H24-152 | CTGCACAGTCAACACGGTC | LOC256135 | XM_172523 | XP_172523 | similar to N-formyl peptide receptor | 151 |
| H24-153 | GCAGAGCCAAATATCAGCC | LOC254502 | XM_174355 | XP_174355 | LOC254502 | 152 |
| H24-154 | GTTCAAGAAGCTGCGCCAC | OPN1MW- | NM_000513 - | NP_000504- | opsin 1 (cone pigments), medium-wave-sensitive | 153 |
| | | OPN1LW | NM_020061 | NP_064445 | (color blindness, deutan) - opsin 1 (cone pigments), | |
| | | | | | long-wave-sensitive (color blindness, protan) | |
| H24-155 | CGTCTTCATCAGCGTGCAC | GPR30 | NM_001505 | NP_001496 | G protein-coupled receptor 30 | 154 |
| H24-156 | GCAGTTCCAAGCTTGCATC | OPN1SW | NM_001708 | NP_001699 | opsin 1 (cone pigments), short-wave-sensitive (color | 155 |
| | | | | | blindness, tritan) | |
| H24-157 | GTACCTGCGCCACTTCTTC | CCR3 | NM_001837 - | NP_001828 - | chemokine (C-C motif) receptor 3 | 156 |
| | | | NM_178329 | NP_847899 | | |
| H24-158 | GACCATCACTATCCTGGCC | FZD7 | NM_003507 | NP_003498 | frizzled homolog 7 (Drosophila) | 157 |
| H24-159 | GTCCTTCTACATCAATGCC | GPR23 | NM_005296 | NP_005287 | G protein-coupied receptor 23 | 158 |
| H24-160 | GAAGAAGCAACTGGGAGCC | GPR64 | NM_005756 | NP_005747 | G protein-coupled receptor 64 | 159 |
| H24-162 | GTTCCAGACCTTCATGTGC | CCR9 | NM_006641 - NM_031200 | NP_006632 - NP_112477 | chemokine (C-C motif) receptor 9 | 160 |
| H24-163; | CTGGACCGAGCTGCTTGAC | ADMR | NM_007264 | NP_009195 | adrenomedullin receptor | 161 |
| H24-226 | | | | | | |
| H24-165 | GCAGAGCATGGGCTTAAGC | G2A | NM_013345 | NP_037477 | G protein-coupled receptor | 162 |
| H24-167; | AGCAGGCGGAACATGTTCC | CACNB3 | NM_000725 | NP_000716 | calcium channel, voltage-dependent, beta 3 subunit | 163 |
| H24-228 | | | | | | |
| H24-169 | CAACCTGTTCATCCTTAAC | GALR2 | NM_003857 | NP_003848 | galanin receptor 2 | 164 |
| H24-170 | ATTTGGTGACACGGCTGAC | C10orf112 | XM_295865 | XP_295865 | chromosome 10 open reading frame 112 | 165 |
| H24-171 | CATATGGCTCTTCAAGTAC | LOC121456 | XM_062645 | XP_062645 | similar to solute carrier family 9 (sodium/hydrogen | 166 |
| | | | | | exchanger), isoform 7; nonselective sodium | |
| | | | | | potassium/proton exchanger | |
| H24-172 | TGGCACAGTAACAGCTGCC | ACAT1 | NM_000019 | NP_000010 | acetyl-Coenzyme A acetyltransferase 1 (acetoacetyl | 167 |
| | | | | | Coenzyme A thiolase) | |
| H24-173 | GTTCTCTCAGCACGTTCGC | PGF | NM_002632 | NP_002623 | placental growth factor, vascular endothelial growth | 168 |
| | | | | | factor-related protein | |
| H24-174 | TGCAGACTTCAGTGCAGCC | RHAG | NM_000324 | NP_000315 | Rhesus blood group-associated glycoprotein | 169 |
| H24-175 | CTTTCTGGAAATCCTGCCC | SLC39A1 | NM_014437 | NP_055252 | solute carrier family 39 (zinc transporter), member 3 | 170 |
| H24-176 | CAACACAGCAGACACAGTC | GALGT | NM_001478 | NP_001469 | UDP-N-acetyl-alpha-D-galactosamine:(N- | 171 |
| | | | | | acetylneuraminyl)- galactosylglucosylceramide N- | |
| | | | | | acetylgalactosaminyltransferase (GalNAc-T) | |
| H24-177 | TGGGACCAGTGACTGCAGC | GPLD1 | NM_001503 | NP_001494 | glycosylphosphatidylinositol specific phospholipase D1 | 172 |
| H24-178 | AGCCAGAGGAGTTTGTGGC | HRMT1L1 | NM_001535 - | NP_001526 - | HMT1 hnRNP methyltransferase-like 1 (S. cerevisiae) | 173 |
| | | | NM_206962 | NP_996845 | | |
| H24-179 | CTCGCAGGAAATTCTGGAC | GS3955 | NM_021643 | NP_067675 | GS3955 protein | 174 |
| H24-180 | ATGCAGGTCAGGTTGTTTC | SLC4A10 | NM_022058 | NP_071341 | solute carrier family 4, sodium bicarbonate transporter- | 175 |
| | | | | | like, member 10 | |
| H24-181 | ATCAGGCCTTACATCCAGC | C8FW | NM_025195 | NP_079471 | phosphoprotein regulated by mitogenic pathways | 176 |
| H24-182 | CTGTGGGAGAAACAGAGAC | LOC165927 | XM_093541 | XP_093541 | similar to hypothetical protein, MNCb-4779 | 177 |
| H24-183 | CTCTGCGACTACTACCTGC | B3GALT6 | NM_080605 | NP_542172 | UDP-Gal:betaGal beta 1,3-galactosyltransferase | 178 |
| | | | | | polypeptide 6 | |
| H24-184 | CTGATGAAGGCCTTCGACC | LOC123326 | XM_063593 | XM_063593 | similar to NADH-ubiquinone oxidoreductase PDSW | 179 |
| | | | | | subunit (Complex I-PDSW) (CI-PDSW) | |
| H24-185 | ACCGTGGAAGGCCTATCGC | CYP24 | NM_000782 | NP_000773 | cytochrome P450, subfamily XXIV (vitamin D 24- | 180 |
| | | | | | hydroxylase) | |
| H24-186 | TTCCAGCTGATATATCCAC | FLJ11149 | NM_018339 | NP_060809 | hypothetical protein FLJ11149 | 181 |
| H24-187 | GCCAGACACTGACAAGTTC | TTBK2 | SK453 - | NP_775771 | | 182 |
| | | | NM_173500 | | | |
| H24-188 | TCGGCAGGGCCAGCATTTC | MST1 | NM_020998 | NP_066278 | macrophage stimulating 1 (hepatocyte growth factor- | 183 |
| | | | | | like) | |
| H24-189 | GTCTTCGACCCTCAGGAAC | HRG | NM_000412 | NP_000403 | histidine-rich glycoprotein | 184 |
| H24-190 | TCAGAAGGTTGTGCAGGAC | APG4B | NM_013325 - | NP_037457 - | KIAA0943 protein | 185 |
| | | | NM_178326 | NP_847896 | | |
| H24-191 | CAACTTGCATGACTACGGC | APP | NM_201414 - | NP_958817 - | amyloid beta (A4) precursor protein (protease nexin-II, | 186 |
| | | | NM_000484- | NP_000475 - | Alzheimer disease) | |
| | | | NM_201413 | NP_958816 | | |
| H24-192 | TGTGCAAGAGGTTCGTTGC | PPP2CB | NM_004156 | NP_004147 | protein phosphatase 2 (formerly 2A), catalytic subunit, | 187 |
| | | | | | beta isoform | |
| H24-193 | ACCAGTGGTAAATGTCAGC | DUSP5 | NM_004419 | NP_004410 | dual specificity phosphatase 5 | 188 |
| H24-194 | CTCTGTATCCCATTCCCTC | MAP3K9 | NM_033141 | NP_149132 | mitogen-activated protein kinase kinase kinase 9 | 189 |
| H24-195 | CTTCTTGAAGCATCTATGC | MAP3K1 | XM_042066 | XP_042066 | mitogen-activated protein kinase kinase kinase 1 | 190 |
| H24-196 | CATGCTGTTGTCCTTTACC | LNPEP | NM_005575 | NP_005566 | leucyl/cystinyl aminopeptidase | 191 |
| H24-197 | CGAAGATGTTGACCTGGTC | RNASEL | NM_021133 | NP_066956 | ribonuclease L (2',5'-oligoisoadenylate synthetase- | 192 |
| | | | | | dependent) | |
| H24-198 | TGTCCTGTTGGATGCACAC | PRKAA2 | NM_006252 | NP_006243 | protein kinase, AMP-activated, alpha 2 catalytic | 193 |
| | | | | | subunit | |
| H24-199 | ATGCAGACCCTGGACCAAC | TNFRSF11A | NM_003839 | NP_003830 | tumor necrosis factor receptor superfamily, member | 194 |
| | | | | | 11a, activator of NFKB | |
| H24-200 | GTAGCACTCTGCGACATGC | SLC39A4 | NM_017767- | NP_060237 - | solute carrier family 39 (zinc transporter), member 4 | 195 |
| | | | NM_130849 | NP_570901 | | |
| H24-201 | CTGGAATGGGATCCGACAC | SDHC | NM_003001 | NP_002992 | succinate dehydrogenase complex, subunit C, integral | 196 |
| | | | | | membrane protein, 15kD | |
| H24-202 | GTTATTCTTCCACCATGGC | NNMT | NM_006169 | NP_006160 | nicotinamide N-methyltransferase | 197 |
| H24-203 | TGCCAGCAGTCTCTTGATC | CNTFR | NM_001842 | NP_001833 | ciliary neurotrophic factor receptor | 198 |
| H24-204 | GATCTTCCGGCCCAAACAC | IGFBP5 | NM_000599 | NP_000590 | insulin-like growth factor binding protein 5 | 199 |
| H24-205 | AGCATGACAGGAAACCTGC | UGCGL2 | NM_020121 | NP_064506 | UDP-glucose ceramide glucosyltransferase-like 2 | 200 |
| H24-206 | TCTTCTCTGTGAACATACC | LOC136234 | XM_069782 | XP_069782 | similar to NADH dehydrogenase subunit 1 | 201 |
| H24-207 | CCTTGTTGGCCAATGATTC | AADAC | NM_207365 | NP_997248 | similar to Arylacetamide deacetylase | 202 |
| H24-208 | CCAGGTTTGTCAACGTGAC | AZU1 | NM_001700 | NP_001691 | azurocidin 1 (cationic antimicrobial protein 37) | 203 |
| H24-209 | TTCCAGCTCAGTGCTAATC | LOC161785 | XM_091124 | XP_091124 | similar to microtubule-associated proteins 1A/1B light | 204 |
| | | | | | chain 3 | |
| H24-210 | AGCAGGAACATCTCTTCGC | LOC219756 | XM_166676 | XP_166676 | similar to MNK1 | 205 |
| H24-211 | CAAGTTCTCCTGCAAGTTC | ATP4B | NM_000705 | NP_000696 | ATPase, H+/K+ exchanging, beta polypeptide | 206 |
| H24-212 | GAGCATGACCTTCGATGGC | GABRR2 | NM_002043 | NP_002034 | gamma-aminobutyric acid (GABA) receptor, rho 2 | 207 |
| H24-213 | CTGGCAGAAGCAGCACAAC | ADAMTS1 | NM_006988 | NP_008919 | a disintegrin-like and metalloprotease (reprolysin type) | 208 |
| | | | | | with thrombospondin type 1 motif, 1 | |
| H24-214 | TACCTGCTGGGCATCAAGC | FGF4 | NM_002007 | NP_001998 | fibroblast growth factor 4 (heparin secretory | 209 |
| | | | | | transforming protein 1, Kaposi sarcoma oncogene) | |
| H24-215 | CTGGAAGTCCTGTGTGATC | IFNA8 | NM_002170 | NP_002161 | interferon, alpha 8 | 210 |
| H24-216 | GGACACCTTCCCAAATGTC | IL19 | NM_013371 - | NP_037503 - | interleukin 19 | 211 |
| | | | NM_153758 | NP_715639 | | |
| H24-217 | GGCAGAAATTCCAGAGAGC | SLC10A2 | NM_000452 | NP_000443 | solute carrier family 10 (sodium/bile acid cotransporter | 212 |
| | | | | | family), member 2 | |
| H24-218 | CAACATCCCAACTGTGGTC | GSR | NM_000637 | NP_000628 | glutathione reductase | 213 |
| H24-219 | TATCCTGACCTTCCTGCGC | KCNG1 | NM_002237 - | NP_002228 - | potassium voltage-gated channel, subfamily G, | 214 |
| | | | NM_172318 | NP_758529 | member 1 | |
| H24-220 | TGTTTACCAGTCCGAAGCC | FGF21 | NM_019113 | NP_061986 | fibroblast growth factor 21 | 215 |
| H24-221 | AACTGTACCGCAGAGTTCC | LOC131961 | XM_067688 | XP_067688 | similar to INOSINE-5-MONOPHOSPHATE | 216 |
| | | | | | DEHYDROGENASE 1 (IMP DEHYDROGENASE 1) | |
| | | | | | (IMPDH-I) (IMPD 1) | |
| H24-222 | AGCATGATGATAGTTCCTC | TOP2B | NM_001068 | NP_001059 | topoisomerase (DNA) II beta (180kD) | 217 |
| H24-223 | CAAGTGCCGTATCATCCAC | SRPK1 | NM_003137 - | NP_003128 | SFRS protein kinase 1 | 218 |
| | | | SK358 | | | |
| H24-224 | TATTCGTGCGGAGGAAGAC | SgK071 - | SK521 - | NP_714921 | chromosome 9 open reading frame 96 | 219 |
| | | C9orf96 | NM_153710 | | | |
| H24-227 | GTACCTGCTGGTGGAGTTC | P4HB | NM_000918 | NP_000909 | procollagen-proline, 2-oxoglutarate 4-dioxygenase | 220 |
| | | | | | (proline 4-hydroxylase), beta polypeptide (protein | |
| | | | | | disulfide isomerase; thyroid hormone binding protein | |
| | | | | | p55) | |

Table 5. Overview of additional knock-down constructs and corresponding target gene sequences

| | | | | SEQ ID NO: |
|---|---|---|---|---|
| **KD Target Sequence** | **Target Gene Symbol** | **GenBank accession** | **Name** | **KD constructs** |
| CGTTGGCAGAATCATTTAC | GPR38 | NM_001507 | G protein-coupled receptor | 247 |
| | | | 38 | |
| TTCGCGGATGATGTACTTC | GPR38 | NM_001507 | G protein-coupled receptor | 248 |
| | | | 38 | |
| CTCTCAGTACTTTAACATC | GPR38 | NM_001507 | G protein-coupled receptor | 249 |
| | | | 38 | |
| CATAACAAAGGCATCGCCC | MKNK2 - | NM_199054 - | MAP kinase-interacting | 250 |
| | MNK2 | NM_017572 - | serine/threonine kinase 2 | |
| | | SK236 | | |
| TTAATGATAGCCATCCAGC | CSNK1G1 - | NM_022048 - | casein kinase 1, gamma 1 | 251 |
| | CK1g1 | SK647 | | |
| ATTCCAGGCCATTTATGGC | GZMK | NM_002104 | granzyme K (serine | 252 |
| | | | protease, granzyme 3; | |
| | | | tryptase II) | |
| TGTTAGACTACGTGATGAC | FLJ14906 | NM_032859 | hypothetical protein | 253 |
| | | | FLJ14906 | |
| CAACCTCACCTACAGGATC | MMP23A - | NM_006983 - | matrix metalloproteinase | 254 |
| | MMP23B | NM_004659 | 23A-matrix | |
| | | | metalloproteinase 23B | |
| TCTACCCGATCAACCACAC | MMP23A - | NM_006983 - | matrix metalloproteinase | 255 |
| | MMP23B | NM_004659 | 23A - matrix | |
| | | | metalloproteinase 23B | |
| CCCTTGGAGGAACTATGCC | LOC254378 | XM_174812 | LOC254378 | 256 |
| CAACTCAGACAACTGCATC | LOC254378 | XM_174812 | LOC254378 | 257 |
| TCATTGTAAGGCTGTGGCC | LOC138529 | XM_070951 | similar to bile salt- | 258 |
| | | | dependent lipase oncofetal | |
| | | | isoform | |
| GGACCAATGGGAGATAGAC | FRK | NM_002031 | fyn-related kinase | 259 |
| AGCTTATCCAGCTTTATGC | FRK | NM_002031 | fyn-related kinase | 260 |
| GTGCATTAACAAGAAGAAC | ULK1 | NM_003565 | unc-51-like kinase 1 (C. | 261 |
| | | | elegans) | |
| ACCTGCAGCCTTCACTTTC | CCR1 | NM_001295 | chemokine (C-C motif) | 262 |
| | | | receptor 1 | |
| TCCCTTCTGGATCGACTAC | CCR1 | NM_001295 | chemokine (C-C motif) | 263 |
| | | | receptor 1 | |
| CAATTATGAGTCCACGGTC | OR1A2 | NM_012352 - | olfactory receptor, family 1, | 264 |
| | | NM_014565 | subfamily A, member 2 | |
| AACGATGGGCATGTATTTC | OR1A2 | NM_012352 | olfactory receptor, family 1, | 265 |
| | | | subfamily A, member 2 | |
| TGTCTCCTATGTTCAGGTC | OR1A2 | NM_012352 | olfactory receptor, family 1, | 266 |
| | | | subfamily A, member 2 | |
| GTATGGACAGAACTGGCTC | SENP3 | NM_015670 | sentrin/SUMO-specific | 267 |
| | | | protease 3 | |
| GATGAACATGTATGGAGAC | SENP3 | NM_015670 | sentrin/SUMO-specific | 268 |
| | | | protease 3 | |
| ATTCCTTCAAACGTATGGC | SENP3 | NM_015670 | sentrin/SUMO-specific | 269 |
| | | | protease 3 | |
| GCTCCTGGAGAACATGTAC | TAS1R3 | XM_371210 | taste receptor, type 1, | 270 |
| | | | member 3 | |
| ACCAACCTCAGAGGTTCTC | TAO1 | NM_016151 - | thousand and one amino | 271 |
| | | NM_004783 | acid protein kinase - | |
| | | | prostate derived STE20- | |
| | | | like kinase PSK | |
| AAGCGGACCTACAAACTTC | TAO1 | NM_016151 - | thousand and one amino | 272 |
| | | NM_004783 | acid protein kinase - | |
| | | | prostate derived STE20- | |
| | | | like kinase PSK | |
| CGTCTACATGTACGTGTTC | SLC16A3 | NM_004207 | solute carrier family 16 | 273 |
| | | | (monocarboxylic acid | |
| | | | transporters), member 3 | |
| GTGTGTACATCAACTGTTC | ARSDR1 | NM_016026 | androgen-regulated short- | 274 |
| | | | chain | |
| | | | dehydrogenase/reductase 1 | |
| GGCACAGTCCAATCTGAAC | ARSDR1 | NM_016026 | androgen-regulated short- | 275 |
| | | | chain | |
| | | | dehydrogenase/reductase | |
| | | | 1 | |
| CGCAAGTTCTACTCCATCC | RASD1 | NM_016084 | RAS, dexamethasone- | 276 |
| | | | induced 1 | |
| GGTGTTCAGTCTGGACAAC | RASD1 | NM_016084 | RAS, dexamethasone- | 277 |
| | | | induced 1 | |
| GTGTTCAGTCTGGACAACC | RASD1 | NM_016084 | RAS, dexamethasone- | 278 |
| | | | induced 1 | |
| ACTTGAACTCTCTCCACAC | C20orf121 | NM_024331 | chromosome 20 open | 279 |
| | | | reading frame 121 | |
| GTCTTCAATAACTTGAAGC | C20orf121 | NM_024331 | chromosome 20 open | 280 |
| | | | reading frame 121 | |
| CCACAACAAGCACGTGTTC | HRMT1L3 | NM_019854 | HMT1 hnRNP | 281 |
| | | | methyltransferase-like 3 | |
| | | | (S. cerevisiae) | |
| ATCAATCGAAAGATTCTTC | IRAK2 | NM_001570 - | interleukin-1 receptor- | 282 |
| | | SK180 | associated kinase 2 | |
| CGACGTTGACAATTCCAGC | IRAK2 | NM_001570 - | interleukin-1 receptor- | 283 |
| | | SK180 | associated kinase 2 | |
| GCAGAGTTGCAGATTTGTC | IRAK2 | NM_001570 - | interleukin-1 receptor- | 284 |
| | | SK180 | associated kinase 2 | |
| TCAGCATTCCTACGATAAC | GRIN2A | NM_000833 | glutamate receptor, | 285 |
| | | | ionotropic, N-methyl D- | |
| | | | aspartate 2A | |
| CCGGCAGAAGGATAACCTC | GRIN2A | NM_000833 | glutamate receptor, | 286 |
| | | | ionotropic, N-methyl D- | |
| | | | aspartate 2A | |
| CCAGAACTGTGAAGTTTAC | GRIN2A | NM_000833 | glutamate receptor, | 287 |
| | | | ionotropic, N-methyl D- | |
| | | | aspartate 2A | |
| GCATGGCAAGAAAGTTAAC | GRIN2A | NM_000833 | glutamate receptor, | 288 |
| | | | ionotropic, N-methyl D- | |
| | | | aspartate 2A | |
| AGCATGTTATGCCTTATGC | GRIN2A | NM_000833 | glutamate receptor, | 289 |
| | | | ionotropic, N-methyl D- | |
| | | | aspartate 2A | |
| TCATTGTTTCTGCCATAGC | GRIN2A | NM_000833 | glutamate receptor, | 290 |
| | | | ionotropic, N-methyl D- | |
| | | | aspartate 2A | |
| GATCTTTAACCAGCCTGAC | FLJ22955 | NM_024819 | hypothetical protein | 291 |
| | | | FLJ22955 | |
| GCACACCGTGGTAGTATAC | FLJ22955 | NM_024819 | hypothetical protein | 292 |
| | | | FLJ22955 | |
| GTCCTTCCACATCAAGAAC | TEM5 | NM_032777 | tumor endothelial marker 5 | 293 |
| | | | precursor | |
| TGCAACCTCTTACCCAAGC | CLIC6 | NM_053277 | chloride intracellular | 294 |
| | | | channel 6 | |
| CTCAATCCCTTCGTCTACC | GPR150 | NM_199243 | G protein-coupled receptor | 295 |
| | | | 150 | |
| CGTGGTCTACGCGTTCTAC | XM_094471 | XM_094471 | LOC167417 | 296 |
| CATTATCACCATGCTCGGC | GPR150 | NM_199243 | G protein-coupled receptor | 297 |
| | | | 150 | |
| TCTCTCTTCCTATCAATCC | CCR3 | NM_178329 - | chemokine (C-C motif) | 298 |
| | | NM_001837 | receptor 3 | |
| CCAAACGTGTCTGGAAGAC | GPR23 | NM_005296 | G protein-coupled receptor | 299 |
| | | | 23 | |
| GTACCTGTAGCCATCTAAC | GPR64 | NM_005756 | G protein-coupled receptor | 300 |
| | | | 64 | |
| GCTAGTGAATAATGATTGC | GPR64 | NM_005756 | G protein-coupled receptor | 301 |
| | | | 64 | |
| ACACGACTATAAGTCTAAC | GPR64 | NM_005756 | G protein-coupled receptor | 302 |
| | | | 64 | |
| ACATTGCAATGGACAACAC | APG4B | NM_013325 - | KIAA0943 protein | 303 |
| | | NM_178326 | | |
| GGAATATCCTGAGTGTTGC | DUSP5 | NM_004419 | dual specificity | 304 |
| | | | phosphatase 5 | |
| TGCCAGAGGGATGAACTAC | MAP3K9 | NM_033141 | mitogen-activated protein kinase kinase kinase 9 | 305 |
| ATGGAAGACTGCTGGAATC | MAP3K9 | NM_033141 | mitogen-activated protein | 306 |
| | | | kinase kinase kinase 9 | |
| GAGCGCTTCAAACGAGATC | MAP3K9 | NM_033141 | mitogen-activated protein | 307 |
| | | | kinase kinase kinase 9 | |
| ACCTGTCCCTAGATTCTTC | APG4B | NM_013325 | KIAA0943 protein | 308 |
| ACGCATCTTGGCAAAGAGC | CSNK1G1 - | NM_022048 - | casein kinase 1, gamma 1 | 309 |
| | CK1g1 | SK647 | | |
| ATTCCAGGGTTTATGTGTC | PPIA - | XM_372328 - | peptidylprolyl isomerase A | 310 |
| | LOC390006 - | XM_371409 - | | |
| | LOC388817 - | NM_203431 - | | |
| | LOC442744 - | XM_499491 - | | |
| | LOC342541 - | XM_292596 - | | |
| | LOC344178 - | XM_292963 - | | |
| | LOC388687 - | XM_371304 - | | |
| | LOC391352 - | XM_372916 - | | |
| | LOC256374 - | XM_170597 - | | |
| | LOC388686 - | XM_371302 - | | |
| | LOC122335 - | NM_021130 - | | |
| | LOC390827 - | XM_063084 - | | |
| | LOC390956 - | XM_497571 - | | |
| | LOC442362 - | XM_372741 - | | |
| | LOC343384 - | NM_203430 - | | |
| | COAS2 - | XM_498254 - | | |
| | LOC440063 - | XM_291544 - | | |
| | LOC392352 | NM_178230 - | | |
| | | XM_495896 - | | |
| | | XM_373301 - | | |
| | | XM_060887 - | | |
| | | XM_377444 | | |
| CAACAGTGCATCTCTTATC | LST-3 | XM_292093 | liver-specific organic anion | 311 |
| | | | transporter 3 | |
| CAGCATCTACCTCCTGAAC | CCR1 | NM_001295 | chemokine (C-C motif) | 312 |
| | | | receptor 1 | |
| CAGCGCTCTCAATCCCTTC | GPR150 | NM_199243 | G protein-coupled receptor | 313 |
| | | | 150 | |
| CATCGACTATATAGCAGGC | CEL | NM_001807 | carboxyl ester lipase (bile | 314 |
| | | | salt-stimulated lipase) | |
| CCAAGAGTCTATTCAAAGC | OR1A2 | NM_012352 | olfactory receptor, family 1, | 315 |
| | | | subfamily A, member 2 | |
| CCACTAATGTCAACAATGC | GPR23 | NM_005296 | G protein-coupled receptor | 316 |
| | | | 23 | |
| CCACTCGTCAGATGTTTGC | LOC254378 | XM_174812 | LOC254378 | 317 |
| CCCTGTCATTGATGGAGAC | CEL | NM_001807 | carboxyl ester lipase (bile | 318 |
| | | | salt-stimulated lipase) | |
| CCGAGCCATATACTTGACC | C20orf121 | NM_024331 | chromosome 20 open | 319 |
| | | | reading frame 121 | |
| CCTAGAGCTGATTGAGTTC | MKNK2 - | NM_199054 - | MAP kinase-interacting | 320 |
| | MNK2 | NM_017572 - | serine/threonine kinase 2 | |
| | | SK236 | | |
| CCTCGACACCAAGTCTTGC | RASD1 | NM_016084 | RAS, dexamethasone- | 321 |
| | | | induced 1 | |
| CGCTCTTTAACATGAATGC | TAO1 | NM_016151- | thousand and one amino | 322 |
| | | NM_004783 | acid protein kinase - | |
| | | | prostate derived STE20- | |
| | | | like kinase PSK | |
| CGTGGACATGGAGTACGAC TAS1 R3 | | XM_371210 | taste receptor, type 1, | 323 |
| | | | member 3 | |
| CTCGTAATGAGACTATAGC | ARSDR1 | NM_016026 | androgen-regulated short- | 324 |
| | | | chain | |
| | | | dehydrogenase/reductase | |
| | | | 1 | |
| CTGCAAATCTTCAGGTTTC | GPR64 | NM_005756 | G protein-coupled receptor | 325 |
| | | | 64 | |
| GAAGCACGATTTGGAGGTC | ULK1 | NM_003565 | unc-51-like kinase 1 (C. | 326 |
| | | | elegans) | |
| GATGATGAAGGAGACGTTC | FLJ22955 | NM_024819 | hypothetical protein | 327 |
| | | | FLJ22955 | |
| GATTTGGTTATAAGGGTTC | LOC126170 - | XM_497621 - | peptidylprolyl isomerase A | 328 |
| | LOC388817 - | XM_371409 - | | |
| | PPIA - KBTBD9 | NM_203431 - | | |
| | - LOC256374 - | XM_496546 - | | |
| | LOC131055- | XM_170597 - | | |
| | LOC390956 - | XM_067176 - | | |
| | LOC391062 - | NM_021130 - | | |
| | LOC343384 - | XM_372741 - | | |
| | LOC401859 | XM_372785 - | | |
| | | NM_203430 - | | |
| | | XM_291544 - | | |
| | | XM_377444 | | |
| GCTACTGCCCTATATGATC | SLC39A4 | NM_017767 - | solute carrier family 39 | 329 |
| | | NM_130849 | (zinc transporter), member | |
| | | | 4 | |
| TCCGGTTCTATTTGATCGC | TEM5 | NM_032777 | tumor endothelial marker 5 | 330 |
| | | | precursor | |
| TCGCCCTTCCTATTCCTTC | MAP3K9 | NM_033141 | mitogen-activated protein | 331 |
| | | | kinase kinase kinase 9 | |
| TGTACGTGTTCATCCTGGC | SLC16A3 | NM_004207 | solute carrier family 16 | 332 |
| | | | (monocarboxylic acid | |
| | | | transporters), member 3 | |
| TGTGACATTATGCCTTTGC | OR1A2 | NM_012352 | olfactory receptor, family 1, | 333 |
| | | | subfamily A, member 2 | |

**Table 6. Mineralising hits**

| | | | | |
|---|---|---|---|---|
| H24-001 | CAACTTGTACCTGGGCAGC | GPR38 | NM_001507 | G protein-coupled |
| | | | | receptor 38 |
| H24-004 | CATGCTGTTTGAGAGCATC | MKNK2 - | NM_017572 - | MAP kinase-interacting |
| | | MNK2 | SK236 | serine/threonine kinase 2 |
| H24-006 | GACGGTGTTAATGATAGCC | CSNK1G1 - | NM_022048 - | casein kinase 1, gamma 1 |
| | | CK1g1 | SK647 | |
| H24-007 | CTTCGGCACTCCTGAGTTC | HSA247087 - | AJ247087 - | myosin light chain kinase |
| | | caMLCK | SK536 | |
| H24-009 | ACGCAAAGTGGCCAGGAGC | IPT | NM_017646 | tRNA |
| | | | | isopentenyltransferase 1 |
| H24-013 | CAACCTGCTGGTGCTCGTC | OPN3 | NM_014322 | opsin 3 (encephalopsin, |
| | | | | panopsin) |
| H24-014 | CTCTCTTAGATCTGGAACC | GZMK | NM_002104 | granzyme K (serine |
| | | | | protease, granzyme 3; |
| | | | | tryptase II) |
| H24-015 | AGCAGGAAGGCGGACATAC | AF073344 - | AF073344 - | ubiquitin-specific protease |
| | | USP3 | NM_006537 | 3 - ubiquitin specific |
| | | | | protease 3 |
| H24-018 | TCAGGTAGTTGGTTCTGAC | F13A1 | NM_000129 | coagulation factor XIII, A1 |
| | | | | polypeptide |
| H24-019 | CTGCGCCGAACAAATGTAC | PSMB3 | NM_002795 | proteasome (prosome, |
| | | | | macropain) subunit, beta |
| | | | | type, 3 |
| H24-020 | TGTGGCGACTTGTGCACAC | CLPX | NM_006660 | CIpX caseinolytic |
| | | | | protease X homolog (E. |
| | | | | coli) |
| H24-021 | TCTCTCAGTGTAGAATGCC | FLJ14906 | NM_032859 | hypothetical protein |
| | | | | FLJ14906 |
| H24-024 | GTGTACTGGTACAAGGACC | MMP23A- | NM_004659 - | matrix metalloproteinase |
| | | MMP23B | NM_006983 | 23A - matrix |
| | | | | metalloproteinase 23B |
| H24-026 | TCTCTCATCAATACTGGTC | APEX | NM_001641 - | APEX nuclease |
| | | | NM_080648 - | (multifunctional DNA |
| | | | NM_080649 | repair enzyme) |
| H24-029 | CTATGCCATCACCTTCTGC | LOC254378 | XM_174812 | LOC254378 |
| H24-030 | TGTGCCGAAGGATGTAAGC | LOC137491 | XM_070459 | similar to a disintegrin and |
| | | | | metalloprotease domain |
| | | | | 25 (testase 2) |
| H24-031 | CCGGGACATAACTAAATCC | LOC138529 | XM_070951 | similar to bile salt- |
| | | | | dependent lipase |
| | | | | oncofetal isoform |
| H24-032 | AGCAGGCTATGGGATCAAC | C9 | NM_001737 | complement component 9 |
| H24-033 | CCACAAGGTTGCAGCATTC | XYLB | NM_005108 | xylulokinase homolog (H. |
| | | | | influenzae) |
| H24-035 | GGGCTCAGCCAGGAGATTC | CABC1 - | NM_020247 - | chaperone, ABC1 activity |
| | | ADCK3 | SK609 | of bc1 complex like (S. |
| | | | | pombe) |
| H24-036 | CAGGTAGACATGGCGGCAC | FRK | NM_002031 | fyn-related kinase |
| H24-038 | GCACGATTTGGAGGTCGCC | ULK1 | NM_003565 | unc-51-like kinase 1 (C. |
| | | | | elegans) |
| H24-041 | GGACTCTCAGTTCAGCATC | PIK3C2B | NM_002646 | phosphoinositide-3- |
| | | | | kinase, class 2, beta |
| | | | | polypeptide |
| H24-049 | GTACCTGCAGGTGCTCAGC | AVPR1B | NM_000707 | arginine vasopressin |
| | | | | receptor 1 B |
| H24-054 | GTACCTGCGGCAGTTGTTC | CCR1 | NM_001295 | chemokine (C-C motif) |
| | | | | receptor 1 |
| H24-062 | TTCGGACACCCACAAATGC | RRH | NM_006583 | retinal pigment |
| | | | | epithelium-derived |
| | | | | rhodopsin homolog |
| H24-064 | GTTGTCCTGTTCTGACGTC | OR1A2 | NM_012352 | olfactory receptor, family |
| | | | | 1, subfamily A, member 2 |
| H24-071 | CACCTGCTTTCTCAATGCC | KIAA1453 | NM_025090 | KIAA1453 protein |
| H24-073 | AGCACCTCGCTGACATTCC | SENP3 | NM_015670 | sentrin/SUMO-specific |
| | | | | protease 3 |
| H24-078 | GCTTCTGGTGGAGAAGGAC | TGM3L | XM_066181 | transglutaminase 3-like |
| H24-079 | GTGTATGAAGTGGTCCACC | LOC160662 | XM_090422 | similar to solute carrier |
| | | | | family 21 (organic anion |
| | | | | transporter), member 8 |
| H24-084 | CAGTGCCAAGAAGGAGCCC | NAV2 | NM_018162 | neuron navigator 2 |
| H24-092 | ATGCAGGTCCATATGTGAC | TRPM6 | NM_017662 | transient receptor |
| | | | | potential cation channel, |
| | | | | subfamily M, member 6 |
| H24-093 | CCTTTCTCTGAACACGGAC | ATR | NM_001184 | ataxia telangiectasia and |
| | | | | Rad3 related |
| H24-095 | CAGGTTCTCCTCAAACGGC | LOC126788 | XM_060177 | similar to TPA: G-protein |
| | | | | coupled receptor |
| H24-097 | ACATCCTGCTGTCAGAGCC | TAO1 - PSK | NM_004783 - | thousand and one amino |
| | | | NM_016151 | acid protein kinase - |
| | | | | prostate derived STE20- |
| | | | | like kinase PSK |
| H24-099 | GTTCTCCAGTGCCATTGGC | SLC16A3 | NM_004207 | solute carrier family 16 |
| | | | | (monocarboxylic acid |
| | | | | transporters), member 3 |
| H24-104 | AGTGCGCATCTTCGGCCTC | FGF14 | NM_004115 | fibroblast growth factor 14 |
| H24-106 | GCCCTGATGTCCATCTTCC | NR1 | NM_014434 | NADPH-dependent FMN |
| | | | | and FAD containing |
| | | | | oxidoreductase |
| H24-107 | CATAGGGAAGGACACTTGC | IL1F8 | NM_014438 | interleukin 1 family, |
| | | | | member 8 (eta) |
| H24-108 | CCTGGATGTGAGAGAGAGC | IL1F8 | NM_014438 | interleukin 1 family, |
| | | | | member 8 (eta) |
| H24-109 | AACTTGTACTATGAAGGCC | RASSF2 | NM_014737 | Ras association |
| | | | | (RaIGDS/AF-6) domain |
| | | | | family 2 |
| H24-110 | GTATTCTGTACACCCTGGC | ARSDR1 | NM_016026 | androgen-regulated short- |
| | | | | chain |
| | | | | dehydrogenase/reductase |
| | | | | 1 |
| H24-111 | TTCTCGCAATGGCCAATGC | PPIL1 | NM_016059 | peptidylprolyl isomerase |
| | | | | (cyclophilin)-like 1 |
| H24-112 | GAAGAACAGCAGCCTGGAC | RASD1 | NM_016084 | RAS, dexamethasone- |
| | | | | induced 1 |
| H24-113 | TCAGGCGGATCTTGACAGC | DCXR | NM_016286 | dicarbonyl/L-xylulose |
| | | | | reductase |
| H24-117 | TCTCTCCACACAAACCTTC | C20orf121 | NM_024331 | chromosome 20 open |
| | | | | reading frame 121 |
| H24-119 | GCGAATTCCACCAGCATTC | SLC26A8 | NM_052961 | solute carrier family 26, |
| | | | | member 8 |
| H24-120 | TGTCCAGGACCTATTGAGC | UGT1A1 | NM_000463 | UDP glycosyltransferase |
| | | | | 1 family, polypeptide A1 |
| H24-128 | TGTGCGAGACCTCGATTTC | HRMT1L3 | NM_019854 | HMT1 hnRNP |
| | | | | methyltransferase-like 3 |
| | | | | (S. cerevisiae) |
| H24-130 | AGCATGAAAGAAACCCTGC | ENSG0000016 | ENSG00000169 | peroxisomal short-chain |
| | | 9066 - | 066 - | alcohol dehydrogenase |
| | | humNRDR | NM_021004 | |
| H24-131 | GAAGATCACCATTGCTGAC | PPIA - | NM_021130 - | similar to peptidylprolyl |
| | | LOC127711 - | X_060625 - | isomerase A (cyclophilin |
| | | LOC128430 - | XM_066074 - | A) - similar to Peptidyl- |
| | | LOC138130 - | XM_070771 - | prolyl cis-trans isomerase |
| | | LOC165317 - | XM_092514 - | A (PPlase) (Rotamase) |
| | | LOC257232 | XM_172314 | (Cyclophilin A) |
| | | | | (Cyclosporin A-binding |
| | | | | protein) (SP18) |
| H24-133 | TGCAGGCAAGCAGACGGTC | OXCT2 | NM_022120 | 3-oxoacid CoA |
| | | | | transferase 2 |
| H24-136 | CTTATTGTTCACATTGGCC | LOC170327 | XM_093255 | similar to glyceraldehyde |
| | | | | 3-phosphate |
| | | | | dehydrogenase |
| H24-138 | TCAGGTGTCCCATTCCAGC | IRAK2 | NM_001570 - | interleukin-1 receptor- |
| | | | SK180 | associated kinase 2 |
| H24-141 | GAGTCCAGCCTTCATGCCC | HUMCYPIIF - | J02906 - | cytochrome P450, |
| | | CYP2F1 | NM_000774 | subfamily IIF, polypeptide |
| | | | | 1 |
| H24-142 | GTCCAGCTGAAGAAGATCC | GRIN2A | NM_000833 | glutamate receptor, |
| | | | | ionotropic, N-methyl D- |
| | | | | aspartate 2A |
| H24-143 | TTCGGCACTGAGGTCTTGC | FLJ22955 | NM_024819 | hypothetical protein |
| | | | | FLJ22955 |
| H24-145 | CCTGCTCTTGAGCAATAAC | TEM5 | NM_032777 | tumor endothelial marker |
| | | | | 5 precursor |
| H24-146 | TGTCCAGACCACATGGAGC | LOC126538 | XM_065152 | similar to cytochrome |
| | | | | P450, subfamily IVF, |
| | | | | polypeptide 2; leukotriene |
| | | | | B4 omega-hydroxylase; |
| | | | | leukotriene-B4 20- |
| | | | | monooxygenase |
| H24-148 | GATTGTGGCCAAGAAGTAC | CLIC6 | XM_092804 | chloride intracellular |
| | | | | channel 6 |
| H24-149 | CCTCATTATCACCATGCTC | LOC167417 | XM_094471 | LOC167417 |
| H24-150 | CTGGTTATTGGCGGGTATC | LOC165245 | XM_103864 | similar to 25- |
| | | | | hydroxyvitamin D-1 alpha |
| | | | | hydroxylase, |
| | | | | mitochondrial precursor |
| | | | | (25-OHD-1 alpha- |
| | | | | hydroxylase) (25- |
| | | | | hydroxyvitamin D3 1- |
| | | | | alpha-hydroxylase) (VD3 |
| | | | | 1A hydroxylase) (P450C1 |
| | | | | alpha) (P450VD1-alpha) |
| H24-154 | GTTCAAGAAGCTGCGCCAC | OPN1MW - | NM_000513- | opsin 1 (cone pigments), |
| | | OPN1LW | NM_020061 | medium-wave-sensitive |
| | | | | (color blindness, deutan) - |
| | | | | opsin 1 (cone pigments), |
| | | | | long-wave-sensitive (color |
| | | | | blindness, protan) |
| H24-156 | GCAGTTCCAAGCTTGCATC | OPN1SW | NM_001708 | opsin 1 (cone pigments), |
| | | | | short-wave-sensitive |
| | | | | (color blindness, tritan) |
| H24-157 | GTACCTGCGCCACTTCTTC | CCR3 | NM_001837 | chemokine (C-C motif) |
| | | | | receptor 3 |
| H24-159 | GTCCTTCTACATCAATGCC | GPR23 | NM_005296 | G protein-coupled |
| | | | | receptor 23 |
| H24-160 | GAAGAAGCAACTGGGAGCC | GPR64 | NM_005756 | G protein-coupled |
| | | | | receptor 64 |
| H24-169 | CAACCTGTTCATCCTTAAC | GALR2 | NM_003857 | galanin receptor 2 |
| H24-173 | GTTCTCTCAGCACGTTCGC | PGF | NM_002632 | placental growth factor, |
| | | | | vascular endothelial |
| | | | | growth factor-related |
| | | | | protein |
| H24-180 | ATGCAGGTCAGGTTGTTTC | SLC4A10 | NM_022058 | solute carrier family 4, |
| | | | | sodium bicarbonate |
| | | | | transporter-like, member |
| | | | | 10 |
| H24-185 | ACCGTGGAAGGCCTATCGC | CYP24 | NM_000782 | cytochrome P450, |
| | | | | subfamily XXIV (vitamin D |
| | | | | 24-hydroxylase) |
| H24-188 | TCGGCAGGGCCAGCATTTC | MST1 | NM_020998 | macrophage stimulating 1 |
| | | | | (hepatocyte growth factor- |
| | | | | like) |
| H24-190 | TCAGAAGGTTGTGCAGGAC | Apg4B | NM_013325 | KIAA0943 protein |
| H24-191 | CAACTTGCATGACTACGGC | APP | NM_000484 | amyloid beta (A4) |
| | | | | precursor protein |
| | | | | (protease nexin-II, |
| | | | | Alzheimer disease) |
| H24-193 | ACCAGTGGTAAATGTCAGC | DUSP5 | NM_004419 | dual specificity |
| | | | | phosphatase 5 |
| H24-194 | CTCTGTATCCCATTCCCTC | MAP3K9 | XM_027237 | mitogen-activated protein |
| | | | | kinase kinase kinase 9 |
| H24-200 | GTAGCACTCTGCGACATGC | SLC39A4 | NM_017767 - | solute carrier family 39 |
| | | | NM_130849 | (zinc transporter), |
| | | | | member 4 |
| H24-202 | GTTATTCTTCCACCATGGC | NNMT | NM_006169 | nicotinamide N- |
| | | | | methyltransferase |
| H24-205 | AGCATGACAGGAAACCTGC | UGCGL2 | NM_020121 | UDP-glucose ceramide |
| | | | | glucosyltransferase-like 2 |
| H24-207 | CCTTGTTGGCCAATGATTC | LOC166161 | XM_093702 | similar to arylacetamide |
| | | | | deacetylase (esterase) |
| H24-211 | CAAGTTCTCCTGCAAGTTC | ATP4B | NM_000705 | ATPase, H+/K+ |
| | | | | exchanging, beta |
| | | | | polypeptide |
| H24-218 | CAACATCCCAACTGTGGTC | GSR | NM_000637 | glutathione reductase |
| H24-219 | TATCCTGACCTTCCTGCGC | KCNG1 | NM_002237 | potassium voltage-gated |
| | | | | channel, subfamily G, member 1 |
| H24-224 | TATTCGTGCGGAGGAAGAC | SgK071 | SK521 | |
| H24-225 | ATGGGCTTCAACAGCCACC | AVPR1B | NM_000707 | arginine vasopressin |
| | | | | receptor 1B |

### REFERENCES

Lipinsky, CA, et al. (2001). Adv Drug Deliv Rev 46: 3-26
Nakashima K, de Crombrugghe B. (2003). Trends Genet. 19(8):458-66.
Marzia M, et al. (2000). J Cell Biol 151:311
Thirunavukkarasu et al., (2000) J Biol Chem 275: 25163-72
Yamada et al., (2003) Blood 101 : 2227-34

## Claims

1. *In vitro* method for inducing differentiation of undifferentiated mammalian cells into osteoblasts, comprising contacting said undifferentiated cells with an inhibitor of a MAP kinase-interacting serine/threonine kinase 2 polypeptide, said inhibitor is selected from the group consisting of an antisense RNA, a ribozyme that cleaves the polyribonucleotide, an antisense oligodeoxynucleotide (ODN), and a small interfering RNA (siRNA) that is homologous to a portion of the polyribonucleotide such that the siRNA is capable of inhibiting the polyribonucleotide that would otherwise cause the production of a MAP kinase-interacting serine/threonine kinase 2 polypeptide.

2. Method according to claim 1, wherein the inhibitor is a siRNA, comprising a first nucleotide sequence of 17-23 nucleotides homologous to a nucleotide sequence selected from a MAP kinase-interacting serine/threonine kinase 2 target gene and a second nucleotide sequence of 17-23 nucleotides complementary to said first nucleotide sequence.

3. Method according to claim 2, wherein the inhibitor is a siRNA, comprising a first nucleotide sequence of 17-23 nucleotides selected from the nucleotide sequences identified by SEQ. ID NO. 6, and a second nucleotide sequence of 17-23 nucleotides complementary to said first nucleotide sequence.

4. Method according to claim 2 or claim 3, wherein the siRNA further comprises a third nucleotide sequence connecting the first and second nucleotide sequence, and capable of forming a stem-loop structure within the siRNA.

5. Method according to claim 4, wherein the third nucleotide sequence consists of the nucleotide sequence UUGCUAUA (SEQ ID NO: 334).

6. Method for identifying a compound that induces differentiation of undifferentiated mammalian cells into osteoblasts, comprising:
a) contacting one or more compounds with a MAP kinase-interacting serine/threonine kinase 2 polypeptide;
b) determining the binding affinity of the compound to the MAP kinase-interacting serine/threonine kinase 2 polypeptide;
c) contacting a population of undifferentiated mammalian cells with the compound that exhibits a binding affinity of at least 10 micromolar; and
d) identifying the compound that induces the differentiation of the undifferentiated mammalian cells.

7. Method for identifying a compound or mixture of compounds that induces differentiation of undifferentiated mammalian cells into osteoblasts, comprising:
a) culturing a population of undifferentiated mammalian cells that express a polypeptide encoded by a MAP kinase-interacting serine/threonine kinase 2 gene;
b) exposing said population of cells to a compound or mixture of compounds; and
c) selecting the compound or mixture of compounds that induces the differentiation of the undifferentiated cells into osteoblasts.

8. Method according to claim 6 or 7, wherein the compounds are selected from the group consisting of low molecular weight compounds, peptides, lipids, natural compounds, and antibodies.

9. siRNA comprising a first nucleotide sequence of 17-23 nucleotides homologous to a nucleotide sequence selected from a MAP kinase-interacting serine/threonine kinase 2 target gene and a second nucleotide sequence of 17-23 nucleotides complementary to said first nucleotide sequence for use in the treatment of a disease selected from the group consisting of osteoporosis, hypercalcemia of malignancy, multiple myelomatosis, hyperparathyroidism, and hyperthyroidism.

10. Method for *in vitro* production of bone tissue, comprising:
a) applying undifferentiated mammalian cells on a substrate to form a cellular substrate;
b) introducing a siRNA comprising a first nucleotide sequence of 17-23 nucleotides homologous to a nucleotide sequence selected from a MAP kinase-interacting serine/threonine kinase 2 target gene and a second nucleotide sequence of 17-23 nucleotides complementary to said first nucleotide sequence, or a vector comprising said siRNA, for a time sufficient to differentiate the undifferentiated cells into osteoblasts, thereby producing a continuous bone matrix.

11. Method for diagnosing a pathological condition involving a systemic or local decrease in mean bone density or a susceptibility to the condition in a subject, comprising:
a) determining the level of expression of a polynucleotide expressed by a MAP kinase-interacting serine/threonine kinase 2 target gene, or the amount of a polypeptide encoded by a MAP kinase-interacting serine/threonine kinase 2 gene, in a biological sample derived from said subject; and
b) comparing said level or amount with the level or amount in a sample derived from a healthy subject; wherein an increase of the level or amount in the sample of the subject compared to the sample of the healthy subject is indicative of the presence of the pathological condition.

12. Method according to claim 11, wherein the pathological condition is selected from the group consisting of osteoporosis, hypercalcemia of malignancy, multiple myelomatosis, hyperparathyroidism, and hyperthyroidism.
